# EUROPÄISCHE PATENTSCHRIFT

(11) **EP 2 021 344 B1**
(45) Veröffentlichungstag und Bekanntmachung des Hinweises auf die Patenterteilung: **08.05.2013**
(21) Anmeldenummer: 07723361.7
(22) Anmeldetag: 19.03.2007
(51) Int. Cl.: C07D 491/04

(54) **BICYCLISCHE ENAMINO(THIO)CARBONYLVERBINDUNGEN**
BICYCLIC ENAMINO(THIO)CARBONYL COMPOUNDS
COMPOSÉS ÉNAMINO(THIO)CARBONYLÉS BICYCLIQUES

(30) Priorität: 31.03.2006 DE 102006015456
(43) Veröffentlichungstag der Anmeldung: 11.02.2009
(73) Patentinhaber: Bayer CropScience AG, 40789 Monheim (DE)
(72) Erfinder: JESCHKE, Peter, 51467 Bergisch Gladbach (DE); VELTEN, Robert, 40764 Langenfeld (DE); SCHENKE, Thomas, 51469 Bergisch Gladbach (DE); BECK, Michael, Edmund, 40789 Monheim (DE); MALSAM, Olga, 51503 Rösrath (DE); RECKMANN, Udo, 51063 Köln (DE); NAUEN, Ralf, 40764 Langenfeld (DE); GÖRGENS, Ulrich, 40882 Ratingen (DE); PITTA, Leonardo, 51371 Leverkusen (DE); ARNOLD, Christian, 40764 Langenfeld (DE); SANWALD, Erich, 24159 Kiel (DE)
(86) Internationale Anmeldenummer: PCT/EP2007/002393
(87) Internationale Veröffentlichungsnummer: WO 2007/115647

(56) Entgegenhaltungen:
- WO-A-01/40202
- WO-A-02/085915

## Beschreibung

Beschrieben werden neue bicyclische Enamino(thio)carbonyl-verbindungen, Verfahren zu Ihrer Herstellung und ihre Verwendung zur Bekämpfung von tierischen Schädlingen, vor allem von Arthropoden, insbesondere Insekten.

Die Synthese von speziellen bicyclischen Enaminocarbonylsystemen ist bekannt (vgl. beispielsweise 5,6-Dihydro-4H-furo[3,2-b]pyridin-2-on: Good, R. H. et al. J. Chem. Soc., Perkin Transactions 1: Organic and Bio-Organic Chemistry (1972-1999) (1972), (19), 2441-2445; Jones, G., Phipps, J. R. J. Chem. Soc., Perkin Transactions 1: Organic and Bio-Organic Chemistry (1972-1999) (1975), (20), 458-461; 5,6,7,7a-Tetrahydro-4H-furo[3,2-b]pyridine-2-on: Good, R. H. et al. Tetrahedron Lett. (1972), 7,609-612; 3-Acetyl-5,6,7,7a-Tetrahydro-6,6-dimethyl-furo[3,2-b]pyridine-2-on: Brown, R. F. C, et al. Australian J. Chem. (1967), 20, 2485-97; 6,6a-Dihydro-4-[2-(phenyl bzw. hetaryl)ethyl]-2H-furo[3,2-b]pyrrole-2,5(4H)-dione: Lee, Y. S. et al., Synth. Commun. (1997), 27, 2799-2812).

Bicyclische Enamino(thiocarbonylverbindungen und deren Verwendung als Mittel zur Bekämpfung von tierischen Schädlingen, vor allem von Arthropoden, insbesondere Insekten, sind bisher nicht bekannt geworden.

Es wurden Verbindungen der Formel (I) gefunden, 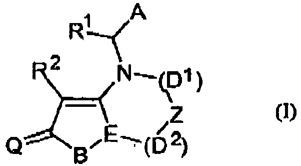 in welcher
- A: für einen gegebenenfalls substituierten Arylrest oder Heterocyclylrest oder für einen Hetarylrest aus der Reihe Pyridyl, Pyrimidinyl, Pyridazinyl, Pyrazinyl, Triazinyl, Pyrazolyl, Thiophenyl, Oxazolyl, Isoxazolyl, Oxadiazolyl, Thiadiazolyl, Isothiazolyl, Imidazolyl, Pyrrolyl, Furanyl, Thiazolyl, Triazolyl, steht, welche gegebenenfalls durch Fluor, Chlor, Brom, Iod, Cyano, Nitro, C₁-C₄-Alkyl (welches gegebenenfalls durch Fluor und/oder Chlor substituiert ist), C₁-C₃-Alkylthio (welches gegebenenfalls durch Fluor und/oder Chlor substituiert ist), oder C₁-C₃-Alkylsulfonyl (welches gegebenenfalls durch Fluor und/oder Chlor substituiert ist), substituiert sind,
- B: für jeweils Sauerstoff, Schwefel, gegebenenfalls substituierten Stickstoff oder Methylen steht,
- E: für CH, C-alkyl oder Stickstoff steht,
- D¹-Z-D²: als Gruppe gemeinsam mit den sie verknüpfenden Atomen einen gegebenenfalls ein oder mehrere Heteroatome enthaltenden und gegebenenfalls substituierten fünf, sechs oder sieben gliedrigen Ring bilden,
- R¹: für Wasserstoff oder Alkyl steht,
- R²: für jeweils Wasserstoff, Alkyl, Cycloalkyl, Halogenalkyl, Nitro, Cyan, Formyl, Carboxyl, Alkoxycarbonyl, Alkylcarbonyl, Halogenalkylcarbonyl, Halogen steht,
- Q: für Sauerstoff oder Schwefel steht.

Weiter wurde gefunden, dass man die neuen substituierten bicyclischen Enamino- (thio)carbonylverbindungen der Formel (I) erhält, wenn man
a) gemäß der Herstellungsmethode 1
   Verbindungen der allgemeinen Formel (II) 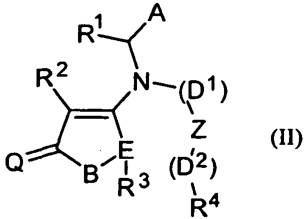 in welcher
   - Z: bevorzugt für gegebenenfalls substituiertes Methylen steht,
   - R³: bevorzugt für Wasserstoff oder in situ erzeugtes Halogen, beispielsweise Brom, steht
   - R⁴: bevorzugt für eine geeignete Abgangsgruppe (Leaving Group), beispielsweise Halogen, oder für Wasserstoff steht, und
   - A, B, E, R¹, R², D¹, D², Q: die weiter oben genannte Bedeutung haben,
   in Gegenwart eines geeigneten basischen Reaktionshilfsmittels und gegebenenfalls in Gegenwart eines geeigneten Verdünnungsmittels intramolekular cyclisiert, oder indem man
b) gemäß der Herstellungsmethode 2
   Verbindungen der allgemeinen Formel (III) 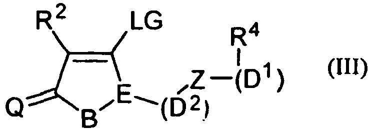 in welcher
   - Z: bevorzugt für gegebenenfalls substituiertes Methylen steht,
   - LG: für eine geeignete Abgangsgruppe (Leaving Group), wie ein sekundärer Aminorest, Hydroxy, Alkoxy oder Alkylthio steht,
   - B, E, R², D¹, D², Q: die weiter oben genannte Bedeutung haben,
   - R⁴: für eine geeignete Abgangsgruppe (Leaving Group), beispielsweise Halogen, steht,
   in einem ersten Reaktionsschritt mit Verbindungen der allgemeinen Formel (IV) 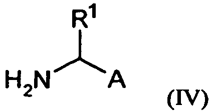 in welcher
   A und R¹ die weiter oben genannte Bedeutung haben,
   in Gegenwart eines geeigneten basischen Reaktionshilfsmittels und gegebenenfalls in Gegenwart eines geeigneten Verdünnungsmittels zu Verbindungen der allgemeinen Formel (V) umsetzt, 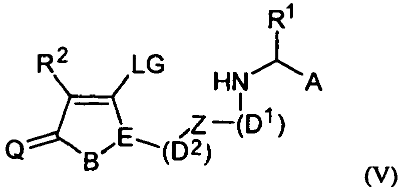 in welcher
   - Z: bevorzugt für gegebenenfalls substituiertes Methylen steht,
   - LG: für eine geeignete Abgangsgruppe (Leaving Group), beispielsweise ein sekundärer Aminorest, Hydroxy, Alkoxy oder Alkylthio steht,
   - A, B, E, R¹, R², D¹, D², Q: die weiter oben genannte Bedeutung haben, und
   diese dann in Gegenwart eines geeigneten sauren Reaktionshilfsmittels und gegebenenfalls in Gegenwart eines geeigneten Verdünnungsmittels in einem zweiten Reaktionsschritt intramolekular cyclisiert, oder indem man
c) gemäß der Herstellungsmethode 3
   Verbindungen der allgemeinen Formel (VI) 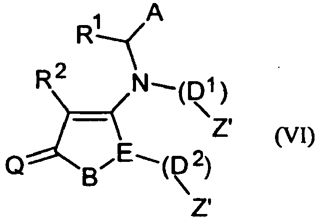 in welcher
   - Z': bevorzugt für eine ungesättigte Kohlenstoff-Kohlenstoff-Bindung steht,
   - A, B, E, R¹, R², D¹, D², Q: die weiter oben genannte Bedeutung haben,
   in Gegenwart eines geeigneten Katalysators und gegebenenfalls in Gegenwart eines geeigneten Verdünnungsmittels umsetzt, oder indem man
d) gemäß der Herstellungsmethode 4
   Verbindungen der allgemeinen Formel (VII) 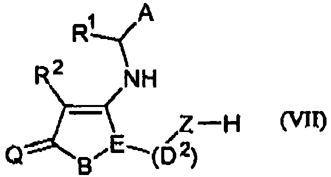 in welcher
   - Z: bevorzugt für ein Heteroatom aus der Reihe Sauerstoff, Schwefel, Stickstoff, steht,
   - A, B, R¹, R², D², Q: die weiter oben genannte Bedeutung haben,
   mit Verbindungen der allgemeinen Formeln (VIII) oder (IX)

   H-CO-R' (VIII)

   oder

   (-O-CHR'-)ₙ (IX)

   in welcher
   - R': für Wasserstoff oder C₁-C₄-Alkyl steht,
   - n: für ≤ 3 steht,
gegebenenfalls in Gegenwart eines sauren Reaktionshilfsmittels und gegebenenfalls in Gegenwart eines Verdünnungsmittels umsetzt.

Schließlich wurde gefunden, dass die neuen Verbindungen der Formel (I) stark ausgeprägte biologische Eigenschaften besitzen und vor allem zur Bekämpfung von tierischen Schädlingen insbesondere von Insekten, Spinnentieren und Nematoden, die in der Landwirtschaft, in den Forsten, im Vorrats- und Materialschutz sowie auf dem Hygienesektor vorkommen geeignet sind.

Die Verbindungen der Formel (I) können gegebenenfalls in Abhängigkeit von der Art der Substituenten als geometrische und/oder als optisch Aktive Isomere oder entsprechende Isomerengemische in unterschiedlicher Zusammensetzung vorliegen. Die Erfindung betrifft sowohl die reinen Isomere als auch die Isomerengemische von erfindungsgemäβen Bicyclische Enaminocarbonyl-verbindungen der Formel (I) 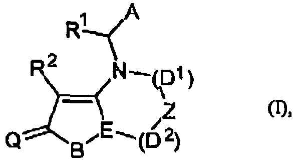 in welcher
- A: für einen Rest steht, ausgewählt aus der Gruppe bestehend aus 6-Chlor-pyrid-3-yl, 6-Brom-pyrid-3-yl, 6-Methyl-pyrid-3-yl, 6-Trifluormethyl-pyrid-3-yl, 2-Methym-pyrimidin-5-yl, 2-Chlor-pyrimid-5-yl, 1*H*-Pyrazol-4-yl, welches gegebenenfalls in 1-Position substituiert ist durch Methyl, Ethyl, und in 3-Position durch Chlor, 1H-Pyrazol-5-yl, 3-Methyl-pyrazol-5-yl, 2-Brom-1,3-thiazol-5-yl, 2-Chlor-1,3-thiazol-5-yl, Isoxazol-5-yl, welches gegebenenfalls in 3-Position substituiert ist durch Methyl, Ethyl, Chlor oder Brom, 3-Methyl-1,2,4-oxadiazol-5-yl, 1-Methyl-1,2,4-triazol-3-yl, 1,2,5-Thiadiazol-3-yl 5-Fluor-6-chlor-pyrid-3-yl, 5,6-Dichlor-pyrid-3-yl, 5-Brom-6-chlor-pyrid-3-yl, 5-Fluor-6-brom-pyrid-3-yl, 5-Chlor-6-brom-pyrid-3-yl, 5,6-Dibrom-pyrid-3-yl, 5-Methyl-6-chlor-pyrid-3-yl, 5-Methyl-6-iod-pyrid-3-yl und 5-Difluormethyl-6-chlor-pyrid-3-yl,
- B: für Sauerstoff, oder Methylen steht,
- E: für CH oder C-alkyl steht,
- D¹-Z-D²: für eine der folgenden chemischen Gruppierungen steht: -CH₂-CH₂-, -HC=CH-, -CH₂-CH₂-CH₂-, -CH₂-C(=CH₂)-CH₂-, -CH₂-CO-CH₂-, -CH₂-CH(OH)-CH₂-, -CH₂-CF₂-CH₂-, -CH₂-CHF-CH₂-, -CH₂-CHCl-CH₂-, -CH=CH-CH₂-, -CH₂-CH₂-CH= -CH₂-CH₂-CH(OH)-, -CH₂-CH(CH₃)-CH₂-, -CH₂-CH₂-CH₂-CH₂-, -CH₂-CH=CH-CH₂, -CH₂-O-CH₂-, -CH₂-CH₂-O-, -CH₂-N(CH₃)-CH₂-, -CH₂-CH₂-N(CH₃)- oder -CH₂-S-CH₂-,
- R¹: für Wasserstoff steht,
- R²: für Wasserstoff, Fluor oder Chlor steht, und
- Q: für Sauerstoff steht.

Bevorzugte Substituenten bzw. Bereiche der in den oben und nachstehend erwähnten Formeln aufgeführten Reste werden im Folgenden erläutert.
- A: steht bevorzugt für Tetrahydrofuryl oder für Pyrid-3-yl, welches gegebenenfalls in 6-Position substituiert ist durch Halogen, C₁-C₄-Alkyl oder C₁-C₄-Halogenalkyl, Pyrimidin-5-yl, welches gegebenenfalls in 2-Position substituiert ist durch Halogen oder C₁-C₄-Alkyl, 1*H-*Pyrazol-4-yl, gegebenenfalls in 1-Position substituiert ist durch C₁-C₄-Alkyl und in 3-Position durch Halogen, 1*H*-Pyrazol-5-yl, gegebenenfalls in 3-Position substituiert ist durch Halogen oder C₁-C₄-Alkyl, Isoxazol-5-yl, gegebenenfalls in 3-Position substituiert ist durch Halogen oder C₁-C₄-Alkyl, 1,2,4-Oxadiazol-5-yl, gegebenenfalls in 3-Position substituiert ist durch Halogen oder C₁-C₄-Alkyl, 1-Methyl-1,2,4-triazol-3-yl, 1,2,5-Thiadiazol-3-yl, 1,3-Thiazolyl-5-yl, gegebenenfalls in 2-Position substituiert ist durch Halogen oder C₁-C₄-Alkyl.

Des Weiteren steht
- A: bevorzugt für einen Rest 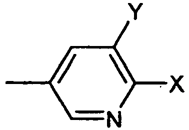 in welchem
X Halogen, C₁-C₄-Alkyl, C₁-C₄-Halogenalkyl
Y Halogen, C₁-C₄-Alkyl, C₁-C₄-Halogenalkyl, C₁-C₄-Halogenalkoxy, Azido und Cyan bedeuten, insbesondere steht
- A: für einen Rest aus der Reihe 5,6-Difluor-pyrid-3-yl, 5-Chlor-6-fluor-pyrid-3-yl, 5-Brom-6-fluor-pyrid-3-yl, 5-Iod-6-fluor-pyrid-3-yl, 5-Fluor-6-chlor-pyrid-3-yl, 5,6-Dichlor-pyrid-3-yl, 5-Brom-6-chlor-pyrid-3-yl, 5-Iod-6-chlor-pyrid-3-yl, 5-Fluor-6-brom-pyrid-3-yl, 5-Chlor-6-brom-pyrid-3-yl, 5,6-Dibrom-pyrid-3-yl, 5-Fluor-6-iod-pyrid-3-yl, 5-Chlor-6-iod-pyrid-3-yl, 5-Brom-6-iod-pyrid-3-yl, 5-Methyl-6-fluor-pyrid-3-yl, 5-Methyl-6-chlor-pyrid-3-yl, 5-Methyl-6-brom-pyrid-3-yl, 5-Methyl-6-iod-pyrid-3-yl, 5-Difluormethyl-6-fluor-pyrid-3-yl, 5-Difluormethyl-6-chlor-pyrid-3-yl, 5-Difluormethyl-6-brom-pyrid-3-yl, 5-Difluormethyl-6-iod-pyrid-3-yl.
- B: steht bevorzugt für Sauerstoff oder Methylen.
- D¹-Z-D²: als Gruppe steht bevorzugt für eine gegebenenfalls durch Halogen, Oxo, Hydroxy, C₁-C₄-Alkyl oder C₁-C₄-Alkylen substituierte C₂-C₄-gesättigte oder ungesättigte Gruppe, in der gegebenenfalls ein Kohlenstoffatom durch ein Heteroatom aus der Reihe Sauerstoff, Schwefel oder Stickstoff ersetzt sein kann.
- R¹: steht bevorzugt für Wasserstoff
- R²: steht bevorzugt für Wasserstoff, Alkyl oder Halogen (wobei Halogen insbesondere für Fluor oder Chlor steht).
- Q: steht bevorzugt für Sauerstoff,
- A: steht besonders bevorzugt für einen Rest aus der Reihe 6-Chlor-pyrid-3-yl, 6-Brom-pyrid-3-yl, 6-Methyl-pyrid-3-yl, 6-Trifluormethyl-pyrid-3-yl, 2-Methyl-pyrimidin-5-yl, 2-Chlor-pyrimid-5-yl, 1*H*-Pyrazol-4-yl, welches gegebenenfalls in 1-Position substituiert ist durch Methyl, Ethyl, und in 3-Position durch Chlor, 1*H*-Pyrazol-5-yl, 3-Methyl-pyrazol-5-yl, 2-Brom-1,3-thiazol-5-yl, 2-Chlor-1,3-thiazol-5-yl, Isoxazol-5-yl, welches gegebenenfalls in 3-Position substituiert ist durch Methyl, Ethyl, Chlor oder Brom, 3-Methyl-1,2,4-oxadiazol-5-yl, 1-Methyl-1,2,4-triazol-3-yl, 1,2,5-Thiadiazol-3-yl.
- A: steht weiterhin besonders bevorzugt für einen Rest aus der Reihe 5-Fluor-6-chlor-pyrid-3-yl, 5,6-Dichlor-pyrid-3-yl, 5-Brom-6-chlor-pyrid-3-yl, 5-Fluor-6-brom-pyrid-3-yl, 5-Chlor-6-brom-pyrid-3-yl, 5,6-Dibrom-pyrid-3-yl, 5-Methyl-6-chlor-pyrid-3-yl, 5-Methyl-6-iod-pyrid-3-yl oder 5-Difluormethyl-6-chlor-pyrid-3-yl.
- B: steht besonders bevorzugt für Sauerstoff oder Methylen.
- D¹-Z-D²: als Gruppe steht besonders bevorzugt für eine gegebenenfalls durch C₁-C₂-Alkyl oder C₁-C₂-Alkylen substituierte C₂-C₃-gesättigte oder ungesättigte Gruppe in der ein Kohlenstoffatom durch ein Heteroatom aus der Reihe Sauerstoff, Schwefel oder Stickstoff ersetzt sein kann, insbesondere für -CH₂-CH₂-, -HC=CH-, -CH₂-CH₂-CH₂-, -CH₂-C(=CH₂)-CH₂-, -CH₂-CO-CH₂-, -CH₂-CH(OH)CH₂-, -CH₂-CF₂-CH₂-, -CH₂-CHF-CH₂-, -CH₂-CHCl-CH₂-, -CH=CH-CH₂-, -CH₂-CH₂-CH=, -CH₂-CH₂-CH(OH)-, -CH₂-CH(CH₃)CH₂-, -CH₂-CH₂-CH₂-CH₂-, -CH₂-CH=CH-CH₂-, -CH₂-O-CH₂-, -CH₂-CH₂-O-, -CH₂-N(CH₃)-CH₂-, -CH₂-CH₂-N(CH₃)- oder -CH₂-S-CH₂-.
- R¹: steht besonders bevorzugt für Wasserstoff.
- R²: steht besonders bevorzugt für jeweils Wasserstoff, Fluor oder Chlor.
- Q: steht besonders bevorzugt für Sauerstoff.
- A: steht ganz besonders bevorzugt für einen Rest aus der Reihe 6-Chlor-pyrid-3-yl, 6-Brom-pyrid-3-yl, 2-Chlor-pyrimid-5-yl, 5-Fluor-6-chlor-pyrid-3-yl, 5,6-Dichlor-pyrid-3-yl, 5-Fluor-6-brom-pyrid-3-yl, 2-Chlor-1,3,-thiazol-5-yl.
- B: steht ganz besonders bevorzugt für Sauerstoff.
- D¹-Z-D²: als Gruppe steht ganz besonders bevorzugt für -CH₂-CH₂-CH₂-, -CH₂-C(=CH₂)-CH₂-, -CH₂-C(CH₃)-CH₂-, -CH₂-CH₂-CH₂-CH₂-, -CH₂-CH=CH-CH₂-, -CH₂-O-CH₂-, - CH₂-CH₂-O-, -CH₂-CHF-CH₂-, -CH₂-CHCl-CH₂-, -CH=CH-CH₂-, - CH₂-CH₂-CH= oder -CH₂-CH₂-CH(OH)-.
- R¹: steht ganz besonders bevorzugt für Wasserstoff,
- R²: steht ganz besonders bevorzugt für Wasserstoff.
- Q: steht ganz besonders bevorzugt für Sauerstoff.

In einer hervorgehobenen Gruppe von Verbindungen der Formel (I) stehen D¹-Z-D² für -CH₂-CH₂-.

In einer weiteren hervorgehobenen Gruppe von Verbindungen der Formel (I) stehen D¹-Z-D² für -HC=CH-.

In einer weiteren hervorgehobenen Gruppe von Verbindungen der Formel (I) stehen D¹-Z-D² für -CH₂-CH₂-CH₂-.

In einer weiteren hervorgehobenen Gruppe von Verbindungen der Formel (I) stehen D¹-Z-D² für -CH₂-C(=CH₂)CH₂-.

In einer weiteren hervorgehobenen Gruppe von Verbindungen der Formel (I) stehen D¹-Z-D² für -CH₂-O-CH₂-.

In einer weiteren hervorgehobenen Gruppe von Verbindungen der Formel (I) stehen D¹-Z-D² für -CH₂-CH₂-O-.

In einer weiteren hervorgehobenen Gruppe von Verbindungen der Formel (I) stehen D¹-Z-D² für -CH₂-N(CH₃)-CH₂-.

In einer weiteren hervorgehobenen Gruppe von Verbindungen der Formel (I) stehen D¹-Z-D² für -CH₂-CH₂-CH=.

In einer weiteren hervorgehobenen Gruppe von Verbindungen der Formel (I) stehen D¹-Z-D² für -CH₂-CH₂-CH(OH)-.

In einer weiteren hervorgehobenen Gruppe von Verbindungen der Formel (I) stehen D¹-Z-D² für -CH₂-CHF-CH₂-.

In einer weiteren hervorgehobenen Gruppe von Verbindungen der Formel (I) stehen D¹-Z-D² für -CH₂-CHCl-CH₂-.

In einer weiteren hervorgehobenen Gruppe von Verbindungen der Formel (I) steht A für 6-Chlor-pyridin-3-yl,

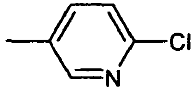

In einer weiteren hervorgehobenen Gruppe von Verbindungen der Formel (I) steht A für 6-Brom-pyridin-3-yl,

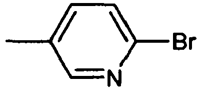

In einer weiteren hervorgehobenen Gruppe von Verbindungen der Formel (I) steht A für 2-Chlor-pyrimidin-5-yl,

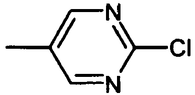

In einer weiteren hervorgehobenen Gruppe von Verbindungen der Formel (I) steht A für 5-Fluor-6-chlor-pyrid-3-yl,

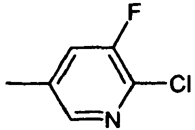

In einer weiteren hervorgehobenen Gruppe von Verbindungen der Formel (I) steht A für 2-Chlor-1,3-thiazol-5-yl,

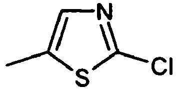

In einer weiteren hervorgehobenen Gruppe von Verbindungen der Formel (I) steht A für 5,6-Dichlor-pyrid-3-yl

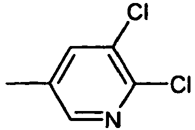

In einer weiteren hervorgehobenen Gruppe von Verbindungen der Formel (I) steht A für 5-Brom-6-Chlor-pyrid-3-yl

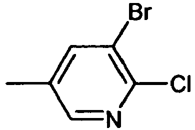

In einer weiteren hervorgehobenen Gruppe von Verbindungen der Formel (I) steht A für 5-Methyl-6-chlor-pyrid-3-yl

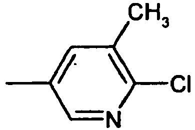

In einer weiteren hervorgehobenen Gruppe von Verbindungen der Formel (I) steht A für 5-Fluor-6-brom-pyrid-3-yl

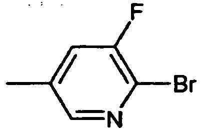

In einer weiteren hervorgehobenen Gruppe von Verbindungen der Formel (I) steht A für 5-Chlor-6-brom-pyrid-3-yl

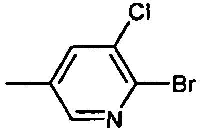

In einer weiteren hervorgehobenen Gruppe von Verbindungen der Formel (I) steht A für 5-Chlor-6-iod-pyrid-3-yl

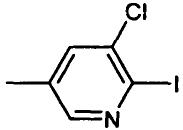

In einer weiteren hervorgehobenen Gruppe von Verbindungen der Formel (I) stehen R¹ und R² für Wasserstoff, E für Kohlenstoff, B und Q für Sauerstoff, A für 6-Chlor-pyrid-3-yl,

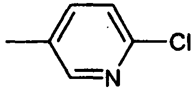

In einer weiteren hervorgehobenen Gruppe von Verbindungen der Formel (I) stehen R¹ und R² für Wasserstoff, E für Kohlenstoff, B und Q für Sauerstoff, A für 6-Brom-pyrid-3-yl,

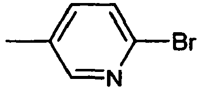

In einer weiteren hervorgehobenen Gruppe von Verbindungen der Formel (I) stehen R' und R² für Wasserstoff, E für Kohlenstoff, B und Q für Sauerstoff, A für 2-Chlor-pyrimidin-5-yl,

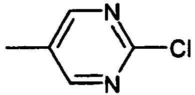

In einer weiteren hervorgehobenen Gruppe von Verbindungen der Formel (I) stehen R¹ und R² für Wasserstoff, E für Kohlenstoff, B und Q für Sauerstoff, A für 5-Fluor-6-chlor-pyrid-3-yl,

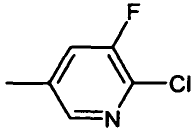

In einer weiteren hervorgehobenen Gruppe von Verbindungen der Formel (I) stehen R¹ und R² für Wasserstoff, E für Kohlenstoff, B und Q für Sauerstoff, A für 2-Chlor-1,3-thiazol-5-yl,

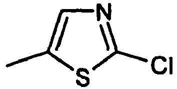

In einer weiteren hervorgehobenen Gruppe von Verbindungen der Formel (I) stehen R¹ und R² für Wasserstoff, E für Kohlenstoff, B und Q für Sauerstoff, A für 5,6-Dichlor-pyrid-3-yl

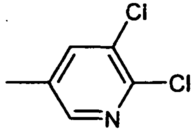

In einer weiteren hervorgehobenen Gruppe von Verbindungen der Formel (I) stehen R¹ und R² für Wasserstoff, E für Kohlenstoff, B und Q für Sauerstoff, A für 5-Brom-6-Chlor-pyrid-3-yl

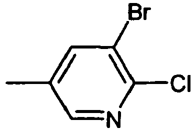

In einer weiteren hervorgehobenen Gruppe von Verbindungen der Formel (I) stehen R¹ und R² für Wasserstoff, E für Kohlenstoff, B und Q für Sauerstoff, A für 5-Methyl-6-chlor-pyrid-3-yl

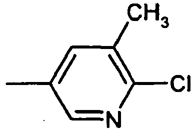

In einer weiteren hervorgehobenen Gruppe von Verbindungen der Formel (I) stehen R¹ und R² für Wasserstoff, E für Kohlenstoff, B und Q für Sauerstoff, A für 5-Fluor-6-brom-pyrid-3-yl

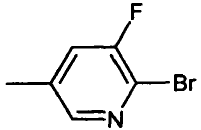

In einer weiteren hervorgehobenen Gruppe von Verbindungen der Formel (I) stehen R¹ und R² für Wasserstoff, E für Kohlenstoff, B und Q für Sauerstoff, A für 5-Chlor-6-brom-pyrid-3-yl

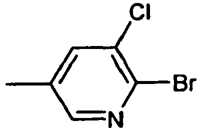

In einer weiteren hervorgehobenen Gruppe von Verbindungen der Formel (I) stehen R¹ und R² für Wasserstoff, E für Kohlenstoff, B und Q für Sauerstoff, A für 5-Chlor-6-iod-pyrid-3-yl

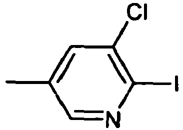

In einer weiteren hervorgehobenen Gruppe von Verbindungen der Formel (I) stehen R¹ und R² für Wasserstoff, D¹-Z-D² für -(CH₂)₃-, E für Kohlenstoff, B und Q für Sauerstoff, A für 6-Chlor-pyrid-3-yl,

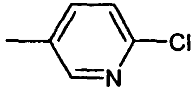

In einer weiteren hervorgehobenen Gruppe von Verbindungen der Formel (I) stehen R¹ und R² für Wasserstoff, D¹-Z-D² für -(CH₂)₃-, E für Kohlenstoff, B und Q für Sauerstoff, A für 6-Brom-pyrid-3-yl,

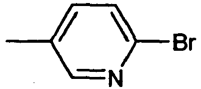

In einer weiteren hervorgehobenen Gruppe von Verbindungen der Formel (I) stehen R¹ und R² für Wasserstoff, D¹-Z-D² für -(CH₂)₃-, E für Kohlenstoff, B und Q für Sauerstoff, A für 2-Chlor-pyrimidin-5-yl,

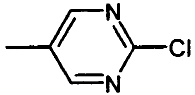

In einer weiteren hervorgehobenen Gruppe von Verbindungen der Formel **(I)** stehen R¹ und R² für Wasserstoff, D¹-Z-D² für -(CH₂)₃-, E für Kohlenstoff, B und Q für Sauerstoff, A für 5-Fluor-6-chlor-pyrid-3-yl,

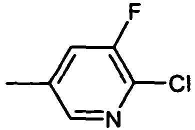

In einer weiteren hervorgehobenen Gruppe von Verbindungen der Formel (I) stehen R¹ und R² für Wasserstoff, D¹-Z-D² für -(CH₂)₃-, E für Kohlenstoff, B und Q für Sauerstoff, A für 2-Chlor-1,3-thiazol-5-yl,

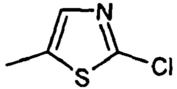

In einer weiteren hervorgehobenen Gruppe von Verbindungen der Formel (I) stehen R¹ und R² für Wasserstoff, D¹-Z-D² für -(CH₂)₃-, E für Kohlenstoff, B und Q für Sauerstoff, A für 5,6-Dichlor-pyrid-3-yl

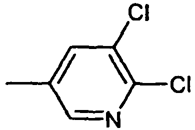

In einer weiteren hervorgehobenen Gruppe von Verbindungen der Formel (I) stehen R¹ und R² für Wasserstoff, D¹-Z-D² für -(CH₂)₃-, E für Kohlenstoff, B und Q für Sauerstoff, A für 5-Brom-6-Chlor-pyrid-3-yl

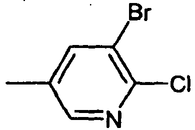

In einer weiteren hervorgehobenen Gruppe von Verbindungen der Formel (I) stehen R¹ und R² für Wasserstoff, D¹-Z-D² für -(CH₂)₃-, E für Kohlenstoff, B und Q für Sauerstoff, A für 5-Methyl-6-chlor-pyrid-3-yl

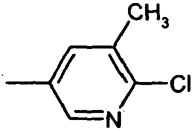

In einer weiteren hervorgehobenen Gruppe von Verbindungen der Formel (I) stehen R¹ und R² für Wasserstoff, D¹-Z-D² für -(CH₂)₃-, E für Kohlenstoff, B und Q für Sauerstoff, A für 5-Fluor-6-brom-pyrid-3-yl

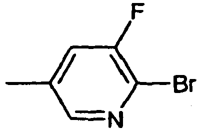

In einer weiteren hervorgehobenen Gruppe von Verbindungen der Formel (I) stehen R¹ und R² für Wasserstoff, D¹-Z-D² für -(CH₂)₃-, E für Kohlenstoff, B und Q für Sauerstoff, A für 5-Chlor-6-brom-pyrid-3-yl

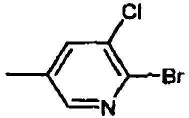

In einer weiteren hervorgehobenen Gruppe von Verbindungen der Formel (I) stehen R¹ und R² für Wasserstoff, D¹-Z-D² für -(CH₂)₃-, E für Kohlenstoff, B und Q für Sauerstoff, A für 5-Chlor-6-iod-pyrid-3-yl

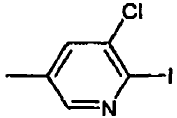

Die oben aufgeführten allgemeinen oder in Vorzugsbereiche aufgeführten Reste-Definitionen bzw. Erläuterungen gelten für die Endprodukte und für die Ausgangsprodukte und Zwischenprodukte entsprechend, Diese Restedefinitionen können untereinander also auch zwischen den jeweiligen Vorzugsbereichen, beliebig kombiniert werden.

bevorzugt werden Verbindungen der Formel (I), in welchen eine Kombination der vorstehend als bevorzugt aufgeführten Bedeutungen vorliegt.

besonders bevorzugt werden Verbindungen der Formel (I), in welchen eine Kombination der vorstehend als besonders bevorzugt aufgeführten Bedeutungen vorliegt.

ganz besonders bevorzugt werden Verbindungen der Formel (I), in welchen eine Kombination der vorstehend als ganz besonders bevorzugt aufgeführten Bedeutungen vorliegt.

Setzt man bei dem Verfahren 1 zur Herstellung der neuen Verbindungen der allgemeinen Formel (I) als Verbindungen der Formel (II) beispielsweise 2-{[6-Chlorpyridin-3-yl]methyl]amino}ethanol (Weg A) oder das 4-[[(6-Chlorpyridin-3-yl)methyl](3-iodpropyl)amino]-furan-2(5H)-on (Weg B) ein, so lässt sich das Herstellungsverfahren 1 durch folgendes Reaktionsschema I wiedergeben:

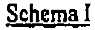

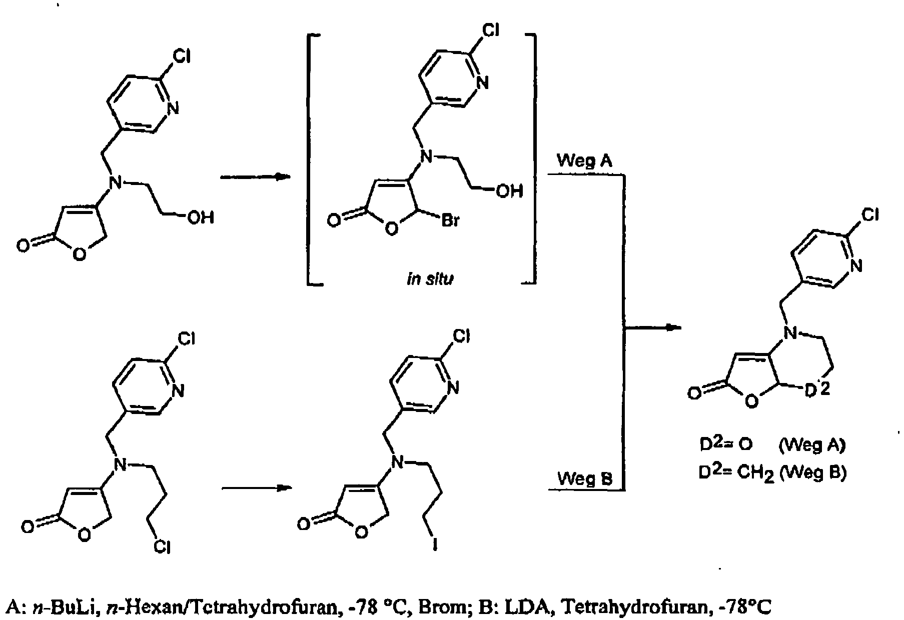

Die zur Herstellung des Verfahrens 1 als Ausgangsstoffe benötigten Verbindungen sind durch die Formel (II) allgemein definiert.

In dieser Formel (II) stehen A, B, E, Z, R¹, R², R³, R⁴, D¹, D², Q vorzugsweise für diejenigen Reste, die bereits im Zusammenhang mit der Beschreibung der Stoffe der allgemeinen Formel (I) als bevorzugte Substituenten genannt werden.

Die Verbindungen der allgemeinen Formel (II) können nach literaturbekannten Methoden (z.B. EP 0539588 A1) nach Reaktionsschcma II erhalten werden.

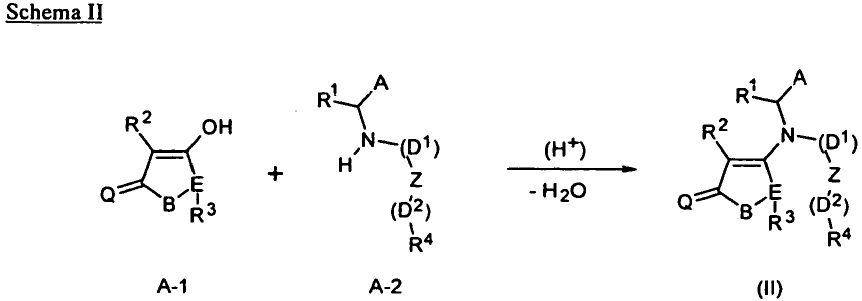

Geeignete Ausgangsverbindungen (A-1) zur Darstellung der Verbindungen der allgemeinen Formel (II) sind Tetronsäuren (B, Q = Sauerstoff, E-R³ = CH-R³ (Said, A. Speciality Chemicals Magazine (1984), 4(4), 7-8; Rao, Y. S. Chem. Rev. (1976), 76, 625-694; Tejedor, D.; Garcia-Tellado, F. Org. Preparations and Procedures International (2004), 36, 35-59; Reviews); B für Schwefel und Q für Sauerstoff steht: Thiotetronsäuren (Thomas, E. J. Special Publication - Royal Society of Chemistry (1988), 65 (Top. Med. Chem.), 284-307, Review), B für Methylen und Q für Sauerstoff steht: Cyclopentan-1,3-dione (Schick, Hans; Eichhorn, Inge. Synthesis (1989), (7), 477-492, Review).

Weitere geeignete Ausgangsverbindungen (A-2) zur Darstellung der Verbindungen der allgemeinen Formel (II) sind sekundäre Amine (D¹ = gegebenenfalls substituierter Kohlenstoff), N,O-disubstituierte Hydroxylamine (D¹ = Sauerstoff) oder N,N'-disubstituierte Hydrazine (D¹ = gegebenenfalls substituierter Stickstoff). Diese Ausgangsverbindungen können nach literaturbekannten Methoden erhalten werden (vgl. z. B. S. Patai "The Chemistry of Amino Group", Interscience Publishers, New York, 1968).

Aminoxyverbindungen, die als Startkomponenten für die Verbindungen (A-2; D¹ = Sauerstoff) fungieren, sind z. T. kommerziell erhältlich und können nach bekannten Methoden erhalten werden. Ein allgemeiner Weg zur Herstellung von Aminoxyverbindungen besteht beispielsweise darin, dass ein Hydroxylaminderivat, welches am Stickstoff eine Schutzgruppe (SG) aufweist (z. B. R" und R'" zusammen: Phthaloyl, Isopropyliden, α-Hydroxy-benzyliden-Gruppe), mit einer Verbindung R^{iv}-LG (O-Alkylierung; R^{iv} steht für ggf. substituiertes Alkyl) in einem Verdünnungsmittel umgesetzt wird and anschließend eine Abspaltung der entsprechenden Schutzgruppe erfolgt. In Verbindung R^{iv}-LG hat R^{iv} die gleiche Bedeutung wie oben und LG steht für eine nucleofuge Abgangsgruppe (Leaving Group), beispielsweise aliphatisch oder aromatisch substituiertes Sulfonyloxy, z.B. Methansulfonyloxy (MesO=Mesyloxy), Salze der Sulfonsäure, *para*-Toluolsulfonyloxy (TosO=Tosyloxy), ferner aber auch z. B. Halogen, insbesondere Brom, Chlor oder Iod (vgl. O-Alkylierung). Im nachfolgenden Reaktionsschema III wird die Darstellung von Aminoxyverbindungen wiedergegeben:

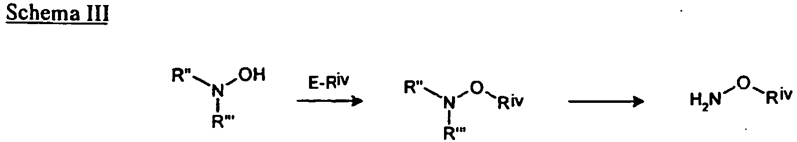

Alternativ kann man bei Verwendung von Hydroxyverbindungen (R^{iv}-OH) z. B. eine intermolekulare Dehydratisierungsreaktion durchführen. Insbesondere kommt dafür eine Variante der Mitsunobu-Reaktion (O. Mitsunobu et al., Synthesis 1981, 1-28) in Frage, bei der die Hydroxyverbindung mit N-geschützten Hydroxylaminderivaten, wie z. B. N-Hydroxyphthalimid, N-Hydroxy-5-norbomen-2,3-dicarbonsäureimid oder Acethydroxamsäureethylester, und z. B. Triphenylphosphin und N,N'-Azodicarbonsäurediethylester.

Die Freisetzung der Aminoxyverbindungen kann unter literaturbekannten Bedingungen, zweckmäßig in folgender Weise durchgeführt werden: Die Hydrazinolyse kann vorzugsweise in einem Verdünnungsmittel, beispielsweise Alkohol, bei Siedetemperatur erfolgen. Die Hydrolyse kann vorzugsweise in einer wässrigen, wässrig-alkoholischen oder alkoholischen Lösung durch mehrstündiges Erhitzen durchgeführt werden. Im Falle, dass R" und R'" zusammen eine Isopropylidengruppe bedeuten, kann von der sauren Hydrolyse und, im Falle dass R" und R'" zusammen eine α-Hydroxy-benzyliden-Gruppe bedeuten oder R'" eine Carbethoxygruppe bedeuten, kann sowohl von der alkalischen als auch sauren Hydrolyse Gebrauch gemacht werden.

Zur Darstellung der Ausgangsverbindungen (A-2) ist es vorteilhaft, dass man beispielsweise Verbindungen der allgemeinen Formel (A-3), in der A die weiter oben genannte Bedeutung hat und LG für eine geeignete Abgangsgruppe steht (z. B. Chlor, Brom, Iod, O-Tosyl, O-Mesyl), mit Verbindungen der allgemeinen Formel (A-4), in der R' für den Rest (D¹)-Z-(D²)-R⁴ steht, in welchem D¹, D² und R⁴ die weiter oben genannten Bedeutungen haben, gegebenenfalls in Gegenwart von Verdünnungsmitteln und gegebenenfalls in Gegenwart von den im Herstellungsverfahren 2 genannten basischen Reaktionshilfsmitteln umsetzt (vgl. Schema III).

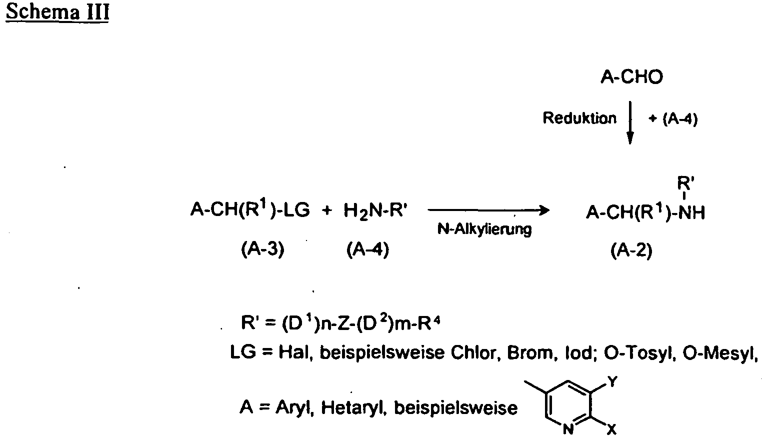

Alternativ und in bestimmten Fällen ist aber auch eine Herstellung von Ausgangsverbindungen (A-2), in der R' für Wasserstoff steht, aus den entsprechenden Aldehyden (A-CHO) und den Verbindungen (A-4) mittels reduktiver Aminierung möglich (vgl. Houben-Weyl, Methoden der Organischen Chemie, Bd. XI/1, Georg Thieme Verlag Stuttgart, S. 602).

Allgemeine Wege zur Herstellung der Ausgangsverbindungen (A-3) sind im Reaktionsschema IV wiedergegeben.

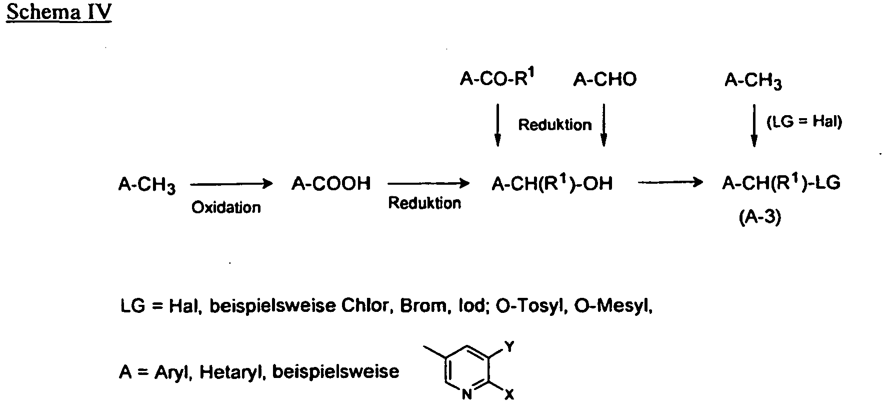

Die Verbindungen (A-3, R¹ = Wasserstoff) sind z. T. kommerziell erhältlich, teilweise bekannt oder können nach bekannten Methoden erhalten werden (z. B. 2-Chlor-5-chlormethyl-1,3-thiazol: DE 3 631 538 (1988), EP 446 913 (1991), EP 780 384 (1997), EP 775 700 (1997), EP 794 180 (1997), WO 9 710 226 (1997); 6-Chlor-3-chlormethyl-pyridin: DE 3 630 046 A1 (1988), EP 373 464 A2 (1990), EP 373 464 A2 (1990), EP 393 453 A2 (1990), EP 569 947 A1 (1993); 6-Chlor-3-brommethyl-pyridin: I. Cabanal-Duvillard et al., Heterocycl. Commun. 5,257-262 (1999); 6-Brom-3-chlormethyl-pyridin, 6-Brom-3-hydroxymethyl-pyridin: US-Pat. 5 420 270 A (1995); 6-Fluor-3-chlormethyl-pyridin: J. A. Pesti et al., J. Org. Chem. 65, 7718-7722 (2000); 2-Methyl-3-chlormethyl-pyridin: EP 302 389 A2 (1989); 2-Trifluormethyl-3-chlormethyl-pyridin: WO 2004082616 A2 (2004); 3-Chlor-6-chlormethyl-pyridazin: EP 284 174 A1 (1988); 2-Chlor-5-pyrazinylmethylchlorid: J. Heterocycl. Chem. 23, 149-151 (1986) 2-Chlor-5-pyrazinylmethylbromid: JP 05 239 034 A2 (1993);

Methylsubstituierte Aromaten oder Heterocyclen (A-CH₃) lassen sich beispielsweise durch Oxidation in entsprechende aromatische oder heterocyclische Carbonsäuren (A-COOH, z. B. 5-Fluor-6-brom-nicotinsäure: F. L. Setliff, G. O. Rankin, J. Chem. Eng. Data (1972), 17, 515-516; 5-Chlor-6-brom-nicotinsäure und 5,6-Dibrom-nicotinsäure: F. L. Setliff et al., J. Chem. Eng. Data (1981), 26, 332-333; 5-lod-6-brom-nicotinsäure: F. L. Setliff et al., J. Chem. Eng. Data (1978), 23, 96-97, 5-Fluor-6-iod-nicotinsäure und 5-Brom-6-iod-nicotinsäure: F. L. Setliff et al., J. Chem. Eng. Data (1973), 18, 449-450, 5-Chlor-6-iod-nicotinsäure: F. L. Setliff, J. E. Lane J. Chem. Eng. Data (1976), 21, 246-247) oder Carbonsäureester (z. B. 5-Methyl-6-fluor-nicotinsäuremethylester: WO 9833772 A1, 1998; 5-Methyl-6-brom-nicotinsäure-methylester: WO 9730032 A1, 1997) überführen. Des Weiteren ist die Synthese von Formylgruppe-haltigen Aromaten oder Heterocyclen (A-CHO, z. B. 6-Chlor-3-formyl-5-methyl-pyridin: DE 4429465 A1, 1996) aus nicht cyclischen Ausgangskomponenten vorbeschrieben; diese kann beispielsweise mittels 1,3-dipolarer Cycloaddition (z. B.: 5-Chlormethyl-3-brom-isoxazol: P. Pevarello, M. Varasi Synth. Commun. (1992), 22, 1939-1948) erfolgen.

Die aromatischen oder heterocyclische Carbonsäuren (A-COOH) oder Alkylcarbonyl Verbindungen (A-CO-R¹; R¹ = Alkyl) können dann nach literaturbekannten Methoden in die entsprechenden aromatischen oder heterocyclischen Hydroxyalkyl-Verbindungen (A-CH(R¹)-OH; R¹ = H, Alkyl) überführt werden, die anschließend nach literaturbekannten Methoden zu aktivierten aromatischen oder heterocyclischen Hydroxymethylverbindungen (A- CH(R¹)-LG, LG = OTosyl, OMesyl) bzw. aromatischen oder heterocyclischen Halogenmethylverbindungen (A- CH(R¹)-LG, LG = Hal) umgesetzt werden. Letztere können auch aus entsprechenden Methylgruppe-haltigen Aromaten oder Heterocyclen (A-CH₃) unter Verwendung von geeigneten literaturbekannten Halogenierungsmitteln erhalten werden. Als Beispiele für diese Vorgehensweise seien die Synthesen der Halogenmethyl-substituierten Heterocyclen genannt: 5-Chlormethyl-2-methyl-pyrimidin (U. Eiermann et al., Chem. Ber. (1990), 123, 1885-9); 3-Chlomethyl-5-brom-6-chlor-pyridin, 3-Brom-5-iod-6-chlor-pyridin (S. Kagabu et al., J. Pestic. Sci. (2005), 30, 409-413).

Ausgangsverbindungen (A-10) in denen A für einen 5,6-disubstituierten Pyrid-3-yl Rest steht können ebenso nach literaturbekannten Methoden erhalten werden. Beispielsweise sind geeignete und literaturbekannte Ausgangsverbindungen die 6-halogen-substituierten 5-Nitro-β-picoline (A-5), die entsprechend bekannter Literaturvorschriften modifiziert werden können, wie im Reaktionsschema V gezeigt.

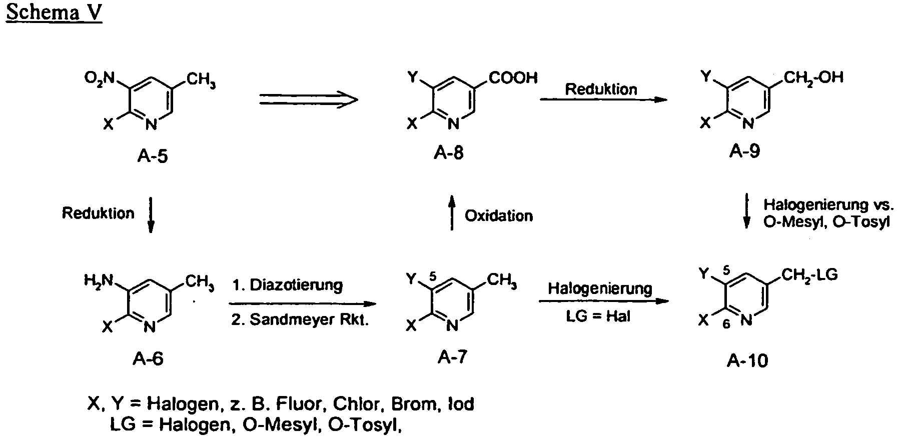

Beispielsweise führt die Reduktion der Nitrogruppe in 6-halogen-substituierten 5-Nitro-β-picolinen (A-5) zu 6-halogen-substituierten 5-Amino-β-picolinen (A-6, z. B. 5-Amino-6-chlor-β-picolin und 5-Amino-6-brom-β-picolin: Setliff, F. L. Org. Preparations and Preparations Int. (1971), 3, 217-222; Kagabu, S. et al. J. Pestic. Sci. (2005), 30, 409-413). Durch nachfolgende Diazotierung sowie Sandmeyer-Reaktion (C. F. H. Allen, J. R. Thirtle, Org. Synth., Coll. Vol. III, 1955, S. 136) ist die Einführung von Halogensubstituenten in 5-Position möglich (A-7, z. B. 5-Fluor-6-chlor-β-picolin und 5-Fluor-6-brom-β-picolin: Setliff, F. L. Org. Preparations and Preparations Int. (1971), 3, 217-222; 5-Iod-6-chlor-β-picolin: Kagabu, S. et al. J. Pestic. Sci. (2005), 30, 409-413; 5,6-Dichlor-picolin: Setliff, F. L.; Lane, J. E. J. Chem. Engineering Data (1976), 21, 246-247). Die Oxidation der Methylgruppe in den 5,6-disubstituierten β-Picolinen (A-7) führt dann zu den entsprechenden 5,6-disubstituierten Nicotinsäuren (A-8, z. B. 5-Fluor-6-chlor-nicotinsäure und 5-Fluor-6-brom-nicotinsäure: Setliff F. L., Rankin G. O. J. Chem. Engineering Data (1972), 17, 515-516; 5-Brom-6-fluor-nicotinsäure, 5-Brom-6-chlor-nicotinsäure und 5-Brom-6-brom-nicotinsäure: F. L. Setliff J. Chem. Engineering Data (1970), 15, 590-591; 5-Chlor-6-brom-nicotinsäure und 5-Iod-6-brom-nicotinsäure: Setliff, F. L., Greene, J. S. J. Chem. Engineering Data (1978), 23, 96-97; bekannt ist auch die 5-Chlor-6-trifluormethyl-nicotinsäure: F. Cottet et al., Synthesis (2004), 10, 1619-1624), die in Gegenwart von Reduktionsmitteln zu den entsprechenden hydroxymethylierten Pyridinen (A-9) umgewandelt werden können (z. B. 5-Brom-6-chlor-3-hydroxymethyl-pyridin: Kagabu, S. et al., J. Pestic. Sci. (2005), 30, 409-413).

Durch Verwendung von 6-Chlor-5-nitro-nicotinsäure (A-8, X=Cl, Y=NO₂; Boyer, J. H.; Schoen, W., J. Am. Chem. Soc. (1956), 78, 423-425) kann mittels Reduktion das 6-Chlor-3-hydroxymethyl-5-nitro-pyridin (A-9, X=Cl, Y=NO₂; Kagabu, S. et al., J. Med. Chem. (2000), 43, 5003-5009) gebildet werden, das nachfolgend zum 6-Chlor-3-hydroxymethyl-5-amino-pyridin (A-9, X=Cl, Y=NH₂; Kagabu, S. et al., J. Med. Chem. (2000), 43, 5003-5009) reduziert und über Diazotierung und Reaktion mit Hydroxylamin in das 6-Chlor-3-hydroxymethyl-5-azido-pyridin (A-9, X=C), Y=N₃; Kagabu, S. et al., J. Med. Chem. (2000), 43, 5003-5009) überführt wird. Nachfolgende Halogenierung mit Thionylchlorid ergibt das 6-Chlor-3-chlormethyl-5-azido-pyridin (VII, X=Cl, Y=N₃, LG=Cl; Kagabu, S. et al., J. Med. Chem. (2000), 43, 5003-5009).

Alternativ führt die Halogenierung der Methylgruppe in 3-Position von (A-7) zu den Verbindungen (A-10), in der LG für Halogen steht (z. B.: 3-Brommethyl-6-chlor-5-fluor-pyridin, 3-Brommethyl-6-chlor-5-iod-pyridin: Kagabu, S. et al. J. Pestic. Sci. (2005), 30, 409-413). Bei Verwendung von 6-halogen-substituierten 5-Nitro-β-picolinen (A-7; Y=NO₂) kann hierbei zunächst die Halogenierung der Methylgruppe in 3-Position erfolgen (z. B. 3-Brommethyl-6-chlor-5-nitro-pyridin: Kagabu, S. et al., J. Pestic. Sci. (2005), 30, 409-413). Die Nitrogruppe kann auch gegebenenfalls erst zu einem späteren Zeitpunkt in der Reaktionssequenz reduziert werden.

Literaturbekannt ist ebenso eine Substituenten Einführung in 5-Position (z. B. Y=N₃) bei Verbindungen (A-10), in der LG für *N*-Morpholino steht. Dieser Rest kann anschließend sehr einfach durch Halogen (LG=Hal) ersetzt werden (vgl. S. Kagabu et al., J. Med. Chem. 2000, 43, 5003-5009; Reaktionsbedingungen: Chlorameisensäureethylester, Tetrahydrofuran, 60°C).

Im Allgemeinen gelingt es, Halogenatome in Nachbarschaft zum Pyridinstickstoff durch andere Halogenatome oder halogenierte Gruppen wie beispielsweise Trifluormethyl, zu ersetzen (Transhalogenierung, z. B.: Chlor gegen Brom oder Iod; Brom gegen Iod oder Fluor; Iod gegen Fluor oder Trifluormethyl). Deshalb besteht ein weiterer alternativer Syntheseweg darin, das Halogenatom (beispielsweise X=Cl) in 6-Position des Pyrid-5-yl-restes (z. B. in A-8 mit X, Y=Cl; 5,6-Dichlor-nicotinsäure: Setliff, F. L.; Lane, J. E. J. Chem. Engineering Data (1976), 21, 246-247) gegen ein anderes Halogenatom, beispielsweise Iod oder Fluor, auszutauschen (z. B.: A-8 mit X=I; 5-Brom-6-iod-nicotinsäure und A-8 mit X=F; 5-Brom-6-fluor-nicotinsäure: Setliff, F. L.; Price, D. W. J. Chem. Engineering Data (1973), 18, 449-450) zu ersetzen. Diese Transhalogenierung kann aber auch gegebenenfalls erst in geeigneten Verbindungen der allgemeinen Formel (I) erfolgen.

Im Allgemeinen ist es vorteilhaft, das Herstellungsverfahren 1 gegebenenfalls in Gegenwart von Verdünnungsmitteln und gegebenenfalls in Gegenwart basischer Reaktionshilfsmittel durchzuführen.

Verdünnungsmittel werden vorteilhaft in einer solchen Menge eingesetzt, dass das Reaktionsgemisch während des ganzen Verfahrens gut rührbar bleibt. Als Verdünnungsmittel zur Durchführung des Verfahrens 1 kommen alle unter den Reaktionsbedingungen inerten organischen Lösungsmittel in Frage.

Als Beispiele sind zu nennen: Halogenkohlenwasserstoffe, insbesondere Chlorkohlenwasserstoffe, wie Tetrachlorethylen, Tetrachlorethan, Dichlorpropan; Methylenchlorid, Dichlorbutan, Chloroform, Tetrachlorkohlenstoff, Trichlorethan, Trichlorethylen, Pentachlorethan, Difluorbenzol, 1,2-Dichlorothan, Chlorbenzol, Brombenzol, Dichlorbenzol, Chlortoluol, Trichlorbenzol; Alkohole wie Methanol, Ethanol, Isopropanpol, Butanol; Ether wie Ethylpropylether, Methyl-tert-butylether, n-Butylether, Anisol, Phenetol, Cyclohexylmethylether, Dimethylether, Diethylether, Dipropylether, Diisopropylether, Di-n-butylether, Diisobutylether, Diisoamylether, Ethylenglycoldimethylether, Tetrahydrofuran, Dioxan, Dichlordiethylether und Polyether des Ethylenoxids und/oder Propylenoxids; Amine wie Trimethyl-, Triethyl-, Tripropyl-, Tributylamin, N-Methylmorpholin, Pyridin und Tetramethylendiamin; Nitrokohlenwasserstoffe wie Nitromethan, Nitroethan, Nitropropan, Nitrobenzol, Chlomitrobenzol, o-Nitrotoluol; Nitrile wie Acetonitril, Propionitril, Butyronitril, Isobutyronitril, Benzonitril, m-Chlorbenzonitril sowie Verbindungen wie Tetrahydrothiophendioxid und Dimethylsulfoxid, Tetramethylensulfoxid, Dipropylsulfoxid, Benzylmethylsulfoxid, Diisobutylsulfoxid, Dibutylsulfoxid, Diisoamylsulfoxid; Sulfone wie Dimethyl-, Diethyl-, Dipropyl-, Dibutyl-, Diphenyl-, Dihexyl-, Methylethyl-, Ethylpropyl-, Ethylisobutyl- und Pentamethylensulfon; aliphatische, cycloaliphatische oder aromatische Kohlenwasserstoffe wie Pentan, Hexan, Heptan, Oktan, Nonan und technische Kohlenwasserstoffe; beispielsweise sogenannte White Spirits mit Komponenten mit Siedepunkten im Bereich beispielsweise von 40°C bis 250°C, Cymol, Benzinfraktionen innerhalb eines Siedeintervalles von 70°C bis 190°C, Cyclohexan, Methylcyclohexan, Petrolether, Ligroin, Octan, Benzol, Toluol, Chlorbenzol, Brombenzol, Nitrobenzol, Xylol; Ester wie Methyl-, Ethyl-, Butyl-, Isobutylacetat, sowie Dimethyl-, Dibutyl-, Ethylencarbonat; Amide wie Hexamethylenphosphorsäuretriamid, Formamid, *N*-Methyl-formamid, *N,N*-Dimethyl-formamid, *N,N*-Dipropyl-formamid, *N,N-*Dibutyl-formanid, *N*-Methyl-pyrrolidin, *N*-Methyl-caprolactam, 1,3-Dimethyl-3,4,5,6-tetrahydro-2(1H)-pyrimidin, Octylpyrrolidon, Octylcaprolactam, 1,3-Dimethyl-2-imidazolindion, *N*-Formyl-piperidin, *N,N'*-1,4-Diformyl-piperazin; Ketone wie Aceton, Acetophenon, Methylethylketon, Methylbutylketon.

Selbstverständlich kann man in das erfindungsgemäße Verfahren auch Gemische der genannten Lösungs- und Verdünnungsmittel einsetzen.

Bevorzugte Verdünnungsmittel zur Durchführung des erfindungsgemäßen Herstellungsverfahrens sind jedoch Ether wie Ethylpropylether, Methyl-*tert*-butylether, n-Butylether, Anisol, Phenetol, Cyclohexylmethylether, Dimethylether, Diethylether, Dipropylether, Diisopropylether, Di-n-butylether, Diisobutylether, Diisoamylether, Ethylenglycoldimethylether, Tetrahydrofuran, Dioxan, Dichlordiethylether und Polyether des Ethylenoxids und/oder Propylenoxids, bevorzugt sind Tetrahydrofuran und Dioxan sowie cycloaliphatische oder aromatische Kohlenwasserstoffe wie Pentan, Hexan, Heptan, Oktan, Nonan und technische Kohlenwasserstoffe; beispielsweise sogenannte White Spirits mit Komponenten mit Siedepunkten im Bereich beispielsweise von 40°C bis 250 °C, bevorzugt sind Pentan und Hexan.

Als basische Reaktionshilfsmittel zur Durchführung des Herstellungsverfahrens 1 können alle geeigneten Säurebindemittel eingesetzt werden wie Amine, insbesondere tertiäre Amine sowie Alkali- und Erdalkaliverbindungen.

Beispielhaft seien dafür erwähnt die Hydroxide, Hydride, Oxide und Carbonate des Lithiums, Natriums, Kaliums, Magnesiums, Calciums und Bariums, ferner weitere basische Verbindungen wie Amidinbasen oder Guanidinbasen wie 7-Methyl-1,5,7-triaza-bicyclo(4,4,0)dec-5-en (MTBD); Diazabicyclo(4.3.0)nonen (DBN), Diazabicyclo (2.2.2)octan (DABCO), 1,8-Diazabicyclo(5.4.4.0)-undecen (DBU), Cyclohexyltetrabutyl-guanidin (CyTBG), Cyclohexyltetramethylguanidin (CyTMG), *N,N,N,N*-Tetramethyl-1,8-naphthalindiamin, Pentamethylpiperidin, tertiäre Amine wie Triethylamin, Triethylamin, Tribenzylamin, Triisopropylamin, Tributylamin, Tricyclohexylamin, Triamylamin, Trihexylamin, *N,N*-Dimethylanilin, *N,N*-Dimethyl-toluidin, *N,N-*Dimethyl-p-aminopyridin, *N-*Methyl-pyrrolidin, N-Methyl-piperidin, N-Methyl-imidazol, *N-*Methyl-pyrazol, *N*-Methyl-morpholin, *N*-Methyl-hexamethylendiamin, Pyridin, 4-Pyrrolidinopyridin, 4-Dimethylamino-pyridin, Chinolin, α-Picolin, β-Picolin, Isochinolin, Pyrimidin, Acridin, *N,N,N',N'*-Tetramethylendiamin, *N,N,N,N'*-Tetraethylendiamin, Chinoxalin, *N*-Propyl-diisopropylamin, *N*-Ethyldiisopropylamin, *N,N'*-Dimethyl-cyclohexylamin, 2,6-Lutidin, 2,4-Lutidin oder Triethyldiamin.

Vorzugsweise findet Lithiumdiisopropylamid (LDA) Verwendung.

Die Umsetzung von Verbindungen der allgemeinen Formel (II) nach dem Herstellungsnrfahren 1 wird durchgeführt indem man die Verbindungen der allgemeinen Formel (II):
Weg A: in Gegenwart eines basischen Reaktionshilfsmittels, beispielsweise
   n-Butyllithium in einem der angegebenen Verdünnungsmittel umsetzt und
   bei -78 °C halogeniert, bevorzugt mit Brom.
Weg B: in Gegenwart eines basischen Reaktionshilfsmittels, beispielsweise
   Lithiumdiisopropylamid (LDA) in einem der angegebenen Verdünnungsmittel
   umsetzt.

Die Reaktionsdauer beträgt 5 Minuten bis 48 Stunden. Die Umsetzung erfolgt bei Temperaturen zwischen -100°C und +200°C, bevorzugt zwischen -90°C und 150°C, besonders bevorzugt zwischen -80°C und 100°C.

Es kann grundsätzlich unter Normaldruck gearbeitet werden. Vorzugsweise arbeitet man bei Normaldruck oder bei Drucken bis zu 15 bar und gegebenenfalls unter Schutzgasatmosphäre (Stickstoff, Helium oder Argon).

Zur Durchführung des Herstellungsverfahrens 1 setzt man pro Mol Verbindung der allgemeinen Formel (II) im allgemeinen 0,5 bis 4,0 Mol, vorzugsweise 0,7 bis 3,0 Mol, besonders bevorzugt 1,0 bis 2,0 Mol an basischem Reaktionshilfsmittel (Wege A und B) und Halogen, insbesondere Brom (Weg B), ein.

Nach vollendeter Umsetzung wird der gesamte Reaktionsansatz eingeengt. Die nach Aufarbeitung anfallenden Produkte lassen sich in üblicher Weise durch Umkristellisieren, Vakuumdestillation oder Säulenchromatographie reinigen (vgl. auch die Herstellungsbeispiele).

Racemate oder Diastereomengemische können mittels Säulenchromatographie, ggf. an einer geeigneten chiralen Phase, in die entsprechenden Enantiomere oder einzelnen Diastereomere aufgetrennt werden. Die Bestimmung der absoluten Konfiguration ist nach Kristallisation der Substanz mit Hilfe der Röntgenstrukturanalyse möglich (vgl. auch die Herstellungsbeispiele).

Setzt man bei dem Herstellungsverfahrens 2 zur Herstellung der neuen Verbindungen der allgemeinen Formel (I) als Verbindungen der Formel (III) beispielsweise 5-[2-(Chlormethyl)prop-2-en-1-yl]-4-pyrrolidin-1-ylfuran-2(5H)-on und als Verbindung der allgemeinen Formel (IV) 3-Aminomethyl-6-chlor-pyridin ein, so können diese in einem ersten Reaktionsschritt zu 5-[2-({[6-Chlorpyridin-3-yl]methyl]amino}methyl)prop-2-en-1-yl]-4-pyrrolidin-1-yl-furan-2(5H)-on als Verbindung der allgemeinen Formel (V) reagieren, die dann im zweiten Reaktionsschritt intramolekular cyclisieren (Reaktionsschema VI):

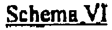

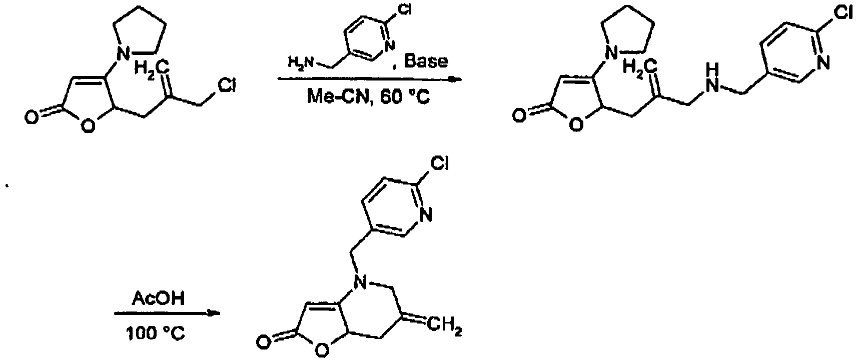

Die zur Herstellung des Herstellungsveffahrens 2 als Ausgangsstoffe benötigten Verbindungen sind durch die Formel (III) und (IV) allgemein definiert.

In diesen Formeln (III) und (IV) stehen A, B, E, Z, LG, R¹, R², R⁴, D¹, D², n, m, Q, vorzugsweise für diejenigen Reste, die bereits im Zusammenhang mit der Beschreibung der Stoffe der allgemeinen Formel (I) als bevorzugte Substituenten genannt werden.

Die insbesondere für die Durchführung des zweiten Reaktionsschritts des Herstellungsverfahrens 2 als Ausgangsstoffe zu verwendenden Verbindungen sind allgemein durch die Formel (V) definiert.

Die Verbindungen der allgemeinen Formel (V) können nach literaturbekannten Methoden erhalten werden, beispielsweise nach dem im Reaktionsschema VII gezeigten Herstellungsverfahren.

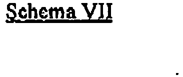

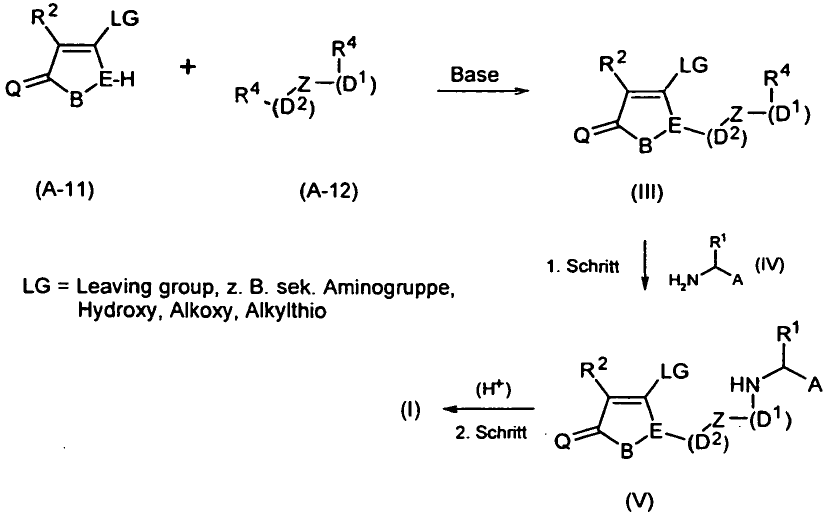

Geeignete Ausgangsverbindungen (A-11) zur Darstellung der Verbindungen der allgemeinen Formel (III) sind beispielsweise 4-Amino-furan-2(5H)-one (B, Q = Sauerstoff, E-R³ = CH₂, LG = N-Pyrrolidinyl, Methoxy; vgl. Shandala, M. Y. et al. J. Heterocycl. Chem. (1984), 21, 1753-1754; Momose T. et al. Heterocycles (1988), 27, 1907-1923).

Im Allgemeinen ist es vorteilhaft, die Reaktion Verbindungen (A-11) und (A-12) zu Verbindungen der allgemeinen Formel (III) gegebenenfalls in Gegenwart von Verdünnungsmitteln durchzuführen und gegebenenfalls in Gegenwart von basischen Reaktionshilfsmitteln durchzuführen.

Verdünnungsmittel werden vorteilhaft in einer solchen Menge eingesetzt, dass das Reaktionsgemisch während des ganzen Verfahrens gut rührbar bleibt. Als Verdünnungsmittel zur Durchführung des erfindungsgemäßen Verfahrens 2 kommen alle unter den Reaktionsbedingungen inerten organischen Lösungsmittel in Frage.

Bevorzugte Verdünnungsmittel zur Durchführung des ersten Reaktionsschrittes des erfindungsgemäßen Herstellungsverfahrens sind Ether wie Ethylpropylether, Methyl-tert-butylether, n-Butylether, Dipropylether, Diisopropylether, Di-n-butylether, Diisobutylether, Diisoamylether, Ethylenglycoldimethylether, Tetrahydrofuran, Dioxan, bevorzugt Tetrahydrofuran und Dioxan sowie aliphatische Kohlenwasserstoffe wie Pentan, Hexan oder Heptan.

Als bevorzugtes basisches Reaktionshilfsmittel zur Herstellung der Verbindungen der allgemeinen Formel (III) findet tert.-Butyllithium Verwendung.

Die Reaktionsdauer beträgt 10 Minuten bis 48 Stunden. Die Umsetzung erfolgt bei Temperaturen zwischen -100 °C und +140 °C, bevorzugt zwischen -90 °C und 120 °C, besonders bevorzugt zwischen -80 °C und 100 °C. Es kann grundsätzlich unter Normaldruck gearbeitet werden. Vorzugsweise arbeitet man bei Normaldruck oder bei Drucken bis zu 15 bar und gegebenenfalls unter Schutzgasatmosphäre (Stickstoff, Helium oder Argon).

Im Allgemeinen ist es vorteilhaft, den ersten Reaktionsschritt des Herstellungsverfahrens 2 gegebenenfalls in Gegenwart von Verdünnungsmitteln und gegebenenfalls in Gegenwart von basischen Reaktionshilfsmitteln durchzuführen.

Bevorzugte Verdünnungsmittel zur Durchführung des ersten Reaktionsschrittes des erfindungsgemäßen Herstellungsverfahrens sind Nitrile wie Acetonitril, Propionitril, Butyronitril, Isobutyronitril, Benzonitril, m-Chlorbenzonitril, besonders bevorzugt Acetonitril.

Selbstverständlich kann man für den ersten Reaktionsschritt des Herstellungsverfahrens 2 auch Gemische der genannten Lösung- und Verdünnungsmittel einsetzen.

Als bevorzugtes basisches Reaktionshilfsmittel zur Durchführung des ersten Reaktionsschrittes des Herstellungsverfahrens 2 finden tertiäre Amine wie Triethylamin, Trimethylamin, Tribenzylamin, Triisopropylamin, Tributylamin, Tricyclohexylamin, Triamylamin, Trihexylamin, N-Methyl-pyrrolidin oder N-Ethyl-N-isopropylpropan-2-amin, insbesondere N-Ethyl-N-isopropylpropan-2-amin Verwendung.

Die Reaktionsdauer beträgt 10 Minuten bis 48 Stunden. Die Umsetzung erfolgt bei Temperaturen zwischen -100°C und +140°C, bevorzugt zwischen -90°C und 120°C, besonders bevorzugt zwischen -80°C und 100°C. Es kann grundsätzlich unter Normaldruck gearbeitet werden. Vorzugsweise arbeitet man bei Normaldruck oder bei Drucken bis zu 15 bar und gegebenenfalls unter Schutzgasatmosphäre (Stickstoff, Helium oder Argon).

Zur Durchführung des zweiten Reaktionsschrittes des Herstellungsverfahrens 2 wird die Verbindung in Gegenwart eines sauren Reaktionshilfsmittels gegebenenfalls in Gegenwart eines Verdünnungsmittels intramolekular cyclisiert.

Als saure Reaktionshilfsmittel kommen dabei praktisch alle Mineralsäuren, organische Säuren oder Lewis Säuren in Frage. Zu den Mineralsäuren gehören vorzugsweise Halogenwasserstoffsäuren wie Fluorwasserstoffsäure, Chlorwasser-stoffsäure, Bromwasserstoffsäure oder Iodwasserstoffsäure sowie Schwefelsäure, Phosphorsäure, Phosphorige Säure, Salpetersäure und zu den Lewis Säure gehören vorzugsweise Aluminium(III)-chlorid, Bortrifluorid oder sein Etherat, Titan-(V)chlorid, Zinn(V)-chlorid. Zu den organischen Säuren gehören Ameisensäure, Essigsäure, Propionsäure, Malonsäure, Milchsäure, Oxalsäure, Fumarsäure, Adipinsäure, Stearinsäure, Weinsäure, Ölsäure, Methansulfonsäure, Benzoesäure, Benzolsulfonsäure oder para-Toluolsulfonsäure.

Bevorzugt wird die intramolekulare Cyclisierung in Gegenwart einer organischen Carbonsäure, beispielsweise Essigsäure durchgerührt.

Nach vollendeter Umsetzung wird der gesamte Reaktionsansatz eingeengt. Die nach Aufarbeitung anfallenden Produkte lassen sich in üblicher Weise durch Umkristallisieren, Vakuumdestillation oder Säulenchromatographie reinigen (vgl. auch die Herstellungsbeispiele).

Die Verbindungen der allgemeinen Formel (Ia), in der D¹-Z-D² als Gruppe für -CH₂-C(=CH₂)-CH₂-steht (vgl. z. B. Herstellungsverfahren 2, Schema VI), lassen sich beispielsweise nach literaturbekannten Methoden (vgl. Reaktionsschritte [A]-[F]) in Verbindungen der allgemeinen Formeln (Ib) bis (Ig), in denen D¹-Z-D² als Gruppe für -CH₂-CH(CH₃)-CH₂- (Ib), -CH₂-COH(CH₂-OH)-CH₂- (Ic), -CH₂-CO-CH₂- (Id), -CH₂-CH(OH)-CH₂- (Ie), -CH₂-C(Hal)₂-CH₂- (If) und -CH₂-CH(Hal)-CH₂- (Ig), überführen (Reaktionsschema IX).

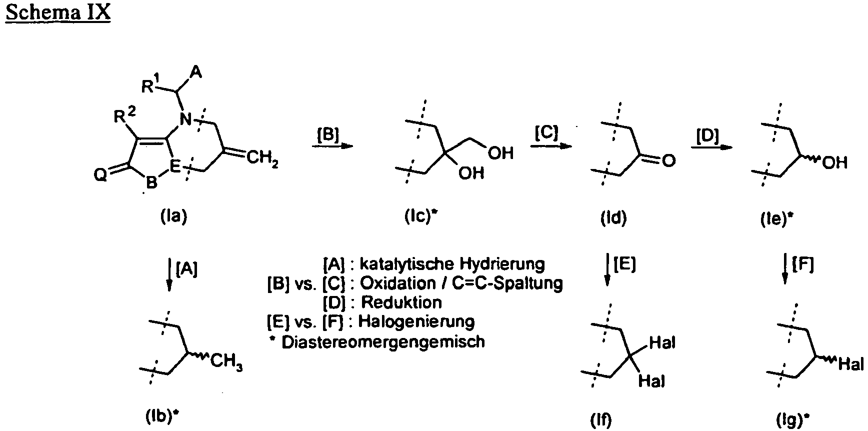

Im Reaktionsschritt [A] können die ungesättigten Verbindungen der allgemeinen Formel (Ia), in der D¹-Z-D² als Gruppe für -CH₂-C(=CH₂)-CH₂- steht, in Gegenwart von geeigneten Hydrierungskatalysatoren in gesättigte Verbindungen der Formel (Ib) (D¹-Z-D² = -CH₂-CH(CH₃)-CH₂-) umgewandelt werden.

Als geeignete Katalysatoren zur Durchführung der katalytischen Hydrierung kommen alle üblichen Hydrierkatalysatoren, wie beispielsweise Platin-Katalysatoren (Platin-Platte, Platin-Schwamm, Platin-Schwarz, kolloidales Platin, Platinoxid, Platindraht usw.), Palladium-Katalysatoren (beispielsweise Palladium-Schwamm, Palladium-Schwarz, Palladiumoxid, Palladium-Kohle, kolloidales Palladium, Palladium-Bariumsulfat, Palladium-Bariumcarbonat, Palladium-Hydroxid usw.), Nickel-Katalysatoren beispielsweise reduziertes Nickel, Nickeloxid, Raney-Nickel usw.), Ruthenium-Katalysatoren, Cobalt-Katalysatoren (beispielsweise reduziertes Cobalt, Raney-Cobalt usw.), Kupfer-Katalysatoren (beispielsweise reduziertes Kupfer, Raney-Kupfer, Ullmann-Kupfer usw.) in Frage. Vorzugsweise verwendet man jedoch Edelmetallkatalysatoren, wie beispielsweise Platin- und Palladium- oder Ruthenium-Katalysatoren gegebenenfalls auf einem geeigneten Träger wie beispielsweise auf Kohlenstoff oder Silizium, Rhodium-Katalysatoren, wie beispielsweise Tris(triphenylphosphin)rhodium(I)-chlorid in Gegenwart von Triphenylphosphin.

Selbstverständlich kann durch Verwendung eines "chiralen Hydrierkatalysators" z. B. mit chiralen Diphosphinliganden, beispielsweise *(2S,3S)-(-)*-2,3-Bis(diphenylphosphino)-butan [(S,S)-Chiraphos] (N. K. Roberts in "Catalytic Aspects of Metal Phosphine Complexes", ACS Washington, S. 337 (1982)) oder *(R)-(*+*)*-2,2'- bzw. *(S)-(-)*-2,2'-Bis(diphenylphosphino)-1,1'-binaphthalin [*R(*+*)*-BINAP bzw. *S(-)*-BINAP] (vgl. A. Miyashita et al. Tetrahedron 40, 1245 (1984)) auch der Anteil eines Isomers im Isomerengemisch deutlich erhöht werden bzw. lässt sich das Entstehen eines weiteren Isomers sogar vollständig unterdrücken.

Für den Reaktionsschritt [A] werden als Hydrierungskatalysatoren bevorzugt Rhodium-Katalysatoren, insbesondere ein Gemisch aus Tris(triphenylphosphin)rhodium(I)-chlorid und Triphenylphosphin, verwendet.

Als inerte organische Verdünnungsmittel können Benzol oder Toluol verwendet werden.

Mit Hilfe der Reaktionsschritte [B] und [C] werden die Verbindungen der allgemeinen Formel (Ia), in der D¹-Z-D² als Gruppe für -CH₂-C(=CH₂)-CH₂₋ steht, in Gegenwart von geeigneten Oxidationsmitteln in Verbindungen der Formel (Id) (D¹-Z-D² = -CH₂-CO-CH₂-) überführt. Dabei können die Verbindungen der Formel (Ic) (D¹-Z-D² = -CH₂-COH(CH₂-OH)-CH₂) als Intermediate isoliert werden.

Für die Oxidation/Spaltung einer Methylengruppierung (vgl. Spaltung von *(i)* unter Ausbildung von *(iii)* und *(iv),* gemäß den Reaktionsschritten [B] und [C] in Reaktionsschema X, sind eine Vielzahl unterschiedlicher Oxidationsmittel bekannt (vgl. z. B. Oxidationsreagenzien in: Comprehensive Organic Transformations; R. C. Larock, Wiley-VCH, 1999; die auf den Seiten 1213-1215 zitierte Literatur; Methods for the Oxidation of Organic Compounds; A. H. Haines, Academic Press 1985; Kapitel 3.4, Seite 117; Houbel-Weyl Methoden der Organischen Chemie, Bd. VII/2b Ketone Teil II, Georg Thieme Verlag Stuttgart, 1976, Seite 1287).

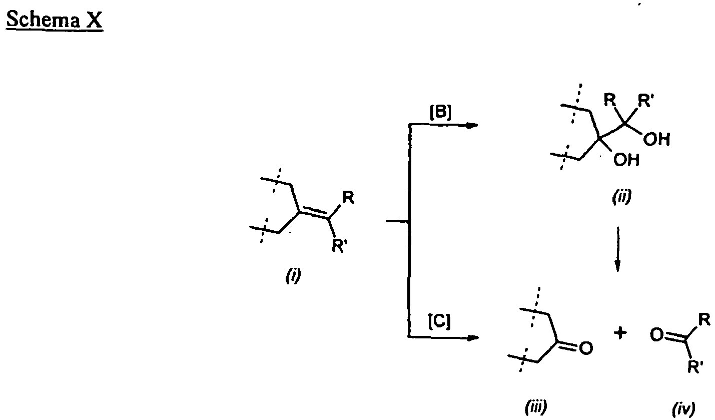

Danach kann eine oxidative Spaltung (d.h. Spaltung von *(i)* unter Ausbildung von *(iii)* und *(iv)*) beispielsweise in Gegenwart von Ozon (vgl. March's Advanced Organic Chemistry, Reactions, Mechanisms, and Structure, (Eds. M. B. Smith, J. March), 5th Edition, Wiley-Interscience Publication, John Wiley & Sons, Inc. 2001, Seite 1522 und Seite 1525) und mit Reaktionsgemischen in denen Ozon verwendet wird, wie beispielsweise in Ozon/ Wasserstoff mit Palladium-Kohle, Ozon/Zink oder Ozon/Jodid und Essigsäure, Ozon/Dimethylsulfid, Ozon/Thioharnstoff, Ozon/Tri(n-butyl)phosphin [n-Bu₃P], Ozon/ Triphenylphosphin, Ozon/Trimethylphosphit [(MeO)₃P], Ozon/Pyridin, in Gegenwart von Permanganaten, wie Kaliumpermanganat, Kaliumpermanganat und Silicagel, einem Gemisch aus Kaliumpermanganat/Aluminium/Wasser, in Gegenwart von Periodaten, wie Natriumperiodat und einer katalytischen Menge Kaliumpermanganat, Natriumperiodat und einer katalytischen Menge Ruthenium(III)-chlorid-hydrat, Natriumperiodat und einer katalytischen Menge Rutheniumdioxid, Natriumperiodat und einer katalytischen Menge Osmium(VIII)-oxid oder Natriumperiodat und einer katalytischen Menge Osmium(VIII)-oxid/N-Methylmorpholin erfolgen.

Die Oxidation/Spaltung einer Methylengruppierung kann auch über eine 1,2-Diolstruktur (Ic; vgl. *ii*) (vgl. Spaltung von *(ii)* unter Ausbildung von *(iii)* und *(iv)*) im Reaktionsschema X) verlaufen. Hierfür sind zahlreiche unterschiedliche Oxidationsmittel bekannt (vgl. z. B. March's Advanced Organic Chemistry, Reactions, Mechanisms, and Structure, (Eds. M. B. Smith, J. March), 5th Edition, Wiley-Interscience Publication, John Wiley & Sons, Inc. 2001, Seite 1519; Houbel-Weyl Methoden der Organischen Chemie, Band VI/1a Alkohole Teil I, Georg Thieme Verlag Stuttgart, 4. Auflage, 1979, Seite 592; Methods for the Oxidation of Organic Compounds; A. H. Haines, Academic Press 1985; Kapitel 3.2, Seite 73). Beispielsweise kann eine Spaltung der 1,2-Diolstruktur bereits unter milden Reaktionsbedingungen in Gegenwart von Metallacetaten, wie beispielsweise Bleitetraacetat oder Periodsäure erfolgen. Desweiteren sind saure Dichromate (vgl. Chromium Oxidations in Organic Chemistry; Cainelli, Cardillo, Springer Verlag: New York, 1984; Reagents for Organic Synthesis; Fieser, Vol. 1, Wiley: New York, 1967, S. 142-147, 1059-1064 und weitere Bände aus dieser Reihe) oder Permanganate, wie Kaliumpermanganat, oder Periodate wie beispielsweise Natriumperiodat, bekannt. Die Oxidationsreagenzien können aber auch polymergebunden vorliegen (vgl. Review: McKillop, Young Synthesis 401-422 (1979)). Sowohl Chromsäuren als auch Pernanganate wurden auf diese Weise als Oxidationsmittel verwendet. Ebenfalls sind zahlreiche Phasen-Transfer-Reaktionen mit Permanganaten, Chromsäuren (Tetrahedron Lett. 4167 (1977), Landini et al. 134 (1979)) und Rutheniumtetroxid (Morris, Kiely J. Org. Chem. 52, 1149 (1987)) bekannt. Selbst Ultraschall-induzierte Oxidationsreaktionen sind denkbar - so ist die Verwendung von Kaliumpermanganat erwähnt (Yamawaki et al. 379 (1983)).

Für den Reaktionsschritt [B] wird als Oxidationsmittel bevorzugt ein Gemisch aus Osmium(VIII)-oxid in *tert-*Butanol und *N*-Methylmorpholin-*N*-oxid verwendet.

Als inertes organisches Verdünnungsmittel werden Ether, wie beispielsweise Methyl-*tert-*butylether, Diisopropylether, Tetrahydrofuran oder Dioxan, insbesondere wird Tetrahydrofuran verwendet.

Für den Reaktionsschritt [C] wird als Oxidationsmittel bevorzugt Natriumperiodat eingesetzt.

Als inertes organisches Verdünnungsmittel wird ein Gemisch aus Halogenkohlenwasserstoffen, insbesondere Chlorkohlenwasserstoffe, wie Methylenchlorid und Wasser im Verhältnis (2:1) verwendet.

Im Reaktionsschritt [D] werden die Verbindungen der allgemeinen Formel (Id), in der D¹-Z-D² als Gruppe für -CH₂-CO-CH₂- steht, in Gegenwart von geeigneten Reduktionsmittel für Carbonylverbindungen in Verbindungen der Formel (Ie) (D¹-Z-D² = -CH₂-CH(OH)-CH₂-) überführt.

Als geeignete Reduktionsmittel zur Reduktion einer Carbonylgruppe kommen die verschiedensten Hydrierungsreagenzien, wie beispielsweise Alkalimetallhydride, insbesondere Natriumborhydrid (NaBH₄), Lithiumaluminiumhydrid (LiAlH₄), Lithiumtriethylborhydrid (Li[Et₃BH]), Lithium-trisec-borhydrid (Li[*sec*-Bu₃BH], Natrium-bis(2-methoxyethoxy)aluminiumhydrid, Alkylaluminiumhydride, insbesondere Diisobutylaluminiumhydrid (DIBAL-H), oder Tetramethylammoniumtriacetoxyborhydrid u. a., in Frage (vgl. H. de Koning, W.N. Houben-Weyl, Methoden der Organischen Chemie, Bd. E 21, Georg Thieme Verlag Stuttgart, S. 1953 sowie dort zitierte Literatur). Selbstverständlich kann auch ein "Borhydrid-Harz" beispielsweise "Borohydride on Amberlite^{®} IRA-406", zur Hydrierung verwendet werden (vgl. A. R. Sande et al. Tetrahedron Lett. 25, 3501 (1984)).

Für den Reaktionsschritt [D] werden als Reduktionsmittel bevorzugt Alkalimetallhydride verwendet, insbesondere wird Natriumborhydrid verwendet.

Als inerte organische Verdünnungsmittel werden bevorzugt Alkohole wie Methanol oder Ethanol, verwendet.

Mit Hilfe der Reaktionsschritte [E] und [F] werden die Verbindungen der allgemeinen Formel (Id) und (Ie), in denen D¹-Z-D² als Gruppe für -CH₂-CO-CH₂- und -CH₂-CH(OH)-CH₂- stehen, in Gegenwart von geeigneten Halogenierungsmitteln in Verbindungen der Formel (If) (D¹-Z-D² = - CH₂-C(Hal)₂-CH₂-) und in Verbindungen der Formel (Ig) (D¹-Z-D² = -CH₂-CH(Hal)-CH₂-) überführt.

Als geeignete Halogenierungsmittel zur Umwandlung (Deoxygenierung) einer Carbonylgruppe oder dessen 1,3-Benzodioxols (wird durch Reaktion der Carbonylverbindung mit 1,2-Dihydroxybenzol gebildet) in eine *gem*-halogenierte Methylengruppe kommen zahlreiche Halogenierungsreagenzien in Frage, beispielsweise Schwefeltetrafluorid (SF₄) / 20 % HF (vgl. D. G. Martin, F. Kagan J. Org. Chem. 27, 3164 (1962)), Carbonylfluorid (COF₂) (F. S. Fawcett et al. J. Am. Chem. Soc. 84, 4275 (1962)), Molybdänhexafluorid (MoF₆) (F. Mathey, J. Bensoam Tetrahedron 27, 3965 (1971)) oder *N,N*-Diethylaminoschwefeltrifluorid (Et₂NSF₃, DAST) (K. A. Jolliffe Aust. J. Chem. 54, 75 (2001)) [für *gem*-Difluormethylen]; Wolframhexachlorid (WCl₆) (M. F. Jung, J. I. Wasserman Tetrahedron Lett. 44, 7273 (2003)) [für gem-Dichloromethylen]; 1,2-Dihydroxybenzol / Bortribromid (via Spaltung des 1,3-Benzodioxols) E. Napolitano et al. Synthesis 2, 122 (1986) [für *gem*-Dibrommethylen]; 1,2-Dihydroxybenzol / Natriumiodid-Acetylchlorid (via Spaltung des 1,3-Benzodioxols) L. Corda et al. J. Het. Chem. 25, 311 (1988) [für *gem*-Diiodmethylen].

Als geeignete Halogenierungsmittel zur Umwandlung einer Hydroxygruppe in eine Halogengruppe kommen alle dafür geeigneten Halogenierungsmittel in Frage, beispielsweise Fluorwasserstoffsäure (HF), Schwefeltetrafluorid / Fluorwasserstoffsäure (HF) (J. Kollonitsch et al. J. Org. Chem. 44, 771 (1979)), *N*-(2-Chlor-1,1,2-trifluoroethyl)diethylamin (vgl. WO 2006103985 A1) *N,N-*Diethylaminoschwefeltrifluorid (Et₂NSF₃, DAST) (K. A. Jolliffe Aust. J. Chem. 54, 75 (2001)), Pyridinium-poly(hydrogenfluorid) (30% Pyridin-70 % HF) (G. A. Olah et al. J. Org. Chem. 44, 3872 (1979)) [für *mono*-Fluormethylen]; Phosgen (COCl₂), Thionylchlorid, Phosphoroxychlorid, Phosphor(III)-chlorid, Phosphor(V)chlorid, ein Gemisch aus Tetrachlormethan (CCl₄) (vgl. z. B. Y. Berger et al., J. Med. Chem. 48, 483 (2005)) und Triphenylphosphin [für *mono-*Chlormethylen]; Thionylbromid, Phosphor(III)-bromid, polymergebundenes Phosphor(III)-bromid (PBr₃ in Amberlite IRA 93) (G. Cainelli et al. Synthesis 4, 306 (1983)), ein Gemisch aus N-Brom-succinimid und Triphenylphosphin (A. K. Bose, B. Lal Tetrahedron 40, 3937 (1973)), ein Gemisch aus Tetrabrommethan (CBr₄) und Triphenylphosphin (vgl. z. B. S. Hanessian et al. Can. J. Chem. 65, 1859 (1987)) [für *mono*-Brommethylen]; Diphosphortetraiodid (P₂I₄) (M. Lauwers et al. Tetrahedron Lett. 20, 1801 (1979)), ein Gemisch aus Chlortrimethylsilan und Natriumiodid (T. Morita et al. Synthesis 5, 379 (1979)), ein Gemisch aus Hexadimethylsilan und Iod (G. A. Olah et al. Angew. Chem. 91, 648 (1979)) [für *mono*-Iodmethylen].

Für den Reaktionsschritt [E] wird als Halogenierungsmittel inbesondere *N,N*-Diethylaminoschwefeltrifluorid (DAST) (Halogen: Fluor) verwendet.

Als inerte organische Verdünnungsmittel werden Halogenkohlenwasserstoffe, insbesondere Chlorkohlenwasserstoffe, wie Methylenchlorid verwendet.

Für den Reaktionsschritt [F] werden als Halogenierungsmittel bevorzugt Gemische aus Tetrahalogenmethanen (Halogen: Brom, Chlor) und Triphenylphosphin oder DAST (Halogen: Fluor) eingesetzt.

Als inertes organisches Verdünnungsmittel werden Nitrile wie beispielsweise Acetonitril, oder Halogenkohlenwasserstoffe, insbesondere Chlorkohlenwasserstoffe, wie Methylenchlorid (Reaktion mit DAST) verwendet.

Die Verbindungen der allgemeinen Formeln (Ib), (Ic), (Ie) und (Ig) liegen als Diastereomerengemische vor, aus denen die Diastereomere mittels Säulenchromatographie erhalten werden können (vgl. auch die Herstellungsbeispiele).

Die Verbindungen der allgemeinen Formel (Ih) und (Ii), in denen D¹-Z-D² als Gruppe für -CH₂-CH₂-CH= oder -CH=CH-CH₂- steht, können beispielsweise leicht aus Verbindungen der allgemeinen Formeln (Ij; worin E = CH) oder (Ie) vs. (Ig), in denen D¹-Z-D² als Gruppe für -CH₂-CH₂-CH(OH)- (Ij), -CH₂-C(OH)-CH₂- (Ie) oder -CH₂-CH(Hal)-CH₂- (Ig; Hal z. B. Chlor) gebildet werden und entstehen daher auch als Nebenprodukte (vgl. Reaktionsschema XI und Herstellungsbeispiele).

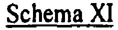

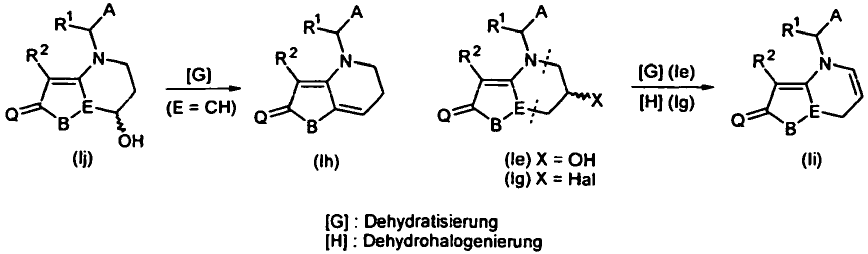

Die Bildung der Verbindungen der allgemeinen Formel (Ih) und (Ii) aus Verbindungen der allgemeinen Formel (Ij) und (Ie) ist beispielsweise mittels Dehydratisierung [Reaktionsschritt G : saures Reaktionshilfsmittel (z. B. Trifluoressigsäure)] oder aus Verbindungen der allgemeinen Formel (Ig) mittels Dehydrohalogenierung [Reaktionsschritt H : Triphenylphosphin, aber auch in Gegenwart basischer Reaktionshilfsmittel] möglich.

Die Verbindungen der allgemeinen Formel (Ik, worin beispielsweise R² = H, B, Q = O, E = CH), in der D¹-Z-D² als Gruppe für -CO-CH₂- steht, sind beispielsweise aus Verbindungen der allgemeinen Formeln (XIa) und (XI) zugänglich (vgl. Reaktionsschema XII und Herstellungsbeispiele).

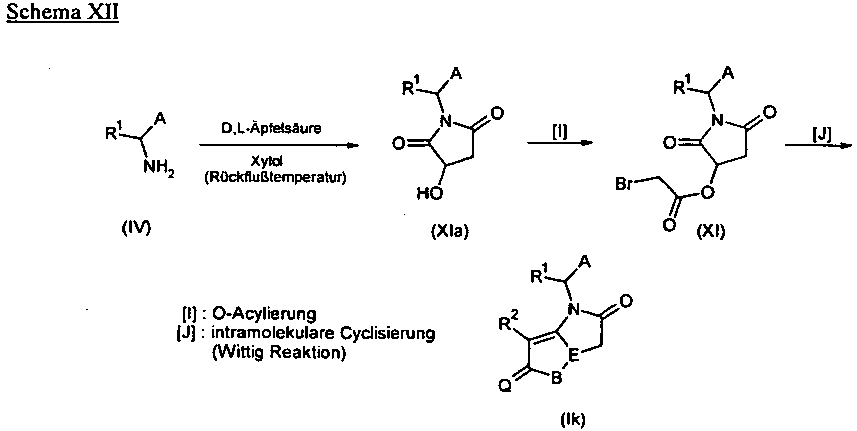

Die Darstellung der Verbindungen der allgemeinen Formel (XIa) kann beispielsweise aus Verbindungen der allgemeinen Formel (IV) und D,L-Äpfelsäure erfolgen. Die O-Acylierung [Reaktionsschritt I: basisches Reaktionshilfsmittel (z. B. Pyridin), Bromacetylbromid, Verdünnungsmittel (z. B. Dichlormethan); vgl. Lee Y. S. et al. Tetrahedron (1999), 55, 4631-4636] unter Bildung von Verbindungen der allgemeinen Formel (XI) sowie deren intramolekulare Cyclisierung zu 4-substituierten 6,6a-Dihydro-2H-furo[3,2-b]pyrrole-2,5(4H)-dion Systemen der allgemeinen Formel (Ik) ist in Anlehnung an literaturbekannte Methoden möglich [intramolekulare Wittig Reaktion, Schritt J: basisches Reaktionshilfsmittel (z. B. Triethylamin), Triphenylphosphin, Vordünnungsmittel (z. B. Acetonitril); R¹, R² = H, B, Q = O; E = CH und A = COOEt: vgl. Niwa H et al. J. Org. Chem. (1987) 52, 2941-2943; analog für A = -CH₂CH₂-CH=CH₂: vgl. Lee Y. S. et al. Tetrahedron (1999), 55, 4631-4636; für A = 6-Chlor-pyrid-3-yl: vgl. auch Herstellungsbeispiele].

Setzt man bei dem Herstellungsverfahren 3 zur Herstellung der neuen Verbindungen der allgemeinen Formel (I) in einem ersten Reaktionsschritt als Verbindungen der Formel (VI) beispielsweise 4-{Allyl[6-chlorpyridin-3-yl]methyl]amino}furan-2(5H)-on ein, so lässt sich das Herstellungsverfahren 3 durch folgendes Reaktionsschema XIII wiedergeben:

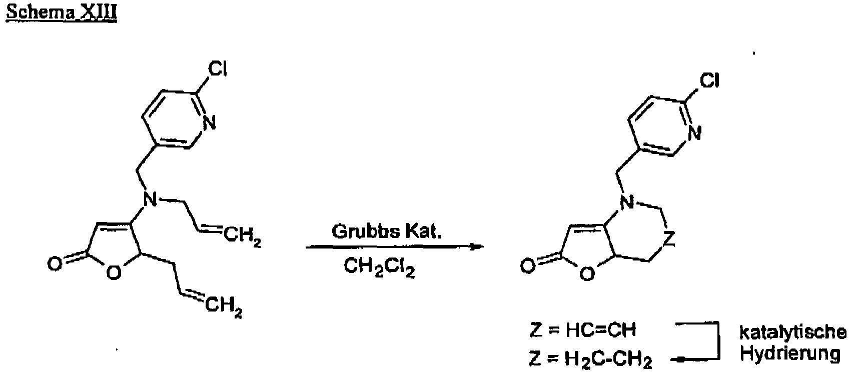

Die insbesondere für die Durchführung des Herstellungsverfahrens 3 als Ausgangsstoffe zu verwendenden Verbindungen sind allgemein durch die Formel (VI) definiert.

In der Formel (VI) haben A, B, E, Z', R¹, R², D¹, D², n, m, Q die weiter oben für Formel (1) genannte Bedeutung.

Die Verbindungen der allgemeinen Formel (VI) können nach literaturbekannten Methoden erhalten werden, beispielsweise nach dem im Reaktionsschema XIV gezeigten Herstellungsverfahren. 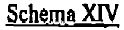 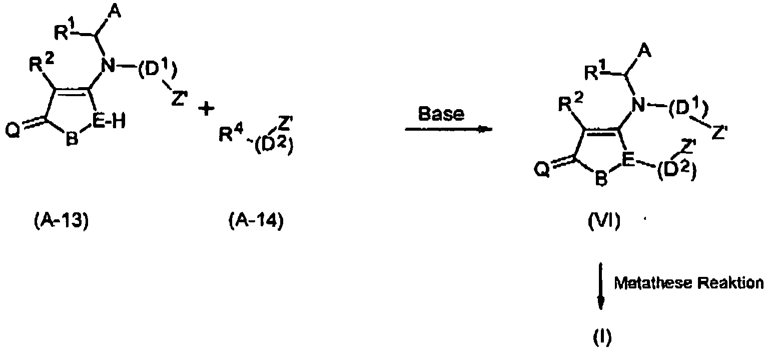 Geeignete Ausgangsverbindungen zur Darstellung der Verbindungen (A-13) sind die weiter oben im Schema II genannten Verbindungen (A-1). Weitere geeignete Ausgangsverbindungen zur Darstellung der Verbindungen (A-13) sind sekundäre Amine (R¹-CH(A)-NH-(D¹)-Z'; D¹ = gegebenenfalls substituierter Kohlenstoff) in denen Z' für eine C=C-Doppelbindung oder C≡C-Dreifachbindung steht.

Die Reaktion der Verbindungen (A-13) mit ungesättigten Verbindungen (A-14) erfolgt gemäß dem weiter oben im Reaktionsschema VII genannten Herstellungsverfahren unter Ausbildung der geeigneten Vorstufen (VI) für die nachfolgende Metathesereaktion.

Die Metathesercaktion ist literaturbekannt und kann entsprechend den dafür bekannten Reaktionsbedingungen unter Verwendung bekannter Katalysatoren durchgeführt werden (vgl. z. B.: Van de Weghe P. et al., Current Topics Med. Chem. (2005), 5, 1461-72. Deiters, A. et al., Chem. Rev. (Washington, DC, United States) (2004), 104, 2199-2238. Nakamura, I.; Yamamoto, Y. Chem. Rev. (Washington, DC, United States) (2004), 104, 2127-2198).

Beispielsweise und bevorzugt werden hierfür Ruthenium-Katalysatoren verwenden, die auch als Grubbs Katalysatoren der ersten und zweiten Generation bekannt geworden sind (z. B. Schmidt, B. Angew. Chem, Intern. Edition (2003), 42, 4996-4999).

Im Allgemeinen ist es vorteilhaft, das Herstellungsverfahrens 3 in Gegenwart von Verdünnungsmitteln durchzuführen. Verdünnungsmittel werden vorteilhaft in einer solchen Menge eingesetzt, dass das Reaktionsgemisch während des ganzen Verfahrens gut rührbar bleibt. Als Verdünnungsmittel zur Durchführung des Verfahrens 3 kommen alle unter den Reaktionsbedingungen inerten organischen Lösungsmittel in Frage.

Bevorzugte Verdünnungsmittel zur Durchführung des Verfahrens 3 sind entsprechend dem weiter oben genannten Verfahren 1, Halogenkohlenwasserstoffe, insbesondere Chlorkohlenwasserstoffe, wie Tetraethylen, Tetrachlorethan, Dichlorpropan, Methylenchlorid, Dichlorbutan, Chloroform, Tetrachlorkohlenstoff, Trichlorethan, Trichlorethylen oder Pentachlorethan, bevorzugt wird Methylenchlorid verwendet.

Die Umsetzung von Verbindungen der allgemeinen Formel (IV) nach dem Herstellungsverfahren 3 wird durchgeführt, indem man die Verbindungen der allgemeinen Formel (IV) in Gegenwart geeigneten Katalysatoren, beispielsweise Grubbs Katalysatoren der zweiten Generation, umsetzt.

Die Reaktionsdauer beträgt 10 Minuten bis 48 Stunden. Die Umsetzung erfolgt bei Temperaturen zwischen -10°C und +200°C, bevorzugt zwischen +10°C und 180°C, besonders bevorzugt zwischen 20°C und 140°C. Man arbeitet ganz besonders bevorzugt bei Raumtemperatur.

Nach vollendeter Umsetzung wird der gesamte Reaktionsansatz eingeengt. Die nach Aufarbeitung anfallenden Produkte lassen sich in üblicher Weise durch Umkristallisieren, Vakuumdestillation oder Säulenchromatographie reinigen (vgl. auch die Herstellungsbeispiele).

In einem nachfolgenden Reaktionsschritt können die ungesättigten die Verbindungen der aligemeinen Formel (I) (D¹-Z-D² = H₂C-HC=CH-CH₂) in Gegenwart von geeigneten Hydrierungskatalysatoren in gesättigte Verbindungen der Formel (I) (Z = H₂C-CH₂CH₂-CH₂) umgewandelt werden (vgl. Schema XII).

Zur Hydrierung von Verbindungen der allgemeinen Formel (I) finden als Hydrierungskatalysatoren bevorzugt Rhodium-Katalysatoren, insbesondere ein Gemisch aus Tris(triphenylphosphin) rhodium(I)-chlorid und Triphenylphosphin, Verwendung.

Als inerte organische Verdünnungsmittel finden die bei dem Verfahren 1 genannten Verdünnungsmittel, wie z. B. Benzol oder Toluol, Verwendung.

Setzt man bei dem Verfahren 4 zur Herstellung der neuen Verbindungen der allgemeinen Formel (I) in einem ersten Reaktionsschritt als Verbindungen der Formel (VII) beispielsweise das 3-chlor-5-hydroxymethyl-4-{[(6-chlorpyridin-3-yl)methyl]amino}furan-2(5H)-on und als Verbindungen der Formel (VIII) oder (IX) Formaldehyd bzw. Paraformaldehyd ein, so lässt sich das Herstellungsverfahren 4 durch folgendes Reaktionsschema XV wiedergeben:

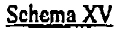

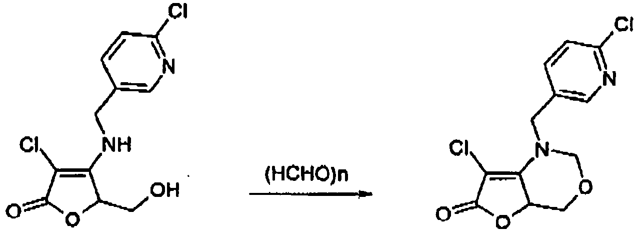

Die insbesondere für die Durchführung des Herstellungsverfahrens 4 als Ausgangsstoffe zu verwendenden Verbindungen sind allgemein durch die Formel (VII) definiert.

In der Formel (VII) haben A, B, E, Z, R¹, R², D², Q die weiter oben für Formel (I) genannte Bedeutung.

Die Verbindungen der allgemeinen Formel (VII) können nach literaturbekannten Methoden (z. B. EP 0539588 A1) oder gemäß dem weiter oben genannten Verfahren 1 erhalten werden. Die im Verfahren 4 verwendete Ausgangsverbindung 4-Hydroxy-furan-2(5H)-on kann beispielsweise aus dem literaturbekannten 5-Benzyloxymethyl-4-hydroxy-furan-2(5H)-on (Aragon, D. T. et al., J. Org. Chem. 68, 3363-3365, 2003) mittels werden Debenzylierung erhalten werden. Anschließende Reaktion mit 3-Aminomethyl-6-chlor-pyridin und Halogenierung nach einer der weiter unten genannten Methode, beispielsweise mit N-Chlor-succinimid, führt dann zum 3-Chlor-5-hydroxymethyl-4-{[(6-chlorpyridin-3-yl)methyl]amino}furan-2(5H)-on (vgl. auch Herstellungsbeispiele).

In der Formel (VIII) oder (IX) hat R' A die weiter oben für Formen (I) genannte Bedeutung.

Die Verbindungen der Formel (VIII) oder (IX) sind allgemein bekannt und können kommerziell erhalten werden.

Im Allgemeinen ist es vorteilhaft, das Herstellungsverfahren 4 in Gegenwart von Verdünnungsmitteln und in Gegenwart eines sauren Reaktionshilfsmittels durchzuführen.

Als Verdünnungsmittel zur Durchführung des Herstellungsverfahrens 4 kommen alle unter den Reaktionsbedingungen inerten organischen Lösungsmittel in Frage, die weiter oben unter dem Verfahren 1 genannt sind.

Bevorzugte Verdünnungsmittel zur Durchführung des erfindungsgemäßen Herstellungsverfahrens 4 sind entsprechend dem weiter oben genannten Herstellungsverfahrens 1, aromatische Kohlenwasserstoffe wie Benzol, Toluol, Chlorbenzol, Brombenzol, Nitrobenzol oder xylol, insbesondere aber Benzol und Toluol. Zur besseren Löslichkeit können gegebenenfalls auch geringfügige Mengen an Amiden wie *N,N*-Dimethyl-formamid, *N,N*-Dipropyl-formamid, *N,N*-Dibutyl-formamid oder *N*-Methyl-pyrrolidin, insbesondere *N,N-*Dimethylformamid, hinzugegeben werden.

Bevorzugte saure Reaktionshilfsmittel sind organischen Säuren gehören, wie Essigsäure, Benzolsulfonsäure oder para-Toluolsulfonsäure, insbesondere para-Toluolsulfonsäure.

Die Umsetzung von Verbindungen der allgemeinen Formel (VII) nach dem Herstellungsverfahren 4 wird durchgeführt, indem man die Verbindungen der allgemeinen Formel (VII) mit Verbindungen der allgemeinen Formel (VIII) oder (IX) in Gegenwart eines sauren Reaktionshilfsmittels in einem der angegebenen Verdünnungsmittel umsetzt.

Die Reaktionsdauer beträgt 10 Minuten bis 48 Stunden. Die Umsetzung erfolgt bei Temperaturen zwischen -10°C und +200°C, bevorzugt zwischen +10°C und 180°C, besonders bevorzugt zwischen 60°C und 140°C. Man arbeitet bevorzugt unter den Reaktionsbedingungen, die das Abscheiden oder Entfernen von Wasser ermöglichen, beispielsweise unter zu Hilfenahme eines Wasserabscheiders.

Nach vollendeter Umsetzung wird der gesamte Reaktionsansatz eingeengt. Die nach Aufarbeitung anfallenden Produkte lassen sich in üblicher Weise durch Umkristallisieren, Vakuumdestillation oder Säulenchromatographie reinigen (vgl. auch die Herstellungsbeispiele).

Zur Darstellung der Verbindungen der allgemeinen Formel (I) in der R² für Halogen steht, können alternativ und erfindungsgemäß auch Verbindungen der allgemeinen Formel (I) in der R² für Wasserstoff steht, mit Halogenierungsmitteln in Gegenwart von basischen Reaktionshilfsmitteln gemäß dem Reaktionsschritt [K] im Reaktionsschema (XVI) umgesetzt werden.

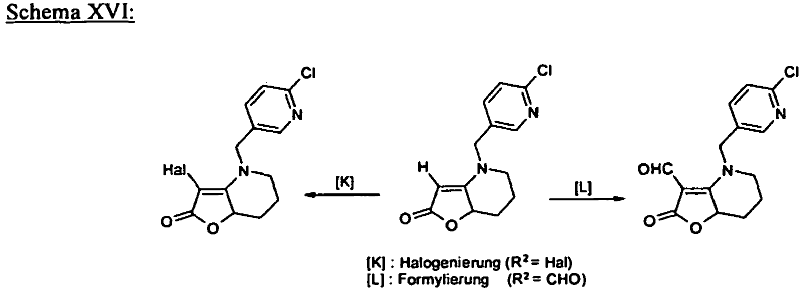

In den für eine Halogenierung als Ausgangsstoffe benötigten Verbindungen der Formel (I) haben A, B, E, Z, Q, D¹, D², R¹ und R² die weiter oben genannte Bedeutung, der Substituent R² hat die Bedeutung von Wasserstoff.

Die Verbindungen der allgemeinen Formel (I) können nach den oben genannten Herstellverfahren 1 bis 4 erhalten werden.

Im Allgemeinen ist es vorteilhaft, die Halogenierung in Gegenwart von Verdünnungsmitteln durchzuführen. Verdünnungsmittel werden vorteilhaft in einer solchen Menge eingesetzt, dass das Reaktionsgemisch während des ganzen Verfahrens gut rührbar bleibt. Als Verdünnungsmittel zur Durchführung der Halogenierung kommen alle unter den Reaktionsbedingungen inerten organischen Lösungsmittel in Frage.

Als Halogenierungsmittel zur Durchführung des erfindungsgemäßen Verfahrens können alle geeigneten Halogenierungsmittel eingesetzt werden wie *N*-Halogen-Verbindungen.

Beispielhaft seien dafür erwähnt die *N*-Halogen-amine wie 1-Chlormethyl-4-fluordiazoniabicyclo-[2.2.2]octan-bis-(tetrafluoroborat) (Selectfluor^{®}), *N,N*-Dihalogen-amine, *N*-Halogen-carbonsäureamide, *N*-Halogen-carbamidsäureester, *N*-Halogen-harnstoff, *N*-Halogen-sulfonylamide, *N*-Halogen-disulfonylamide, *N*-Halogen-sulfonylimide wie *N-*fluorbis[(trifluormethyl)sulfonyl]imid und *N*-Halogen-carbonsäurediamide wie *N*-Chlor-phthalimid, *N-*Brom-phthalimid, *N*-Iod-phthalimid, N-Chlor-succinimid (NCS), *N*-Brom-succinimid (NBS), *N*-Brom-saccharin oder *N*-Iod-succinimid.

Bevorzugte Halogenierungsmittel zur Durchführung der Halogenierung sind die *N*-Halogen-carbonsäurediamide (Hal = Brom, Chlor oder Iod oder 1-Chlormethyl-4-fluordiazoniabicyclo[2.2.2]octan-*bis*-(tetrafluoroborat) (Selectfluor^{®}; vgl. auch P. T. Nyffeler et al. Angew. Chem. (2004), 116, 2-23) (Hal = Fluor).

Bevorzugte Verdünnungsmittel zur Durchführung der Halogenierung sind entsprechend dem weiter oben genannten Verfahren 2, Nitrile wie Acetonitril, Propionitril, Butyronitril, Isobutyronitril, Benzonitril, m-Chlorbenzonitril.

Selbstverständlich kann man in das erfindungsgemäße Verfahren auch Gemische der genannten Lösungs- und Verdünnungsmittel einsetzen.

Bevorzugte Verdünnungsmittel zur Durchführung des erfindungsgemäßen Herstellungsverfahrens sind jedoch Nitrile wie Acetonitril, Propionitril oder Butyronitril.

Die Halogenierung von Verbindungen der allgemeinen Formel (I) wird durchgeführt, indem man diese mit geeigneten Halogenierungsmitteln in Gegenwart von basischen Reaktionshilfsmitteln umsetzt.

Die Reaktionsdauer beträgt 10 Minuten bis 48 Stunden. Die Umsetzung erfolgt bei Temperaturen zwischen -10°C und +100°C, bevorzugt zwischen 0°C und 60°C, besonders bevorzugt zwischen 10°C und Raumtemperatur.

Nach vollendeter Umsetzung wird der gesamte Reaktionsansatz eingeengt. Die nach Aufarbeitung anfallenden Produkte lassen sich in üblicher Weise durch Umkristallisieren, Vakuumdestillation oder Säulenchromatographie reinigen (vgl. auch die Herstellungsbeispiele).

Zur Darstellung der Verbindungen der allgemeinen Formel (I) in der R² für Formyl steht, können alternativ und erfindungsgemäß auch Verbindungen der allgemeinen Formel (I) in der R² für Wasserstoff steht, mit geeigneten Methoden umgesetzt werden, die gemäß dem Reaktionsschritt [L] die Einführung der Formylgruppe ermöglichen (vgl. Reaktionsschema XVI und Herstellungsbeispiele).

Eine geeignete Methode zur Einführung der Formylgruppe ist beispielsweise die Vilsmeier-Reaktion (vgl. Houben-Weyl, Methoden der Organischen Chemie, Bd. VII/1, Georg Thieme Verlag Stuttgart, S. 30; L. N. Ferguson Chem. Rev. (1946), 38, 230). Hierbei können *N*-Methylformanilid, *N-*Methyl- bzw. *N,N*-Dimethyl-formamid und Phosphorylchlorid oder alternativ *N,N* Dimethylthioformamid und Phthalsäureanhydrid verwendet werden.

Für den Reaktionsschritt [L] wird zur Einführung der Formylgruppe in Abwesenheit eines inerten organischen Verdünnungsmittels bevorzugt ein Gemisch aus *N,N-*Dimethyl-formamid und Phosphorylchlorid verwendet (vgl. auch Herstellungsbeispiele).

Zur Darstellung der Verbindungen der allgemeinen Formel (I) in der E für CH-Alkyl steht, können alternativ und erfindungsgemäß auch Verbindungen der allgemeinen Formel (I) in der E für CH steht, mit geeigneten Alkylierungsmitteln in Gegenwart von basischen Hilfsmitteln gemäß dem Reaktionsschema (XVII) umgesetzt werden.

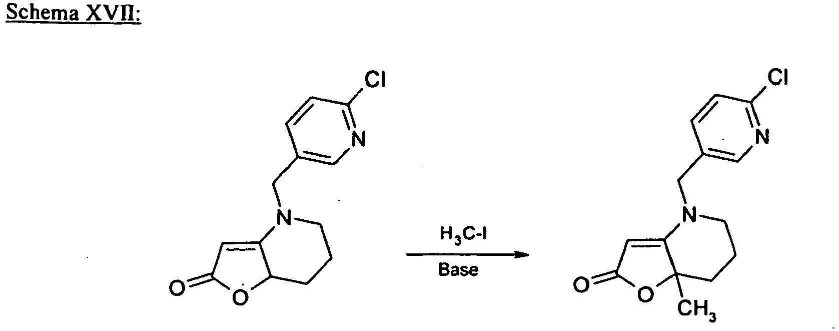

In den zur Herstellung der C-Alkylierung als Ausgangsstoffe benötigten Verbindungen der Formel (I) haben A, B, E, Z, Q, D¹, D², R¹ und R² die weiter oben genannte Bedeutung, der Substituent R² hat die Bedeutung von Wasserstoff.

Die Verbindungen der allgemeinen Formel (I) können nach den oben genannten Herstellverfahren 1 bis 4 erhalten werden.

Im Allgemeinen ist es vorteilhaft, die C-Alkylierung in Gegenwart von Verdünnungsmitteln durchzuführen. Verdünnungsmittel werden vorteilhaft in einer solchen Menge eingesetzt, dass das Reaktionsgemisch während des ganzen Verfahrens gut rührbar bleibt. Als Verdünnungsmittel zur Durchführung der C-Alkylierung kommen alle unter den Reaktionsbedingungen inerten organischen Lösungsmittel in Frage.

Bevorzugte Verdünnungsmittel zur Durchführung der C-Alkylierung sind Ether wie Methyl-tert-butylether, n-Butylether, Anisol, Phenetol, Cyclohexylmethylether, Diisopropylether, Diisobutylether, Diisoamylether, Ethylenglycoldimethylether, Tetrahydrofuran, Dioxan, Dichlordiethylether und Polyether des Ethylenoxids und/oder Propylenoxids.

Selbstverständlich kann man in das erfindungsgemäße Verfahren auch Gemische der genannten Lösungs- und Verdünnungsmittel einsetzen.

Bevorzugte Verdünnungsmittel zur Durchführung des erfindungsgemäßen Verfahrens sind jedoch Ether wie Methyl-tert-butylether oder cyclische Ether wie Tetrahydrofuran und Dioxan.

Die C-Alkylierung von Verbindungen der Formel (I) erfolgt durch Umsetzung mit geeigneten Alkylierungsmitteln in Gegenwart von basischen Reaktionshilfsmitteln.

Die Reaktionsdauer beträgt 10 Minuten bis 48 Stunden. Die Umsetzung erfolgt bei Temperaturen zwischen -100°C und +20°C, bevorzugt zwischen -90°C und 10°C, besonders bevorzugt zwischen -80°C und 0°C.

Nach vollendeter Umsetzung wird der gesamte Reaktionsansatz eingeengt. Die nach Aufarbeitung anfallenden Produkte lassen sich in üblicher Weise durch Umkristallisieren, Vakuumdestillation oder Säulenchromatographie reinigen (vgl. auch die Herstellungsbeispiele).

Die Verbindungen der Formel (I) können gegebenenfalls in verschiedenen polymorphen Formen oder als Mischung verschiedener polymorpher Formen vorliegen. Sowohl die reinen Polymorphe als auch die Polymorphgemische sind Gegenstand der Erfindung und können erfindungsgemäß verwendet werden.

Die erfindungsgemäßen Wirkstoffe eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes und zur Bekämpfung von tierischen Schädlingen, insbesondere Insekten, Spinnentieren, Helminthen, Nematoden und Mollusken, die in der Landwirtschaft, im Gartenbau, bei der Tierzucht, in Forsten, in Gärten und Freizeiteinrichtungen, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:

Aus der Ordnung der Anoplura (Phthiraptera) z.B. Damalinia spp., Haematopinus spp., Linognathus spp., Pediculus spp., Trichodectes spp..

Aus der Klasse der Arachnida z.B. Acarus siro, Aceria sheldoni, Aculops spp., Aculus spp., Amblyomma spp., Argas spp., Boophilus spp., Brevipalpus spp., Bryobia praetiosa, Chorioptes spp., Dermanyssus gallinae, Eotetranychus spp., Epitrimerus pyri, Eutetranychus spp., Eriophyes spp., Hemitarsonemus spp., Hyalomma spp., Ixodes spp., Latrodectus mactans, Metatetranychus spp., Oligonychus spp., Ornithodoros spp., Panonychus spp., Phyllocoptruta oleivora, Polyphagotarsonemus latus, Psoroptes spp., Rhipicephalus spp., Rhizoglyphus spp., Sarcoptes spp., Scorpio maurus, Stenotarsonemus spp., Tarsonemus spp., Tetranychus spp., Vasates lycopersici.

Aus der Klasse der Bivalva z.B. Dreissena spp..

Aus der Ordnung der Chilopoda z.B. Geophilus spp., Scutigera spp..

Aus der Ordnung der Coleoptera z.B. Acanthoscelides obtectus, Adoretus spp., Agelastica alni, Agriotes spp., Amphimallon solstitialis, Anobium punctatum, Anoplophora spp., Anthonomus spp., Anthrenus spp., Apogonia spp., Atomaria spp., Attagenus spp., Bruchidius obtectus, Bruchus spp., Ceuthorhynchus spp., Cleonus mendicus, Conoderus spp., Cosmopolites spp., Costelytra zealandica, Curculio spp., Cryptorhynchus lapathi, Dermestes spp., Diabrotica spp., Epilachna spp., Faustinus cubae, Gibbium psylloides, Heteronychus arator, Hylamorpha elegans, Hylotrupes bajulus, Hypera postica, Hypothenemus spp., Lachnosterna consanguinea, Leptinotarsa decemlineata, Lissorhoptrus oryzophilus, Lixus spp., Lyctus spp., Meligethes aeneus, Melolontha melolontha, Migdolus spp., Monochamus spp., Naupactus xanthographus, Niptus hololeucus, Oryctes rhinoceros, Oryzaephilus surinamensis, Otiorrhynchus sulcatus, Oxycetonia jucunda, Phaedon cochleariae, Phyllophaga spp., Popillia japonica, Premnotrypes spp., Psylliodes chrysocephala, Ptinus spp., Rhizobius ventralis, Rhizopertha dominica, Sitophilus spp., Sphenophorus spp., Sternechus spp., Symphyletes spp., Tenebrio molitor, Tribolium spp., Trogoderma spp., Tychius spp., Xylotrechus spp., Zabrus spp..

Aus der Ordnung der Collembola z.B. Onychiurus armatus.

Aus der Ordnung der Dermaptera z.B. Forficula auricularia.

Aus der Ordnung der Diplopoda z.B. Blaniulus guttulatus.

Aus der Ordnung der Diptera z.B. Aedes spp., Anopheles spp., Bibio hortulanus, Calliphora erythrocephala, Ceratitis capitata, Chrysomyia spp., Cochliomyia spp., Cordylobia anthropophaga, Culex spp., Cuterebra spp., Dacus oleae, Dermatobia hominis, Drosophila spp., Fannia spp., Gastrophilus spp., Hylemyia spp., Hyppobosca spp., Hypoderma spp., Liriomyza spp.. Lucilia spp., Musca spp., Nezara spp., Oestrus spp., Oscinella frit, Pegomyia hyoscyami, Phorbia spp., Stomoxys spp., Tabanus spp., Tannia spp., Tipula paludosa, Wohlfahrtia spp.

Aus der Klasse der Gastropoda z.B. Arion spp., Biomphalaria spp., Bulinus spp., Deroceras spp., Galba spp., Lymnaea spp., Oncomelania spp., Succinea spp..

Aus der Klasse der Helminthen z.B. Ancylostoma duodenale, Ancylostoma ceylanicum, Acylostoma braziliensis, Ancylostoma spp., Ascaris lubricoides, Ascaris spp., Brugia malayi, Brugia timori, Bunostomum spp., Chabertia spp., Clonorchis spp., Cooperia spp., Dicrocoelium spp, Dictyocaulus filaria, Diphyllobothrium latum, Dracunculus medinensis, Echinococcus granulosus, Echinococcus multilocularis, Enterobius vermicularis, Faciola spp., Haemonchus spp., Heterakis spp., Hymenolepis nana, Hyostrongulus spp., Loa Loa, Nematodirus spp., Oesophagostomum spp., Opisthorchis spp., Onchocerca volvulus, Ostertagia spp., Paragonimus spp., Schistosomen spp, Strongyloides fuellebomi, Strongyloides stercoralis, Stronyloides spp., Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Wuchereria bancrofti.

Weiterhin lassen sich Protozoen, wie Eimeria, bekämpfen.

Aus der Ordnung der Heteroptera z.B. Anasa tristis, Antestiopsis spp., Blissus spp., Calocoris spp., Campylomma livida, Cavelerius spp., Cimex spp., Creontiades dilutus, Dasynus piperis, Dichelops furcatus, Diconocoris hewetti, Dysdercus spp., Euschistus spp., Eurygaster spp., Heliopeltis spp., Horcias nobilellus, Leptocorisa spp., Leptoglossus phyllopus, Lygus spp., Macropes excavatus, Miridae, Nezara spp., Oebalus spp., Pentomidae, Piesma quadrata, Piezodorus spp., Psallus seriatus, Pseudacysta persea, Rhodnius spp., Sahlbergella singularis, Scotinophora spp., Stephanitis nashi, Tibraca spp., Triatoma spp.

Aus der Ordnung der Homoptera z.B. Acyrthosipon spp., Aeneolamia spp., Agonoscena spp., Aleurodes spp., Aleurolobus barodensis, Aleurothrixus spp., Amrasca spp., Anuraphis cardui, Aonidiella spp., Aphanostigma piri, Aphis spp., Arboridia apicalis, Aspidiella spp., Aspidiotus spp., Atanus spp., Aulacorthum solani, Bemisia spp., Brachycaudus helichrysii, Brachycolus spp., Brevicoryne brassicae, Calligypona marginata, Carneocephala fulgida, Ceratovacuna lanigera, Cercopidae, Ceroplastes spp., Chaetosiphon fragaefolii, Chionaspis tegalensis, Chlorita onukii, Chromaphis juglandicola, Chrysomphalus ficus, Cicadulina mbila, Coccomytilus halli, Coccus spp., Cryptomyzus ribis, Dalbulus spp., Dialeurodes spp., Diaphorina spp., Diaspis spp., Doralis spp., Drosicha spp., Dysaphis spp., Dysmicoccus spp., Empoasca spp., Eriosoma spp., Erythroneura spp., Euscelis bilobatus, Geococcus coffeae, Homalodisca coagulata, Hyalopterus arundinis, Icerya spp., Idiocerus spp., Idioscopus spp., Laodelphax striatellus, Lecanium spp., Lepidosaphes spp., Lipaphis erysimi, Macrosiphum spp., Mahanarva fimbriolata, Melanaphis sacchari, Metcalfiella spp., Metopolophium dirhodum, Monellia costalis, Monelliopsis pecanis, Myzus spp., Nasonovia ribisnigri, Nephotettix spp., Nilaparvata lugens, Oncometopia spp., Orthezia praelonga, Parabemisia myricae, Paratrioza spp., Parlatoria spp., Pemphigus spp., Peregrinus maidis, Phenacoccus spp., Phloeomyzus passerinii, Phorodon humuli, Phylloxera spp., Pinnaspis aspidistrae, Planococcus spp., Protopulvinaria pyriformis, Pseudaulacaspis pentagona, Pseudococcus spp., Psylla spp., Pteromalus spp., Pyrilla spp., Quadraspidiotus spp., Quesada gigas, Rastrococcus spp., Rhopalosiphum spp., Saissetia spp., Scaphoides titanus, Schizaphis graminum, Selenaspidus articulatus, Sogata spp., Sogatella furcifera, Sogatodes spp., Stictocephala festina, Tenalaphara malayensis, Tinocallis caryaefoliae, Tomaspis spp., Toxoptera spp., Trialeurodes vaporariorum, Trioza spp., Typhlocyba spp., Unaspis spp., Viteus vitifolii.

Aus der Ordnung der Hymenoptera z.B. Diprion spp., Hoplocampa spp., Lasius spp., Monomorium pharaonis, Vespa spp..

Aus der Ordnung der Isopoda z.B. Armadillidium vulgare, Oniscus asellus, Porcellio scaber.

Aus der Ordnung der Isoptera z.B. Reticulitermes spp., Odontotermes spp..

Aus der Ordnung der Lepidoptera z.B. Acronicta major, Aedia leucomelas, Agrotis spp., Alabama argillacea, Anticarsia spp., Barathra brassicae, Bucculatrix thurberiella, Bupalus piniarius, Cacoecia podana, Capua reticulana, Carpocapsa pomonella, Cheimatobia brumata, Chilo spp., Choristoneura fumiferana, Clysia ambiguella, Cnaphalocerus spp., Earias insulana, Ephestia kuehniella, Euproctis chrysorrhoea, Euxoa spp., Feltia spp., Galleria mellonella, Helicoverpa spp., Heliothis spp., Hofmannophila pseudospretella, Homona magnanima, Hyponomeuta padella, Laphygma spp., Lithocolletis blancardella, Lithophane antennata, Loxagrotis albicosta, Lymantria spp., Malacosoma neustria, Mamestra brassicae, Mocis repanda, Mythimna separata, Oria spp., Oulema oryzae, Panolis flammea, Pectinophora gossypiella, Phyllocnistis citrella, Pieris spp., Plutella xylostella, Prodenia spp., Pseudaletia spp., Pseudoplusia includens, Pyrausta nubilalis, Spodoptera spp., Thermesia gemmatalis, Tinea pellionella, Tineola bisselliella, Tortrix viridana, Trichoplusia spp..

Aus der Ordnung der Orthoptera z.B. Acheta domesticus, Blatta orientalis, Blattella germanica, Gryllotalpa spp., Leucophaea maderae, Locusta spp., Melanoplus spp., Periplaneta americana, Schistocerca gregaria.

Aus der Ordnung der Siphonaptera z.B. Ceratophyllus spp., Xenopsylla cheopis.

Aus der Ordnung der Symphyla z.B. Scutigerella immaculata.

Aus der Ordnung der Thysanoptera z.B. Baliothrips biformis, Enneothrips flavens, Frankliniella spp., Heliothrips spp., Hercinothrips femoralis, Kakothrips spp., Rhipiphorothrips cruentatus, Scirtothrips spp., Taeniothrips cardamoni, Thrips spp..

Aus der Ordnung der Thysanura z.B. Lepisma saccharina.

Zu den pflanzenparasitären Nematoden gehören z.B. Anguina spp., Aphelenchoides spp., Belonoaimus spp., Bursaphelenchus spp., Ditylenchus dipsaci, Globodera spp., Heliocotylenchus spp., Heterodera spp., Longidorus spp., Meloidogyne spp., Pratylenchus spp., Radopholus similis, Rotylenchus spp., Trichodorus spp., Tylenchorhynchus spp., Tylenchulus spp., Tylenchulus semipenetrans, Xiphinema spp..

Die erfindungsgemäßen Verbindungen können gegebenenfalls in bestimmten Konzentrationen bzw. Aufwandmengen auch als Herbizide, Safener, Wachstumsregulatoren oder Mittel zur Verbesserung der Pflanzeneigenschaften, oder als Mikrobizide, beispielsweise als Fungizide, Antimykotika, Bakterizide, Virizide (einschließlich Mittel gegen Viroide) oder als Mittel gegen MLO (Mycoplasma-like-organism) und RLO (Rickettsia-like-organism) verwendet werden. Sie lassen sich gegebenenfalls auch als Zwischen- oder Vorprodukte für die Synthese weiterer Wirkstoffe einsetzen.

Die Wirkstoffe können in die üblichen Formulierungen überführt werden, wie Lösungen, Emulsionen, Spritzpulver, wasser- und ölbasierte Suspensionen, Pulver, Stäubemittel, Pasten, lösliche Pulver, lösliche Granulate, Streugranulate, Suspensions-Emulsions-Konzentrate, Wirkstoff-imprägnierte Naturstoffe, Wirkstoff-imprägnierte synthetische Stoffe, Düngemittel sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln. Die Herstellung der Formulierungen erfolgt entweder in geeigneten Anlagen oder auch vor oder während der Anwendung.

Als Hilfsstoffe können solche Stoffe Verwendung finden, die geeignet sind, dem Mittel selbst oder und/oder davon abgeleitete Zubereitungen (z.B. Spritzbrühen, Saatgutbeizen) besondere Eigenschaften zu verleihen, wie bestimmte technische Eigenschaften und/oder auch besondere biologische Eigenschaften. Als typische Hilfsmittel kommen in Frage: Streckmittel, Lösemittel und Trägerstoffe.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (poly-)Ether, der einfachen und substituierten Amine, Amide, Lactame (wie *N*-Alkylpyrrolidone) und Lactone, der Sulfone und Sulfoxide (wie Dimethylsysulfoxid).

Im Falle der Benutzung von Wasser als Streckmittel können z.B. auch organische Lösemittel als Hilfslösungsmittel verwendet werden. Als flüssige Lösemittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Papier, Sägemehl, Kokosnussschalen, Maiskolben und Tabakstängeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage nicht-ionische und/oder ionische Stoffe, z.B. aus den Klassen der Alkohol-POE- und/oder POP-Ether, Säure- und/oder POP- POE-Ester, Alkyl-Aryl- und/oder POP- POE-Ether, Fett- und/oder POP- POE-Addukte, POE- und/oder POP-Polyol Derivate, POE- und/oder POP-Sorbitan- oder Zucker-Addukte, Alky- oder Aryl-Sulfate, Sulfonate und Phosphate oder die entsprechenden PO-Ether-Addukte. Ferner geeignete Oligo- oder Polymere, z.B. ausgehend von vinylischen Monomeren, von Acrylsäure, aus EO und/oder PO allein oder in Verbindung mit z.B. (poly-) Alkoholen oder (poly-) Aminen. Ferner können Einsatz finden Lignin und seine Sulfonsäure-Derivate, einfache und modifizierte Cellulosen, aromatische und/oder aliphatische Sulfonsäuren sowie deren Addukte mit Formaldehyd.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Weitere Additive können Duftstoffe, mineralische oder vegetabile gegebenenfalls modifizierte Öle, Wachse und Nährstoffe (auch Spurennährstoffe), wie Salze von Eisen, Mangan, Bor, Kupfer, Kobalt, Molybdän und Zink sein.

Weiterhin enthalten sein können Stabilisatoren wie Kältestabilisatoren, Konservierungsmittel, Oxidationsschutzmittel, Lichtschutzmittel oder andere die chemische und/oder physikalische Stabilität verbessernde Mittel.

Die Formulierungen enthalten im allgemeinen zwischen 0,01 und 98 Gew.% Wirkstoff, vorzugsweise zwischen 0,5 und 90 %.

Der erfindungsgemäße Wirkstoff kann in seinen handelsüblichen Formulierungen sowie in den aus diesen Formulierungen bereiteten Anwendungsformen in Mischung mit anderen Wirkstoffen wie Insektiziden, Lockstoffen, Sterilantien, Bakteriziden, Akariziden, Nematiziden, Fungiziden, wachstumsregulierenden Stoffen, Herbiziden, Safenern, Düngemitteln oder Semiochemicals vorliegen.

Besonders günstige Mischpartner sind z.B. die folgenden:

### Fungizide:

### Inhibitoren der Nucleinsäure Synthese

Benalaxyl, Benalaxyl-M, Bupirimat, Chiralaxyl, Clozylacon, Dimethirimol, Ethirimol, Furalaxyl, Hymexazol, Metalaxyl, Metalaxyl-M, Ofurace, Oxadixyl, Oxolinsäure

### Inhibitoren der Mitose und Zellteilung

Benomyl, Carbendazim, Diethofencarb, Fuberidazole, Pencycuron, Thiabendazol, Thiophanat-methyl, Zoxamid

### Inhibitoren der Atmungskette Komplex I

Diflumetorim

### Inhibitoren der Atmungskette Komplex II

Boscalid, Carboxin, Fenfuram, Flutolanil, Furametpyr, Mepronil, Oxycarboxin, Penthiopyrad, Thifluzamid

### Inhibitoren der Atmungskette Komplex III

Azoxystrobin, Cyazofamid, Dimoxystrobin, Enestrobin, Famoxadon, Fenamidon, Fluoxastrobin, Kresoximmethyl, Metominostrobin, Orysastrobin, Pyraclostrobin, Picoxystrobin, Trifloxystrobin

### Entkoppler

Dinocap, Fluazinam

### Inhibitoren der ATP Produktion

Fentinacetat, Fentinchlorid, Fentinhydroxid, Silthiofam

### Inhibitoren der Aminosäure- und Proteinbiosynthese

Andoprim, Blasticidin-S, Cyprodinil, Kasugamycin, Kasugamycinhydrochlorid Hydrat, Mepanipyrim, Pyrimethanil

### Inhibitoren der Signal-Transduktion

Fenpiclonil, Fludioxonil, Quinoxyfen

### Inhibitoren der Fett- und Membran Synthese

Chlozolinat, Iprodion, Procymidon, Vinclozolin

Ampropylfos, Kalium-Ampropylfos, Edifenphos, Iprobenfos (IBP), Isoprothiolan, Pyrazophos

Tolclofos-methyl, Biphenyl

Iodocarb, Propamocarb, Propamocarb hydrochlorid

### Inhibitoren der Ergosterol Biosynthese

Fenhexamid,

Azaconazol, Bitertanol, Bromuconazol, Cyproconazol, Diclobutrazol, Difenoconazol, Diniconazol, Diniconazol-M, Epoxiconazol, Etaconazol, Fenbuconazol, Fluquinconazol, Flusilazol, Flutriafol, Furconazol, Furconazol-cis, Hexaconazol, Imibenconazol, Ipconazol, Metconazol, Myclobutanil, Paclobutrazol, Penconazol, Propiconazol, Prothioconazol, Simeconazol, Tebuconazol, Tetraconazol, Triadimefon, Triadimenol, Triticonazol, Uniconazol, Voriconazol, Imazalil, Imazalilsulfat, Oxpoconazol, Fenarimol, Flurprimidol, Nuarimol, Pyrifenox, Triforin, Pefurazoat, Prochloraz, Triflumizol, Viniconazol,

Aldimorph, Dodemorph, Dodemorphacetat, Fenpropimorph, Tridemorph, Fenpropidin, Spiroxamin,

Naftifin, Pyributicarb, Terbinafin

### Inhibitoren der Zellwand Synthese

Benthiavalicarb, Bialaphos, Dimethomorph, Flumorph, Iprovalicarb, Polyoxins, Polyoxorim, Validamycin A

### Inhibitoren der Melanin Biosynthese

Capropamid, Diclocymet, Fenoxanil, Phtalid, Pyroquilon, Tricyclazol

### Resistenzinduktion

Acibenzolar-S-methyl, Probenazol, Tiadinil

### Multisite

Captafol, Captan, Chlorothalonil, Kupfersalze wie: Kupferhydroxid, Kupfernaphthenat, Kupferoxychlorid, Kupfersulfat, Kupferoxid, Oxin-Kupfer und Bordeaux Mischung, Dichlofluanid, Dithianon, Dodin, Dodin freie Base, Ferbam, Folpet, Fluorofolpet, Guazatin, Guazatinacetat, Iminoctadin, Iminoctadinalbesilat, Iminoctadintriacetat, Mankupfer, Mancozeb, Maneb, Metiram, Metiram Zink, Propineb, Schwefel und Schwefelpräparate enthaltend Calciumpolysulphid, Thiram, Tolylfluanid, Zineb, Ziram

### Unbekannter Mechanismus

Amibromdol, Benthiazol, Bethoxazin, Capsimycin, Carvon, Chinomethionat, Chloropicrin, Cufraneb, Cyflufenamid, Cymoxanil, Dazomet, Debacarb, Diclomezine, Dichlorophen, Dicloran, Difenzoquat, Difenzoquat Methylsulphat, Diphenylamin, Ethaboxam, Ferimzon, flumetover, Flusulfamid, Fluopicolid, Fluoroimid, Hexachlorobenzol, 8-Hydroxychinolinsulfat, Irumamycin, Methasulphocarb, Metrafenon, Methyl Isothiocyanat, Mildiomycin, Natamycin, Nickel dimethyldithiocarbamat, Nitrothal-isopropyl, Octhilinon, Oxamocarb, Oxyfenthiin, Pentachlorophenol und Salze, 2-Phenylphenol und Salze, Piperalin, Propanosin -Natrium, Proquinazid, Pyrrolnitrin, Quintozen, Tecloftalam, Tecnazen, Triazoxid, Trichlamid, Zarilamid und 2,3,5,6-Tetrachlor-4-(methylsulfonyl)-pyridin, *N*-(4-Chlor-2-nitrophenyl)-*N*-ethyl-4-methyl-benzenesulfonamid, 2-Amino-4-methyl-N-phenyl-5-thiazolecarboxamid, 2-Chlor-*N*-(2,3-dihydro-1,1,3-trimethyl-1H-inden-4-yl)-3-pyridincarboxamid, 3-[5-(4-Chlorphenyl)-2,3-dimethylisoxazolidin-3-yl]pyridin, cis-1-(4-Chlorphenyl)-2-(1H-1,2,4-triazol-1-yl)-cycloheptanol, 2,4-Dihydro-5-methoxy-2-methyl-4-[[[[1-[3-(trifluoromethyl)-phenyl]-ethyliden]-amino]-oxy]-methyl]-phenyl]-3H-1,2,3-triazol-3-on (185336-79-2), Methyl 1-(2,3-dihydro-2,2-dimethyl-1H-inden-1-yl)-1H-imidazole-5-carboxylat, 3,4,5-Trichlor-2,6-pyridindicarbonitril, Methyl 2-[[[cyclopropyl[(4-methoxyphenyl) imino]methyl]thio]methyl]-.alpha.-(methoxymethylen)-benzacetat, 4-Chlor-alpha-propinyloxy-*N*-[2-[3-methoxy-4-(2-propinyloxy)phenyl]ethyl]-benzacetamide, (2S)-*N-*[2-[4-[[3-(4-chlorophenyl)-2-propinyl]oxy]-3-methoxyphenyl]ethyl]-3-methyl-2-[(methylsulfonyl)amino]-butanamid, 5-Chlor-7-(4-methylpiperidin-1-yl)-6-(2,4,6-trifluorophenyl)[1,2,4]triazolo[1,5-a]pyrimidin, 5-Chlor-6-(2,4,6-tritluorophenyl)-*N*-[(1R)-1,2,2-trimethylpropyl][1,2,4]triazolo[1,5-a]pyrimidin-7-amin, 5-Chlor-*N*-[(1R)-1,2-dimethylpropyl]-6-(2,4,6-trifluorophenyl) [1,2,4]triazolo[1,5-a]pyrimidin-7-amine, *N*-[1-(5-Brom-3-chloropyridin-2-yl)ethyl]-2,4-dichloronicotinamid, *N*-(5-Brom-3-chlorpyridin-2-yl)methyl-2,4-dichlornicotinamid, 2-Butoxy-6-iod-3-propyl-benzopyranon-4-on, *N*-{(Z)-[(cyclopropylmethoxy) imino][6-(difluormethoxy)-2,3-difluorphenyl]methyl}-2-benzacetamid, *N*-(3-Ethyl-3,5,5-trimethyl-cyclohexyl)-3-formylamino-2-hydroxy-benzamid, 2-[[[[1-[3(1Fluor-2-phenylethyl)oxy]phenyl]ethyliden]amino]oxy]methyl]-alpha-(methoxy-imino)-*N*-methyl-alphaE-benzacetamid, *N*-{2-[3-Chlor-5-(trifluormethyl)pyridin-2-yl]ethyl}-2-(trifluoromethyl)benzamid, *N*-(3',4'-dichlor-5-fluorbiphenyl-2-yl)-3-(difluormethyl)-1-methyl-1H-pyrazol-4-carboxamid, *N*-(6-Methoxy-3-pyridinyl)-cyclopropan carboxamid, 1-[(4-Methoxyphenoxy)methyl]-2,2-dimethylpropyl-1H-imidazol-1- carbonsäure, O-[1-[(4-Methoxyphenoxy)methyl]-2,2-dimethylpropyl]-1H-imidazol- 1- carbothioic acid, 2-(2-{[6-(3-Chlor-2-methylphenoxy)-5-fluorpyrimidin-4-yl]oxy}phenyl)-2-(methoxyimino)-*N-*methylacetamid

### Bakterizide:

Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

### Acetylcholinesterase (AChE) Inhibitoren

Carbamate,
zum Beispiel Alanycarb, Aldicarb, Aldoxycarb, Allyxycarb, Aminocarb, Bendiocarb, Benfuracarb, Bufencarb, Butacarb, Butocarboxim, Butoxycarboxim, Carbaryl, Carbofuran, Carbosulfan, Cloethocarb, Dimetilan, Ethiofencarb, Fenobucarb, Fenothiocarb, Formetanate, Furathiocarb, Isoprocarb, Metam-sodium, Methiocarb, Methomyl, Metolcarb, Oxamyl, Pirimicarb, Promecarb, Propoxur, Thiodicarb, Thiofanox, Trimethacarb, XMC, Xylylcarb, Triazamate

Organophosphate,
zum Beispiel Acephate, Azamethiphos, Azinphos (-methyl, -ethyl), Bromophos-ethyl, Bromfenvinfos (-methyl), Butathiofos, Cadusafos, Carbophenothion, Chlorethoxyfos, Chlorfenvinphos, Chlormephos, Chlorpyrifos (-methyl/-ethyl), Coumaphos, Cyanofenphos, Cyanophos, Chlorfenvinphos, Demeton-S-methyl, Demeton-S-methylsulphon, Dialifos, Diazinon, Dichlofenthion, Dichlorvos/DDVP, Dicrotophos, Dimethoate, Dimethylvinphos, Dioxabenzofos, Disulfoton, EPN, Ethion, Ethoprophos, Etrimfos, Famphur, Fenamiphos, Fenitrothion, Fensulfothion, Fenthion, Flupyrazofos, Fonofos, Formothion, Fosmethilan, Fosthiazate, Heptenophos, Iodofenphos, Iprobenfos, Isazofos, Isofenphos, Isopropyl O-salicylate, Isoxathion, Malathion, Mecarbam, Methacrifos, Methamidophos, Methidathion, Mevinphos, Monocrotophos, Naled, Omethoate, Oxydemeton-methyl, Parathion (-methyl/ethyl), Phenthoate, Phorate, Phosalone, Phosmet, Phosphamidon, Phosphocarb, Phoxim, Pirimiphos (-methyl/-ethyl), Profenofos, Propaphos, Propetamphos, Prothiofos, Prothoate, Pyraclofos, Pyridaphenthion, Pyridathion, Quinalphos, Sebufos, Sulfotep, Sulprofos, Tebupirimfos, Temephos, Terbufos, Tetrachlorvinphos, Thiometon, Triazophos, Triclorfon, Vamidothion

### Natrium-Kanal-Modulatoren / Spannungsabhängige Natrium-Kanal-Blocker

Pyrethroide,
zum Beispiel Acrinathrin, Allethrin (d-cis-trans, d-trans), Beta-Cyfluthrin, Bifenthrin, Bioallethrin, Bioallethrin-S-cyclopentyl-isomer, Bioethanomethrin, Biopermethrin, Bioresmethrin, Chlovaporthrin, Cis-Cypermethrin, Cis-Resmethrin, Cis-Permethrin, Clocythrin, Cycloprothrin, Cyfluthrin, Cyhalothrin, Cypermethrin (alpha-, beta-, theta-, zeta-), Cyphenothrin, Deltamethrin, Empenthrin (1R-isomer), Esfenvalerate, Etofenprox, Fenfluthrin, Fenpropathrin, Fenpyrithrin, Fenvalerate, Flubrocythrinate, Flucythrinate, Flufenprox, Flumethrin, Fluvalinate, Fubfenprox, Gamma-Cyhalothrin, Imiprothrin, Kadethrin, Lambda-Cyhalothrin, Metofluthrin, Permethrin (cis-, trans-), Phenothrin (1R-trans isomer), Prallethrin, Profluthrin, Protrifenbute, Pyresmethrin, Resmethrin, RU 15525, Silafluofen, Tau-Fluvalinate, Tefluthrin, Terallethrin, Tetramethrin (-1R- isomer), Tralomethrin, Transfluthrin, ZXI 8901, Pyrethrins (pyrethrum)
DDT
Oxadiazine,
zum Beispiel Indoxacarb
Semicarbazon,
zum Beispiel Metaflumizon (BAS3201)

### Acetylcholin-Rezeptor-Agonisten/-Antagonisten

Chloronicotinyle,
zum Beispiel Acetamiprid, Clothianidin, Dinotefuran, Imidacloprid, Nitenpyram, Nithiazine, Thiacloprid, Imidaclothiz, AKD-1022, Thiamethoxam
Nicotine, Bensultap, Cartap

### Acetylcholin-Rezeptor-Modulatoren

Spinosyne,
zum Beispiel Spinosad, Spinetoram (XDE-175)

### GABA-gesteuerte Chlorid-Kanal-Antagonisten

Organochlorine,
zum Beispiel Camphechlor, Chlordane, Endosulfan, Gamma-HCH, HCH, Heptachlor, Lindane, Methoxychlor
Fiprole,
zum Beispiel Acetoprole, Ethiprole, Fipronil, Pyrafluprole, Pyriprole, Vaniliprole

### Chlorid-Kanal-Aktivatoren

Mectine,
zum Beispiel Abamectin, Emamectin, Emamectin-benzoate, Ivermectin, Lepimectin, Milbemycin

### Juvenilhonmon-Mimetika,

zum Beispiel Diofenolan, Epofenonane, Fenoxycarb, Hydroprene, Kinoprene, Methoprene, Pyriproxifen, Triprene

### Ecdysonagonisten/disruptoren

Diacylhydrazine,
zum Beispiel Chromafenozide, Halofenozide, Methoxyfenozide, Tebufenozide

### Inhibitoren der Chitinbiosynthese

Benzoylharnstoffe,
zum Beispiel Bistrifluron, Chlofluazuron, Diflubenzuron, Fluazuron, Flucycloxuron, Flufenoxuron, Hexaflumuron, Lufenuron, Novaluron, Noviflumuron, Penfluron, Teflubenzuron, Triflumuron
Buprofezin
Cyromazine

### Inhibitoren der oxidativen Phosphorylierung, ATP-Disruptoren

Diafenthiuron

Organozinnverbindungen,
zum Beispiel Azocyclotin, Cyhexatin, Fenbutatin-oxide

### Entkoppler der oxidativen Phosphorylierung durch Unterbrechung des H-Protongradienten

Pyrrole,
zum Beispiel Chlorfenapyr

Dinitrophenole,
zum Beispiel Binapacyrl, Dinobuton, Dinocap, DNOC

### Seite-I-Elektronentransportinhibitoren

METI's,
zum Beispiel Fenazaquin, Fenpyroximate, Pyrimidifen, Pyridaben, Tebufenpyrad, Tolfenpyrad

Hydramethylnon

Dicofol

### Seite-II-Elektronentransportinhibitoren

Rotenone

### Seite-III-Elektronentransportinhibitoren

Acequinocyl, Fluacrypyrim

### Mikrobielle Disruptoren der Insektendarmmembran

Bacillus thuringiensis-Stämme

### Inhibitoren der Fettsynthese

Tetronsäuren,
zum Beispiel Spirodiclofen, Spiromesifen

Tetramsäuren,
zum Beispiel Spirotetramat

Carboxamide,
zum Beispiel Flonicamid

Oktopaminerge Agonisten,
zum Beispiel Amitraz

### Inhibitoren der Magnesium-stimulierten ATPase,

Propargite

Nereistoxin-Analoge,
zum Beispiel Thiocyclam hydrogen oxalate, Thiosultap-sodium

### Agonisten des Ryanodin-Rezeptors,

Benzoesäuredicarboxamide,
zum Beispiel Flubendiamid

Anthranilam ide,
zum Beispiel Rynaxypyr (3-Bromo-*N*-{4-chloro-2-methyl-6-[(methylamino)carbonyl]-phenyl}-1-(3-chloropyridin-2-yl)-1H-pyrazole-5-carboxamide)

### Biologika, Hormone oder Pheromone

Azadirachtin, Bacillus spec., Beauveria spec., Codlemone, Metarrhizium spec., Paecilomyces spec., Thuringiensin, Verticillium spec.

### Wirkstoffe mit unbekannten oder nicht spezifischen Wirkmechanismen

Begasungsmittel,
zum Beispiel Aluminium phosphide, Methyl bromide, Sulfuryl fluoride

Fraßhemmer,
zum Beispiel Cryolite, Flonicarnid, Pymetrozine

Milbenwachstumsinhibitoren,
zum Beispiel Clofentezine, Etoxazole, Hexythiazox

Amidoflumet, Benclothiaz, Benzoximate, Bifenazate, Bromopropylate, Buprofezin, Chinomethionat, Chlordimeform, Chlorobenzilate, Chloropicrin, Clothiazoben, Cycloprene, Cyflumetofen, Dicyclanil, Fenoxacrim, Fentrifanil, Flubenzimine, Flufenerim, Flutenzin, Gossyplure, Hydramethylnone, Japonilure, Metoxadiazone, Petroleum, Piperonyl butoxide, Potassium oleate, Pyridalyl, Sulfluramid, Tetradifon, Tetrasul, Triarathene,Verbutin

Auch eine Mischung mit anderen bekannten Wirkstoffen, wie Herbiziden, Düngemitteln, Wachstumsregulatoren, Safenern, Semiochemicals, oder auch mit Mitteln zur Verbesserung der Pflanzeneigenschaften ist möglich.

Die erfindungsgemäßen Wirkstoffe können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne dass der zugesetzte Synergist selbst aktiv wirksam sein muss.

Die erfindungsgemäßen Wirkstoffe können ferner beim Einsatz als Insektizide in ihren handelsüblichen Formulierungen sowie in den aus diesen Formulierungen bereiteten Anwendungsformen in Mischungen mit Hemmstoffen vorliegen, die einen Abbau des Wirkstoffes nach Anwendung in der Umgebung der Pflanze, auf der Oberfläche von Pflanzenteilen oder in pflanzlichen Geweben vermindern.

Der Wirkstoffgehalt der aus den handelsüblichen Formulierungen bereiteten Anwendungsformen kann in weiten Bereichen variieren. Die Wirkstoffkonzentration der Anwendungsformen kann von 0,00000001 bis zu 95 Gew.-% Wirkstoff, vorzugsweise zwischen 0,00001 und 1 Gew.-% liegen.

Die Anwendung geschieht in einer den Anwendungsformen angepassten üblichen Weise.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stängel, Stämme, Blüten, Fruchtkörper, Früchte und Saatgut sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Saatgut.

Die erfindungsgemäße Behandlung der Pflanzen und Pflanzenteile mit den erfindungsgemäβen Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen, Injizieren und bei Vermehrungsmaterial, insbesondere bei Saatgut, weiterhin durch ein- oder mehrschichtiges Umhüllen.

Die erfindungsgemäße Behandlung der Pflanzen und Pflanzenteile mit den Wirkstoffkombinationen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Ausstreichen und bei Vermehrungsmaterial, insbesondere bei Saatgut, weiterhin durch ein- oder mehrschichtiges Umhüllen.

Insbesondere eignen sich die erfindungsgemäßen Mischungen zur Behandlung von Saatgut. Bevorzugt sind dabei die vorstehend als bevorzugt oder besonders bevorzugt genannten erfindungsgemäßen Kombinationen zu nennen. So entsteht ein großer Teil des durch Schädlinge verursachten Schadens an Kulturpflanzen bereits durch den Befall des Saatguts während der Lagerung und nach dem Einbringen des Saatguts in den Boden sowie während und unmittelbar nach der Keimung der Pflanzen. Diese Phase ist besonders kritisch, da die Wurzeln und Sprosse der wachsenden Pflanze besonders empfindlich sind und bereits ein geringer Schaden zum Absterben der ganzen Pflanze führen kann. Es besteht daher ein insbesondere großes Interesse daran, das Saatgut und die keimende Pflanze durch den Einsatz geeigneter Mittel zu schützen.

Die Bekämpfung von Schädlingen durch die Behandlung des Saatguts von Pflanzen ist seit langem bekannt und ist Gegenstand ständiger Verbesserungen. Dennoch ergeben sich bei der Behandlung von Saatgut eine Reihe von Problemen, die nicht immer zufriedenstellend gelöst werden können. So ist es erstrebenswert, Verfahren zum Schutz des Saatguts und der keimenden Pflanze zu entwickeln, die das zusätzliche Ausbringen von Pflanzenschutzmitteln nach der Saat oder nach dem Auflaufen der Pflanzen überflüssig machen. Es ist weiterhin erstrebenswert, die Menge des eingesetzten Wirkstoffs dahingehend zu optimieren, dass das Saatgut und die keimende Pflanze vor dem Befall durch Schädlinge bestmöglich geschützt wird, ohne jedoch die Pflanze selbst durch den eingesetzten Wirkstoff zu schädigen. Insbesondere sollten Verfahren zur Behandlung von Saatgut auch die intrinsischen insektiziden Eigenschaften transgener Pflanzen einbeziehen, um einen optimalen Schutz des Saatguts und auch der keimenden Pflanze bei einem minimalen Aufwand an Pflanzenschutzmitteln zu erreichen.

Die vorliegende Erfindung bezieht sich daher insbesondere auch auf ein Verfahren zum Schutz von Saatgut und keimenden Pflanzen vor dem Befall von Schädlingen, indem das Saatgut mit einem erfindungsgemäßen Mittel behandelt wird. Die Erfindung bezieht sich ebenfalls auf die Verwendung der erfindungsgemäßen Mittel zur Behandlung von Saatgut zum Schutz des Saatguts und der daraus entstehenden Pflanze vor Schädlingen. Weiterhin bezieht sich die Erfindung auf Saatgut, welches zum Schutz vor Schädlingen mit einem erfindungsgemäßen Mittel behandelt wurde.

Einer der Vorteile der vorliegenden Erfindung ist es, dass aufgrund der besonderen systemischen Eigenschaften der erfindungsgemäßen Mittel die Behandlung des Saatguts mit diesen Mitteln nicht nur das Saatgut selbst, sondern auch die daraus hervorgehenden Pflanzen nach dem Auflaufen vor Schädlingen schützt. Auf diese Weise kann die unmittelbare Behandlung der Kultur zum Zeitpunkt der Aussaat oder kurz danach entfallen.

Ein weiterer Vorteil besteht in der synergistischen Erhöhung der insektiziden Wirksamkeit der erfindungsgemäßen Mittel gegenüber dem insektiziden Einzelwirkstoff, die über die zu erwartende Wirksamkeit der beiden einzeln angewendeten Wirkstoffe hinausgeht. Vorteilhaft ist auch die synergistische Erhöhung der fungiziden Wirksamkeit der erfindungsgemäßen Mittel gegenüber dem fungiziden Einzelwirkstoff, die über die zu erwartende Wirksamkeit des einzeln angewendeten Wirkstoffs hinausgeht. Damit wird eine Optimierung der Menge der eingesetzten Wirkstoffe ermöglicht.

Ebenso ist es als vorteilhaft anzusehen, dass die erfindungsgemäßen Mischungen insbesondere auch bei transgenem Saatgut eingesetzt werden können, wobei die aus diesem Saatgut hervorgehenden Pflanzen zur Expression eines gegen Schädlinge gerichteten Proteins befähigt sind. Durch die Behandlung solchen Saatguts mit den erfindungsgemäßen Mitteln können bestimmte Schädlinge bereits durch die Expression des z.B. insektiziden Proteins kontrolliert werden, und zusätzlich durch die erfindungsgemäßen Mittel vor Schäden bewahrt werden.

Die erfindungsgemäßen Mittel eignen sich zum Schutz von Saatgut jeglicher Pflanzensorte wie bereits vorstehend genannt, die in der Landwirtschaft, im Gewächshaus, in Forsten oder im Gartenbau eingesetzt wird. Insbesondere handelt es sich dabei um Saatgut von Mais, Erdnuss, Canola, Raps, Mohn, Soja, Baumwolle, Rübe (z.B. Zuckerrübe und Futterrübe), Reis, Hirse, Weizen, Gerste, Hafer, Roggen, Sonnenblume, Tabak, Kartoffeln oder Gemüse (z.B. Tomaten, Kohlgewächs). Die erfindungsgemäßen Mittel eignen sich ebenfalls zur Behandlung des Saatguts von Obstpflanzen und Gemüse wie vorstehend bereits genannt. Besondere Bedeutung kommt der Behandlung des Saatguts von Mais, Soja, Baumwolle, Weizen und Canola oder Raps zu.

Wie vorstehend bereits erwähnt, kommt auch der Behandlung von transgenem Saatgut mit einem erfindungsgemäßen Mittel eine besondere Bedeutung zu. Dabei handelt es sich um das Saatgut von Pflanzen, die in der Regel zumindest ein heterologes Gen enthalten, das die Expression eines Polypeptids mit insbesondere insektiziden Eigenschaften steuert. Die heterologen Gene in transgenem Saatgut können dabei aus Mikroorganismen wie Bacillus, Rhizobium, Pseudomonas, Serratia, Trichoderma, Clavibacter, Glomus oder Gliocladium stammen. Die vorliegende Erfindung eignet sich besonders für die Behandlung von transgenem Saatgut, das zumindest ein heterologes Gen enthält, das aus Bacillus sp. stammt und dessen Genprodukt Wirksamkeit gegen Maiszünsler und/oder Maiswurzel-Bohrer zeigt. Besonders bevorzugt handelt es sich dabei um ein heterologes Gen, das aus Bacillus thuringiensis stammt.

Im Rahmen der vorliegenden Erfindung wird das erfindungsgemäßes Mittel alleine oder in einer geeigneten Formulierung auf das Saatgut aufgebracht. Vorzugsweise wird das Saatgut in einem Zustand behandelt, in dem so stabil ist, dass keine Schäden bei der Behandlung auftreten. Im Allgemeinen kann die Behandlung des Saatguts zu jedem Zeitpunkt zwischen der Ernte und der Aussaat erfolgen. Üblicherweise wird Saatgut verwendet, das von der Pflanze getrennt und von Kolben, Schalen, Stängeln, Hülle, Wolle oder Fruchtfleisch befreit wurde.

Im Allgemeinen muss bei der Behandlung des Saatguts darauf geachtet werden, dass die Menge des auf das Saatgut aufgebrachten erfindungsgemäßen Mittels und/oder weiterer Zusatzstoffe so gewählt wird, dass die Keimung des Saatguts nicht beeinträchtigt bzw. die daraus hervorgehende Pflanze nicht geschädigt wird. Dies ist vor allem bei Wirkstoffen zu beachten, die in bestimmten Aufwandmengen phytotoxische Effekte zeigen können.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Die Begriffe "Teile" bzw. "Teile von Pflanzen" oder "Pflanzenteile" wurden oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Zuckerrüben, Tomaten, Erbsen und andere Gemüsesorten, Baumwolle, Tabak, Raps, sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besonders hervorgehoben die erhöhte Abwehr der Pflanzen gegen Insekten, Spinnentiere, Nematoden und Schnecken durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucotn® (Baumwolle) und NewLeaf® (Kartoffel) vertrieben werden. Als Beispiele für Herbizid-tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid- resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel I bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ekto- und Endoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:

Aus der Ordnung der Anoplurida z.B. Haematopinus spp., Linognathus spp., Pediculus spp., Phtirus spp., Solenopotes spp..

Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp..

Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..

Aus der Ordnung der Siphonapterida z.B. Pulex spp., Ctenocephalides spp., Xenopsylla spp., Ceratophyllus spp..

Aus der Ordnung der Heteropterida z.B. Cimex spp., Triatoma spp., Rhodnius spp., Panstrongylus spp..

Aus der Ordnung der Blattarida z.B. Blatta orientalis, Periplaneta americana, Blattela germanica, Supella spp..

Aus der Unterklasse der Acari (Acarina) und den Ordnungen der Meta- sowie Mesostigmata z.B. Argas spp., Omithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Stemostoma spp., Varroa spp..

Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor und bei der Tierhaltung in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändem, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, dass die erfindungsgemäßen Verbindungen eine hohe insektizide Wirkung gegen Insekten zeigen, die technische Materialien zerstören.

Beispielhaft und vorzugsweise - ohne jedoch zu limitieren - seien die folgenden Insekten genannt:

Käfer wie Hylotrupes bajulus, Chlorophorus pilosis, Anobium punctatum, Xestobium rufovillosum, Ptilinus pecticomis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus;

Hautflügler wie Sirex juvencus, Urocerus gigas, Urocerus gigas taignus, Urocerus augur;

Termiten wie Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus;

Borstenschwänze wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel.

Die anwendungsfertigen Mittel können gegebenenfalls noch weitere Insektizide und gegebenenfalls noch ein oder mehrere Fungizide enthalten.

Hinsichtlich möglicher zusätzlicher Zumischpartner sei auf die oben genannten Insektizide und Fungizide verwiesen.

Zugleich können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Weiter können die erfindungsgemäßen Verbindungen allein oder in Kombinationen mit anderen Wirkstoffen als Antifouling-Mittel eingesetzt werden.

Die Wirkstoffe eignen sich auch zur Bekämpfung von tierischen Schädlingen im Haushalts-, Hygiene- und Vorratsschutz, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam. Zu diesen Schädlingen gehören:

Aus der Ordnung der Scorpionidea z.B. Buthus occitanus.

Aus der Ordnung der Acarina z.B. Argas persicus, Argas reflexus, Bryobia ssp., Dermanyssus gallinae, Glyciphagus domesticus, Ornithodorus moubat, Rhipicephalus sanguineus, Trombicula alfreddugesi, Neutrombicula autumnalis, Dermatophagoides pteronissimus, Dermatophagoides forinae.

Aus der Ordnung der Araneae z.B. Aviculariidae, Araneidae.

Aus der Ordnung der Opiliones z.B. Pseudoscorpiones chelifer, Pseudoscorpiones cheiridium, Opiliones phalangium.

Aus der Ordnung der Isopoda z.B. Oniscus asellus, Porcellio scaber.

Aus der Ordnung der Diplopoda z.B. Blaniulus guttulatus, Polydesmus spp..

Aus der Ordnung der Chilopoda z.B. Geophilus spp..

Aus der Ordnung der Zygentoma z.B. Ctenolepisma spp., Lepisma saccharina, Lepismodes inquilinus.

Aus der Ordnung der Blattaria z.B. Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.

Aus der Ordnung der Saltatoria z.B. Acheta domesticus.

Aus der Ordnung der Dermaptera z.B. Forficula auricularia.

Aus der Ordnung der Isoptera z.B. Kalotermes spp., Reticulitermes spp.

Aus der Ordnung der Psocoptera z.B. Lepinatus spp., Liposcelis spp.

Aus der Ordnung der Coleoptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.

Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga carnaria, Simulium spp., Stomoxys calcitrans, Tipula paludosa.

Aus der Ordnung der Lepidoptera z.B. Achroia grisella, Galleria mellonella, Plodia interpunctella, Tinea cloacella, Tinea pellionella, Tineola bisselliella.

Aus der Ordnung der Siphonaptera z.B. Ctenocephalides canis, Ctenocephalides felis, Pulex irritans, Tunga penetrans, Xenopsylla cheopis.

Aus der Ordnung der Hymenoptera z.B. Camponotus hereuleanus, Lasius fuliginosus, Lasius niger, Lasius umbratus, Monomorium pharaonis, Paravespula spp., Tetramorium caespitum.

Aus der Ordnung der Anoplura z.B. Pediculus humanus capitis, Pediculus humanus corporis, Pemphigus spp., Phylloera vastatrix, Phthirus pubis.

Aus der Ordnung der Heteroptera z.B. Cimex hemipterus, Cimex lectularius, Rhodinus prolixus, Triatoma infestans.

Die Anwendung im Bereich der Haushaltsinsektizide erfolgt allein oder in Kombination mit anderen geeigneten Wirkstoffen wie Phosphorsäureestern, Carbamaten, Pyrethroiden, Neonicotinoiden, Wachstumsregulatoren oder Wirkstoffen aus anderen bekannten Insektizidklassen.

Die Anwendung erfolgt in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

### Herstellungsbeispiele:

### Methode 1

### Beispiel I-1 1-[(6-Chlorpyridin-3-yl)methyl]-2,3-dibydro-1H-furo [2,3-b][1,4]oxazin-6(4aH)-on

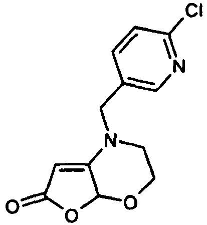

Eine Mischung aus 0.6 mL (1.50 mmol) einer 2.5 M Lösung von n-Butyllithium in Hexan und 3 mL Tetrahydrofuran werden bei -78°C mit einer ebenfalls auf -78°C gekühlten Lösung aus 202 mg (0.75 mmol) 4-[[(6-Chlorpyridin-3-yl)methyl](2-hydroxyethyl)amino]furan-2(5H)-on (II-1) in 7 mL Tetrahydrofuran versetzt. Nach 20 Minuten Rühren bei -78°C gibt man portionsweise 39 µL (0.75 mmol) Brom hinzu und rührt weitere 30 min bei -78°C. Nach Zugabe von gesättigter wässriger Ammoniumchlorid-Lösung wird auf Raumtemperatur aufgewärmt und mit Essigsäureethylester extrahiert. Die vereinigte organische Phase wird über Magnesiumsulfat getrocknet und im Vakuum eingeengt. Nach Reinigung des Rückstandes durch Säulenchromatographie über Kieselgel (Kieselgel 60, Merck, Korngröße: 0.04 bis 0.063 mm) mit dem Laufmittelgemisch Dichlormethan : Methanol (95:5) erhält man 67 mg (32 % der Theorie) 1-[(6-Chlorpyridin-3-yl)methyl]-2,3-dihydro-1H-furo[2,3-b][1,4]oxazin-6(4aH)-on.
¹H-NMR (CDCl₃): δ [ppm] = 3.16 (m, 1 H), 3.26 (m, 1H), 4.10 (m, 2 H), 4.19 (d, 1 H), 4.40 (d, 1 H), 4.88 (s, 1 H), 5.72 (s, 1 H), 7.38 (d, 1 H), 7.61 (dd, 1 H), 8.35 (d, 1 H).

### Beispiel I-2 4-[(6-Chlorpyridin-3-yl)methyl]-5,6,7,7a-tetrahydrofuro [3,2-b]pyridin-2(4H)-on

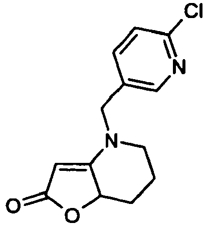

31.55 g (80.36 mmol) 4-[[(6-Chlorpyridin-3-yl)methyl](3-iodpropyl)amino]furan-2(5H)-on (II-3) werden in 900 mL Tetrahydrofuran gelöst, auf -78°C abgekühlt und mit 42.19 mL (84.38 mmol) einer 2.0 M Lösung von Lithiumdiisopropylamid in Tetrahydrofuran versetzt. Nach 10 Minuten Rühren bei -78°C wärmt man auf Raumtemperatur auf und rührt weitere 30 Minuten bei Raumtemperatur. Nach Zugabe von 20 mL Methanol wird im Vakuum eingeengt. Reinigung des Rückstandes durch Säulenchromatographie über Kieselgel (Kieselgel 60, Merck, Korngröße: 0.04 bis 0.063 mm) mit dem Laufmittel Essigsäureethylester liefern 18.05 g (85 % der Theorie) 4-[(6-Chlorpyridin-3-yl)methyl]-5,6,7,7a-tetrahydrofuro[3,2-b]pyridin-2(4H)-on.
¹H-NMR (CD₃CN): δ [ppm] = 1.48 (m, 1 H), 1.85-2.00 (m, 2 H), 2.32 (m, 1 H), 3.08 (m, 1 H), 3.27 (m, 1 H), 4.25 (d, 1 H), 4.40 (d, 1 H), 4.64 (s, 1 H), 4.76(dd, 1 H), 7.37 (d, 1 H), 7.67 (dd, 1 H), 8.31 (d, 1 H).

Enantiomerentrennung von 4-[(6-Chlorpyridin-3-yl)methyl]-5,6,7,7a-tetrahydrofuro [3,2-b]pyridin-2(4H)-on (Beispiel 1-2) durch präparative HPLC an einer chiralen Phase.

### Beispiel I-3 Röntgenstrukturbestimmung:

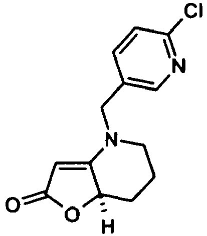

Für die Röntgenstrukturbestimmung geeignete Kristalle wurden durch Kristallisation aus Aceton erhalten. Die Bestimmung der Gitterkonstanten und die Erfassung der Reflexintensitäten erfolgten mit einem Siemens P4 Diffraktometer. Die Strukturlösung erfolgte mit Hilfe direkter Methoden (Programmsystem SHELXTL Version 5.10). Die Verfeinerung der Struktur erfolgte mit dem Programm SHELXTL Version 6.10 gegen F².

### Kristalldaten und Strukturverfeinerung:

| | | |
|---|---|---|
| Summenformel | C₁₃H₁₃ClN₂O₂ | |
| Molmasse | 264.70 | |
| Temperatur | 153 K | |
| Wellenlänge | 0.71073 Å | |
| Kristallsystem | Monoklin | |
| Raumgruppe | *P*2₁ | |
| Zelldimensionen | a = 5.4002(2) Å | α= 90°. |
| | b = 8.9237(3) Å | β= 96.8250(10)°. |
| | c = 13.0576(5) Å | γ=90°. |
| Zellvolumen | 624.78(4) Å3 | |
| Formeleinheiten pro Zelle Z | 2 | |
| Dichte (berechnet) | 1.407 Mg/m³ | |
| Absorptionskoeffizient | 0.301 mm⁻¹ | |
| F(000) | 276 | |
| Kristallabmessungen | 0.04 x 0.20 x 0.25 mm³ | |
| Theta-Bereich für Datensammlung | 1.57 bis 31.49°. | |
| Index-Bereich | -7<=h<=7, -13<=k<=13, -19<=1<=19 | |
| Gemessene Reflexe | 9509 | |
| Unabhängige Reflexe | 3964 [R(int) = 0.0363] | |
| Vollständigkeit zu Theta = 31.49° | 96.6 % | |
| Absorptionskorrektur | SADABS (Bruker-AXS) | |
| Verfeinerungsmethode | Vollmatrix kleinste Quadrate an F² | |
| Daten / Restraints / Parameter | 3964/1/173 | |
| Goodness-of-fit an F2 | 1.003 | |
| Endgültige R Indices [I>2sigma(I)] | R1 = 0.0443, wR2 = 0.1084 | |
| R-Werte (sämtliche Daten) | R1 = 0.0478, wR2 = 0.1106 | |
| Größtes Maximum und Minimum | 0.256 and -0.387 e.Å⁻³ | |

### Beispiel I-4

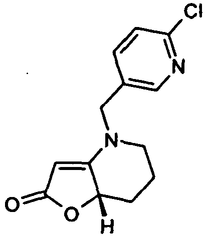

¹H-NMR (CDCl₃): δ [ppm] = 1.59 (m, 1 H), 1.85-2.05 (m, 2 H), 2.44 (m, 1 H), 3.02 (m, 1 H), 3.23 (m, 1 H), 4.16 (d, 1 H), 4.36 (d, 1 H), 4,74 (dd, 1 H), 4.79 (s, 1 H), 7.36 (d, 1 H), 7.58 (dd, 1 H), 8.31 (d, 1 H).

### Methode 2

### Beispiel I-5 4-[(6-Chlorpyridin-3-yl)methyl]-6-methylen-5,6,7,7a-tetrahydrofuro [3,2-b]pyridin-2(4H)-on

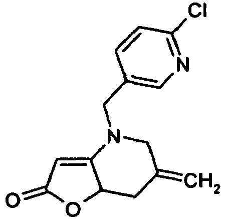

540 mg (1.55 mmol) 5-[2-({[(6-Chlorpyridin-3-yl)methyl]amino}methyl)prop-2-en-1-yl]-4-pyrrolidin-1-ylfuran-2(5H)-on (III-1) werden in 20 mL Essigsäure 2 Stunden bei 100°C gerührt. Nach Einengen der Reaktionsmischung im Vakuum nimmt man mit Dichlormethan auf und wäscht mit Wasser. Die wässrige Phase wird zweimal mit Dichlormethan extrahiert. Die vereinigte organische Phase wird mit 1 N wässriger Natriumhydroxid-Lösung gewaschen und die wässrige Phase (Natriumhydroxid-Lösung) wiederum zweimal mit Dichlormethan extrahiert. Die vereinigte organische Phase wird über Natriumsulfat getrocknet und im Vakuum eingeengt. Nach Reinigung des Rückstandes durch Umkristallisation aus Essigsäureethylester erhält man 407 mg (94 % der Theorie) 4-[(6-Chlorpyridin-3-yl)methyl]-6-methylen-5,6,7,7a-tetrahydrofuro[3,2-b]pyridin-2(4H)-on.
¹H-NMR (CD₃CN): δ [ppm] = 2.26 (td, 1 H), 3.08 (dd, 1 H), 3.77 (d, 1 H), 3.99 (d, 1 H), 4.35 (d, 1 H), 4.45 (d, 1 H), 4.65 (s, 1 H), 4,81 (dd, 1 H), 5.03 (m, 2 H), 7.37 (d, 1 H), 7.65 (dd, 1 H), 8.30 (d, 1 H).

### Methode 3

### Beispiel I-6 4-[(6-Chlorpyridin-3-yl)methyl]-4,5,8,8a-tetrahydro-2H-furo [3,2-b]azepin-2-on

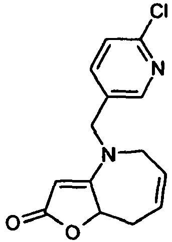

40 mg (0.13 mmol) 5-Allyl-4-{allyl[(6-chlorpyridin-3-yl)methyl]amino}furan-2(5H)on (VI-1) werden in 4 mL Dichlormethan gelöst, mit 11 mg (0.013 mmol) Grubbs Katalysator 2. Generation versetzt und 2 Stunden bei Raumtemperatur gerührt. Einengen im Vakuum und Reinigung des Rückstandes durch Säulenchromatographie über Kieselgel (Kieselgel 60, Merck, Kerngröße: 0.04 bis 0.063 mm) mit dem Laufmittel Essigsäureethylester liefern 29 mg (80 % der Theorie) 4-[(6-Chlorpyridin-3-yl)methyl]-4,5,8,8a-tetrahydro-2H-furo[3,2-b]azepin-2-on.
¹H-NMR (CDCl₃): δ [ppm] = 2.38 (m, 1 H), 2.97 (m, 1 H), 3.38 (dd, 1 H), 4.31 (m, 1 H), 4.39 (d, 1 H), 4.49 (d, 1 H), 4.69 (s, 1 H), 5.31 (dd, 1 H), 5.67 (m, 1 H), 5.80 (m, 1 H), 7.33 (d, 1 H), 7.53 (dd, 1 H), 8.30 (d, 1 H).

### Beispiel I-7 4-[(6-chlorpyridin-3-yl)methyl]-4,5,6,7,8,8a-hexahydro-2H-furo [3,2-b]azepin-2-on

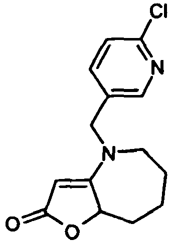

10 mg (0.036 mmol) 4-[(6-Chlorpyridin-3-yl)methyl]-4,5,8,8a-tetrahydro-2H-furo[3,2-b]azepin-2-on (1-6) werden in 10 mL Toluol gelöst, mit 5mg (0.005 mmol) Tris(triphenylphosphin)-rhodium(I)chlorid und 4 mg (0.015 mmol) Triphenylphosphin versetzt und 24 Stunden unter 3 bar Wasserstoff bei 110-120°C hydriert. Man filtriert und engt das Filtrat im Vakuum ein. Reinigung des Rückstandes durch Säulenchromatographie über Kieselgel (Kieselgel 60, Merck, Korngröße: 0.04 bis 0.063 mm) mit dem Laufmittelgemisch Essigsäureethylester : Cyclohexan (2:1 bis 4:1) liefert 8 mg (79 % der Theorie) 4-[(6-chlorpyridin-3-yl)methyl]-4,5,6,7,8,8a-hexahydro-2H-furo-[3,2-b]azepin-2-on.
¹H-NMR (CDCl₃): δ [ppm] = 1.40 (m, 1 H), 1.51-1.70 (m, 2 H), 1.88 (m, 1 H), 2.05 (m, 1 H, 2.40 (m, 1 H), 3.24 (dd, 1 H), 3.40 (dd, 1 H), 4.40 (m, 2 H), 4.67 (s, 1 H), 4.95 (dd, 1 H), 7.35 (d, 1 H), 7.57 (dd, 1 H), 8.30 (d, 1 H).

### Methode 4

### Beispiel I-8 1-[(6-Chlorpyridin-3-yl)methyl]-2,3-dihydro-1H-furo [4,5d][1,3]oxazin-5(3aH)-on

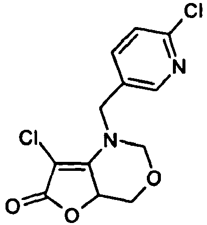

139,0 mg (0,48 mmol) 2-Chlor-5-hydroxymethyl-4-{[(6-chlorpyridin-3-yl)methyl]amino}furan-2(5H)-on (VII-1) werden in einem Gemisch aus 34,75 ml Toluol und 5 ml *N,N*-Dimethylformamid (DMF) vorgelegt und mit 173,2 mg (5,76 mmol) Paraformaldehyd und 20,8 mg (0,11 mmol) *para-*Toluolsulfonsäure-monohydrat versetzt. Anschließend wird das Reaktionsgemisch ca. 18 Stunden unter Rühren am bei Rückflusstemperatur am Wasserabscheider erhitzt. Nach Einengen der organischen Phase im Vakuum wird der verbleibende Rückstand mittels präp. HPLC (neutral) gereinigt. Man erhält 54 mg (33 % der Theorie) 1-[(6-Chlorpyridin-3-yl)methyl]-2,3-dihydro-1H-furo[4,5-d][1,3]oxazin-5(3aH)-on.
LC-MS (*m*/*z*): 301 (M⁺) C₁₂H₁₀Cl₂N₂O₃ (301,1)
¹³C-NMR mit ¹H-NMR-Entkopplung. (DMF-d₇, 400 MHz): δ [ppm] = 49.2 (CH₂-N); 66.3 (CH₂-O); 70.8 (O-CH), 78.9 (N-CH₂-O-); 85.0 (=C-Cl); 133.6 (C-Py); 125,1, 139.3, 149.6 (CH-Py); 150.7 (C-Cl-Py); 158.9 (=C-); 169.5 (CO-O).

### Halogenierung (R² = Halogen)

### Beispiel I-9 3-Chlor-4-[(6-chlorpyridin-3-yl)methyl]-5,6,7,7a-tetrahydrofuro [3,2-b]pyridin-2(4H)-on

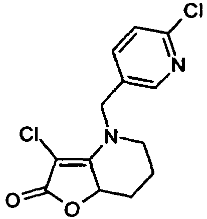

524 mg (1.98 mmol) 4-[(6-Chlorpyridin-3-yl)methyl]-5,6,7,7a-tetrahydrofuro[3,2-b]pyridin-2(4H)-on (1-2) werden in 20 mL Acetonitril gelöst und bei Raumtemperatur mit 414 µL (2.97 mmol) Triethylamin und 317 mg (2.37 mmol) N-Chlorsuccinimid versetzt. Nach einer Stunde Rühren werden weitere 159 mg (1.19 mmol) N-Chlorsuccinimid zugegeben. Nach einer weiteren Stunde Rühren wird der gesamte Reaktionsansatz im Vakuum eingeengt. Man nimmt den Rückstand mit Dichlormethan auf, wäscht nacheinander zweimal mit 1 N wässriger Salzsäure, zweimal mit 1 N wässriger Natriumhydroxid-Lösung und gesättigter Natriumchlorid-Lösung und trocknet über Natriumsulfat. Nach Einengen der organischen Phase im Vakuum und Reinigung des Rückstandes durch Säulenchromatographie über Kieselgel (Kieselgel 60, Merck, Korngröße: 0.04 bis 0.063 mm) mit dem Laufmittelgemisch Essigsäureethylester : Cyclohexan (2:1) erhält man 430 mg (72 % der Theorie) 3-Chlor-4-[(6-chlorpyridin-3-yl)methyl]-5,6,7,7a-tetrahydrofuro[3,2-b]pyridin-2(4H)-on.
¹H-NMR (CD₃CN): δ [ppm] = 1.53 (m, 1 H), 1.83 (m, 2 H), 2.37 (m, 1 H), 3.13 (m, 1 H), 3.25 (m, 1 H), 4.79 (d, 1 H), 4,83 (dd, 1 H), 4.96 (d, 1 H), 7.43 (d, 1 H), 7.71 (dd, 1 H), 8.35 (d, 1 H).

### C-Alkylierung (R³ = Alkyl)

### Beispiel I-10 4-[(6-Chlorpyridin-3-yl)methyl]-7a-methyl-5,6,7,7a-tetrahydrofuro [3,2-b]pyridin-2(4H)-on

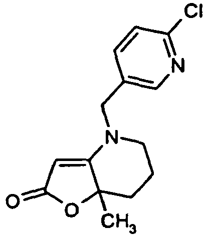

43 mg (0.16 mmol) 4-[(6-Chlorpyridin-3-yl)methyl]-5,6,7,7a-tetrahydrofuro[3,2-b]pyridin-2(4H)-on (1-2) werden in 2.5 mL Tetrahydrofuran gelöst, auf -78°C abgekühlt und mit 96 µL (0.16 mmol) einer 1.7 M Lösung von *tert*.-Butyllithium in Pentan versetzt. Nach 30 Minuten Rühren bei -78°C gibt man 15 µL (0.24 mmol) Methyliodid hinzu, rührt weitere 30 min bei -78°C, wärmt auf Raumtemperatur auf und rührt weitere 3 Stunden bei Raumtemperatur. Einengen im Vakuum und Reinigung des Rückstandes durch Säulenchromatographie über Kieselgel (Kieselgel 60, Merck, Korngröße: 0.04 bis 0.063 mm) mit dem Laufmittel Essigsäureethylester liefern 16 mg (35 % der Theorie) 4-[(6-Chlorpyridin-3-yl)methyl]-7a-methyl-5,6,7,7a-tetrahydrofuro[3,2-b]pyridin-2(4H)-on.
¹H-NMR (CDCl₃): δ [ppm] = 1.57 (s, 3 H), 1.81 (m, 1 H), 1.95 (m, 2 H), 2.18 (m, 1 H), 2.92 (m, 1 H), 3.28 (m, 1 H), 4.05 (d, 1 H), 4.32 (d, 1 H), 4.76 (s, 1 H), 7.36 (d, 1 H), 7.57 (dd, 1 H), 8.30 (d, 1 H).

### Formylierung (R² = CHO)

### Beispiel I-11 3-Formyl-4-[(6-chlorpyridin-3-yl)methyl]-5,6,7,7a-tetrahydrofuro 13,2-blpyridin-2(4H)-on

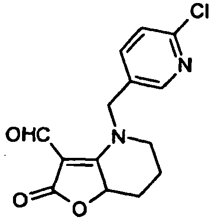

1,89 mL (24.56 mmol) *N,N*-Dimethylformamid und 0.28 mL (3.02 mmol) Phosphorylchlorid wird unter Eiskühlung vorgelegt und nach einer Stunde mit 500 mg (1.89 mmol) 4-[(6-Chlorpyridin-3-yl)methyl]-5,6,7,7a-tetrahydrofuro[3,2-b]pyridin-2(4H)-on (1-2) versetzt. Danach wird zwei Stunden bei Raumtemperatur gerührt. Anschließend stellt man das Reaktionsgemisch mit Natriumcarbonat alkalisch, filtriert den ausgefallenen Feststoff ab und wäscht diesen mit Wasser. Man erhält 0.33 g (56 % der Theorie) 3-Formyl-4-[(6-chlorpyridin-3-yl)methyl]-5,6,7,7a-tetrahydrofuro[3,2-b] pyridin-2(4H)-on.
¹H-NMR (CD₃CN): δ [ppm] = 1.45 (m, 1H), 1.7 (m, 1 H), 1.82 (m, 1 H), 2.42 (m, 1 H), 3.32 (m, 2 H), 4.78 (dd, 1H), 5.45 (m, 2 H), 7.38 (d, 1H), 7.65 (dd, 1 H), 8.30 (d, 1H), 9.51 (s, 1 H).

### Beispiel I-12 4-(6-Chlorpyridin-3-yl)methyl]-6-methyl-5,6,7,7a-tetrahydrofuro [3,2-b]pyridin-2(4H)-on

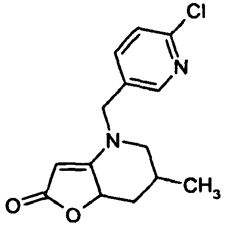

150.0 mg (0.54 mmol) 4-[(6-Chlorpyridin-3-yl)methyl]-6-methylen-5,6,7,7a-tetrahydrofuro[3,2-b]pyridin-2(4H)-on (vgl. 1-5) werden in 75 ml Toluol gelöst, mit 40 mg (0.043 mmol) Tris(triphenylphosphin)rhodium(I)chlorid und 15 mg (0.057 mmol) Triphenylphosphin versetzt und 10 Stunden unter 3 bar Wasserstoff bei 110-120°C hydriert. Man filtriert und engt das Filtrat im Vakuum ein. Reinigung des Rückstandes durch Säulenchromatographie über Kieselgel (Kieselgel 60, Merck, Korngröße: 0.04 bis 0.063 mm) mit dem Laufmittel Essigsäureethylester : Cyclohexan (2:1 bis 4:1) liefert beide möglichen Diastereomere von 4-[(6-Chlorpyridin-3-yl)methyl]-6-methyl-5,6,7,7a-tetrahydrofuro[3,2-b]pyridin-2(4H)-on: (a) 85 mg (56 % der Theorie) Diastereomer A und (b) nach präparative HPLC 18 mg (12 % der Theorie) Diastereomer B.

### Beispiel I-12a (Diastereomer A)

¹H-NMR (CD₃CN): δ [ppm] = 0.96 (d, 3 H), 2.13 (m), 2.31 (m, 1 H), 2.68 (dd, 1 H), 3.28 (dd, 1 H), 4.19 (d, 1 H), 4.39 (d, 1 H), 4.70 (s, 1 H), 4.77 (dd, 1 H), 7.39 (d, 1 H), 7.70 (dd, 1 H), 8.32 (d, 1 H).

### Beispiel I-12b (Diastereomer B)

¹H-NMR (CD₃CN): δ [ppm] = 0.97 (d, 3 H), 1.85-2.20 (m), 2.31 (m, 1H), 2.99 (dd, 1 H), 3.17 (dd, 1 H), 4.39 (d, 1 H), 4.46 (d, 1 H), 4.62 (s, 1 H), 4.88 (dd, 1 H), 7.39 (d, 1 H), 7.68 (dd, 1 H), 8.31 (d, 1 H).

### Beispiel I-14 4-[(6-Chlorpyridin-3-yl)methyl]-6-oxo-6-hydroxymethyl-5,6,7,7a-tetrahydrofuro[3,2-b]pyridin-2(4H)-on

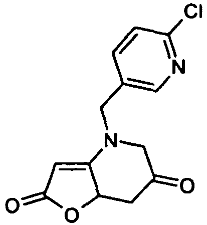

80 mg (0.26 mmol) 4-[(6-Chlorpyridin-3-yl)methyl]-6-hydroxy-6-hydroxymethylen-5,6,7,7a-tetra-hydrofuro[3,2-b]pyridin-2(4H)-on (vgl. I-13) werden in 2 mL eines Gemisches aus Dichlormethan : Wasser (2:1) gelöst und bei 0°C mit 275 mg Natriumperiodat versetzt. Man rührt 2 Stunden bei 0°C, verdünnt mit Wasser und extrahiert viermal mit Dichlormethan. Die vereinigten organischen Phasen werden über Natriumsulfat getrocknet und im Vakuum eingeengt. Die Reinigung des Rückstandes mittels Säulenchromatographie über Kieselgel (Kieselgel 60, Merck, Korngröße: 0.04 bis 0.063 mm) mit dem Laufmittel Essigsäureethylester liefern 65 mg (72 % der Theorie) 4-[(6-Chlorpyridin-3-yl)methyl]-6-oxo-6-hydroxymethyl-5,6,7,7a-tetrahydrofuro[3,2-b]pyridin-2(4H)-on.
¹H-NMR (CD₃CN): δ [ppm] = 2.50 (dd, 1 H), 3.07 (dd, 1 H), 3.67 (d, 1 H), 3.98 (d, 1 H), 4.42 (d, 1 H), 4.54 (d, 1 H), 4.79 (s, 1 H), 5.21 (dd, 1 H), 7.38 (d, 1 H), 7.68 (dd, 1 H), 8.32 (d, 1 H).

### Beispiel I-15 4-[(6-Chlorpyridin-3-yl)methyl]-6,6-difluor-5,6,7,7a-tetrahydrofuro [3,2-b]pyridin-2(4H)-on

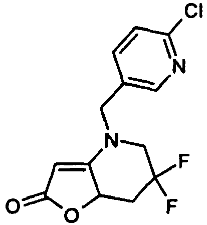

13 mg (0.047 mmol) 4-[(6-Chlorpyridin-3-yl)methyl]-6-oxo-5,6,7,7a-tetrahydrofuro[3,2-b]pyridin-2(4H)-on (vgl. I-14) werden in 1 mL Dichlormethan gelöst und in zwei Portionen innerhalb von 30 Minuten mit 31 µL (0.23 mmol) Diethylaminoschwefeltrifluorid versetzt. Man rührt eine Stunde bei Raumtemperatur, versetzt mit gesättigter wässriger Natriumhydrogencarbonat-Lösung und extrahiert die wässrige Phase mit Dichlormethan. Die vereinigten organischen Phasen werden über Natriumsulfat getrocknet und im Vakuum eingeengt. Die Reinigung des Rückstandes mittels Säulenchromatographie über Kieselgel (Kieselgel 60, Merck, Korngröße: 0.04 bis 0.063 mm) mit dem Laufmittel Essigsäureethylester liefert 8 mg (55 % der Theorie) 4-[(6-Chlorpyridin-3-yl)methyl]-6,6-difluor-5,6,7,7a-tetrahydrofuro[3,2-b]pyridin-2(4H)-on.
¹H-NMR (CDCl₃): δ [ppm] = 2.13 (dd, 1 H), 3.03 (m, 1 H), 3.38 (m, 1 H), 3.53 (m, 1 H), 4.36 (d, 1 H), 4.47 (d, 1 H), 4.89 (s, 1 H), 4.98 (dd, 1 H), 7.39 (d, 1 H), 7.57 (dd, 1 H), 8.32 (d, 1 H).

### Beispiel I-16 4-[(6-Chlorpyridin-3-yl)methyl]-6-hydroxy-5,6,7,7a-tetrahydrofuro [3,2-b]pyridin-2(4H)-on

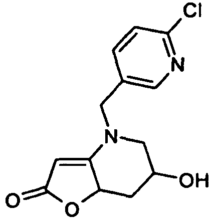

560 mg (1.61 mmol) 4-[(6-Chlorpyridin-3-yl)methyl]-6-oxo-5,6,7,7a-tetrahydrofuro[3,2-b]pyridin-2(4H)-on (Bsp. I-14) werden in 14 mL Methanol gelöst und bei 0°C mit 45 mg (1.20 mmol) Natriumborhydrid versetzt. Man rührt das Reaktionsgemisch eine Stunde bei 0°C. Nach Zugabe von 7.2 mL 5%-iger Chlorwasserstoffsäure extrahiert man mit Essigsäureethylester. Die organische Phase wird über Natriumsulfat getrocknet und im Vakuum eingeengt. Die Reinigung des Rückstandes mittels Säulenchromatographie über Kieselgel (Kieselgel 60, Merck, Korngröße: 0.04 bis 0.063 mm) mit dem Laufmittelgemisch Dichlormethan : Methanol (95:5) liefert beide möglichen Diastereomere von 4-[(6-Chlorpyridin-3-yl)methyl]-6-hydroxy -5,6,7,7a-tetrahydrofuro[3,2-b]pyridin-2(4H)-on: (a) 152 mg (34 % der Theorie) Diastereomer A und (b) 63 mg (11 % der Theorie) Diastereomer B.

### Beispiel I-16a (Diastereomer A)

¹H-NMR (CD₃CN): δ [ppm] = 1.73 (m, 1 H), 2.32 (m, 1 H), 3.18 (dd, 1 H), 3.30 (m, 2 H), 4.18 (m, 1 H), 4.29 (d, 1 H), 4.42 (d, 1 H), 4.68 (s, 1 H), 5.06 (dd, 1 H), 7.40 (d, 1 H), 7.69 (dd, 1 H), 8.31 (d, 1 H).

### Beispiel I-16b (Diastereomer B)

¹H-NMR (CD₃CN): δ [ppm] = 1.33 (m, 1 H), 2.75 (m, 1 H), 3.08 (dd, 1 H), 3.18 (d, 1 H), 3.38 (dd, 1 H), 4.10 (m, 1 H), 4.37 (d, 1 H), 4.50 (d, 1 H), 4.62 (s, 1 H), 4.74 (dd, 1 H), 7.39 (d, 1 H), 7.74 (dd, 1H), 8.34 (d, 1 H).

### Beispiel I-17 4-[(6-Chlorpyridin-3-yl)methyl]-6-chlor-5,6,7,7a-tetrahydroluro [3,2-b]pyridin-2(4H)-on und

### Beispiel I-18 4-[(6-Chlorpyridin-3-yl)methyl]-7,7a-dihydrofuro[3,2-b]pyridin-2(4H)-on

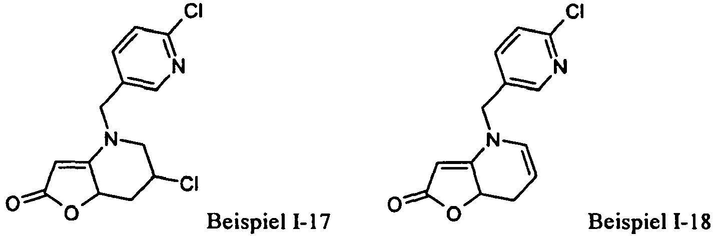

146.0 mg (0.52 mmol) 4-[(6-Chlorpyridin-3-yl)methyl]-6-hydroxy-5,6,7,7a-tetrahydrofuro[3,2-b]pyridin-2(4H)-on (vgl. 1-16) und 274.2 mg (1.05 mmol) Triphenylphosphin werden in einem Gemisch aus 2.5 mL Acetonitril und 2.5 mL Tetrachlormethan 30 min bei 70°C gerührt. Nach Abkühlen auf Raumtemperatur verdünnt man mit Dichlormethan und wäscht nacheinander mit verdünnter wässriger Ammoniumhydroxid-Lösung und gesättigter Natriumchlorid-Lösung: Die organische Phase wird über Natriumsulfat getrocknet und im Vakuum eingeengt. Die Reinigung des Rückstandes mittels Säulenchromatographie über Kieselgel (Kieselgel 60, Merck, Korngröße: 0.04 bis 0.063 mm) mit dem Laufmittel Essigsäureethylester und anschließende präparative HPLC liefert beide möglichen Diastereomere von 4-[(6-Chlorpyridin-3-yl)methyl]-6-chlor-5,6,7,7a-tetra-hydrofuro[3,2-b]pyridin-2(4H)-on: (a) 4 mg Diastereomer A (3 % der Theorie) und (b) 15 mg Diastereomer B (9 % der Theorie). Daneben erhält man (c) 10 mg (7 % der Theorie) 4-[(6-Chlorpyridin-3-yl)methyl]-7,7a-dihydrofuro[3,2-b]pyridin-2(4H)-on.

### Beispiel I-17a (Diastereomer A)

¹H-NMR (CD₃CN): δ [ppm] = 2.10 (m, 1 H), 2.62 (m, 1 H), 3.45 (dd, 1 H), 3.57 (dd, 1 H), 4.30 (d, 1H), 4.45 (d, 1H), 4.54 (m, 1 H), 4.78 (s, 1 H), 5.17 (dd, 1 H), 7.40 (d, 1 H), 7.70 (dd, 1H), 8.33 (d, 1H).

### Beispiel I-17b (Diastereomer B)

¹H-NMR (CD₃CN): δ [ppm] = 1.78 (m, 1 H), 3.03 (m, 1 H), 3.32 (dd, 1 H), 3.69 (dd, 1 H), 4.40 (d, 1H), 4.47 (m, 1H), 4.54 (d, 1 H), 4.76 (s, 1 H), 4.82 (dd, 1 H), 7.40 (d, 1 H), 7.73 (dd, 1H), 8.35 (d, 1H).

### Beispiel I-18

¹H-NMR (CD₃CN): δ [ppm] = 2.21 (m, 1 H), 2.61 (m, 1 H), 4.55 (d, 1 H), 4.63 (d, 1 H), 4.85 (s, 1 H), 4.97 (dd, 1 H), 5.10 (m, 1 H), 6.16 (dd, 1 H), 7.39 (d, 1 H), 7.67 (dd, 1 H), 8.30 (d, 1 H).

### Beispiel I-19 4-[(6-Chlorpyridin-3-yl)methyl]-7-hydroxy-5,6,7,7a-tetrahydrofuro [3,2-b]pyridin-2(4H)-on und

### Beispiel I-20 4-[(6-Chlorpyridin-3-yl)methyl]-5,6-dihydrofuro[3,2-b]pyridin-2(4H)-on

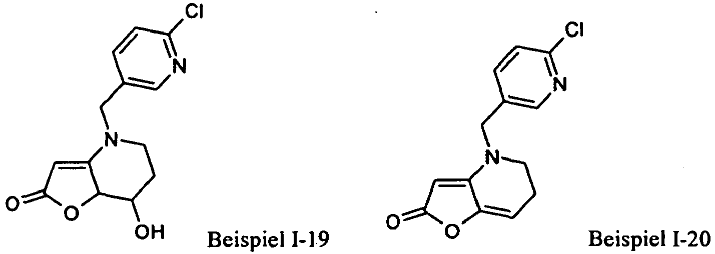

176 mg (0.35 mmol) *tert*-Butyl-[(6-chlorpyridin-3-yl)methyl][3-hydroxy-3-(5-oxo-3-pyrrolidin-1-yl-2,5-dihydrofuran-2-yl)propyl]carbamat (vgl. V-2) werden eine Stunde in einem Gemisch von 7 mL Dichlormethan und 3.5 mL Trifluoressigsäure bei Raumtemperatur gerührt. Nach Einengen im Vakuum gibt man zu dem Rückstand 10.5 mL Essigsäure und rührt 2 Stunden bei 100°C. Nach Einengen im Vakuum wird der Rückstand durch Säulenchromatographie über Kieselgel (Kieselgel 60, Merck, Korngröße: 0.04 bis 0.063 mm) mit dem Laufmittelgemisch Dichlormethan : Methanol (99:1 bis 80:20) gereinigt. Man erhält (a) 6 mg (6 % der Theorie) 4-[(6-Chlorpyridin-3-yl)methyl]-7-hydroxy-5,6,7,7a-tetrahydrofuro[3,2-b]pyridin-2(4H)-on als Diastereomerengemisch und (b) 30 mg (32 % der Theorie) 4-[(6-Chlorpyridin-3-yl)methyl]-5,6-dihydrofuro[3,2-b]pyridin-2(4H)-on.

### Beispiel 1-19 (Diastereomerengemisch)

LC-MS: *m*/*z* = 281.0 [M+H]⁺ (100 %).

### Beispiel I-20

¹H-NMR (CD₃CN): δ [ppm] = 2.48 (m, 2 H), 3.27 (t, 2 H), 4.41 (s, 2 H), 4.81 (s, 1 H), 5.63 (m, 1 H), 7.40 (d, 1 H), 7.72 (dd, 1 H), 8.35 (d, 1 H).

Analog zu diesem Verfahren wurden auch die in der nachstehenden Tabelle 1 aufgeführten Verbindungen (I-22) bis (I-24) und (I-27a) und (I-27b) hergestellt.

**Tabelle 1**

| | | | | | |
|---|---|---|---|---|---|
| 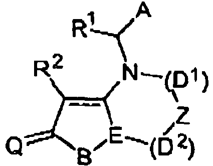 | | | | | Q-O/R¹=H |

| **Bsp. Nr.** | **B** | **R²** | **(D²)-Z-(D¹)** | **A** | **Physikalische Daten: ¹H-NMR, δ [ppm]** |
|---|---|---|---|---|---|
| I-22 | CH ₂ | H | 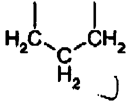 | 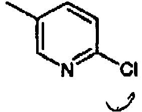 | CDCl₃, δ = 1.40 (m, 1H), 1.80-2.50 (m, 3H), 2.18 (dd, 1H), 2.60 (dd, 1H), 2.88 (m, 1H), 3.14 (m, 1H), 3.36 (m, 1H), 4.28 (d, 1H), 4.41 (d, 1H), 5.12 (s, 1H), 7.33 (d, 1H), 7.57 (dd, 1H), 8.28 (d, 1H) |
| I-23 | O | F | 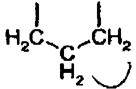 | 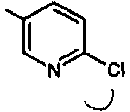 | CDCl₃, δ = 1.54 (m, 1H), 1.87 (m, 1H), 2.00 (m, 1H), 2.42 (m, 1H), 2.95 (m, 1H), 3.12 (m, 1H), 4.32 (d, 1H), 4,63 (m, 1H), 4.65 (d, 1H), 7.3 8 (d, 1H, 7.63 (dd, 1H), 8.34 (d, 1H) |
| I-24 | O | H | 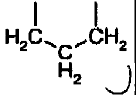 | 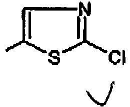 | CDCl₃, δ = 1.54 (m, 1H), 1.90 (m, 1H), 2.00 (m, 2H), 2.42 (m, 1H), 3.00 (m, 1H), 3.25 (m, 1H), 4.31 (d, 1H), 4.42 (d, 1H), 4.69 (dd, 1H), 4.83 (s, 1H), 7.46 (s, 1H) |
| I-27a | O | H | 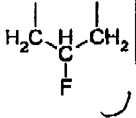 | 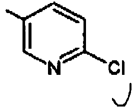 | CD₃CN, δ = 1.60 (dm, 1H), 2.95 (m, 1H), 3.46 (m, 2H), 4.44 (d, 1H), 4.53 (d, 1H), 4.69 (s, 1H), 4.78 (dd, 1H), 5.04 (dm, 1H), 7.39 (d, 1H), 7.71 (dd, 1H), 8.33 (d, 1H) |
| I-27b | O | H | 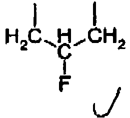 | 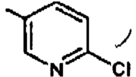 | CD₃CN, δ = 1.82 (ddd, 1H), 2.66 (m, 1H), 3.45 (m, 2H), 4.28 (d, 1H), 4.43 (d, 1H), 4.76 (s, 1H), 5.02 (dd, 1H), 5.14 (dm, 1H), 7.41 (d, 1H), 7.69 (dd, 1H), 8.32 (d, 1H) |

### Herstellung der Ausgangsverbindungen

### Verbindungen der allgemeinen Formel (II)

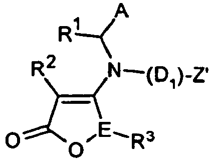

### Beispiel II-1 4-[[(6-Chlorpyridin-3-yl)methyl](2-hydroxyethyl)amino]furan-2(5H)-on (R¹ = H, -(D¹)-Z' = CH₂CH₂-OH; R² = H; E-R³ = CH₂, A = 6-Chlor-pyrid-3-yl)

3.00 g (16.07 mmol) 2-{[(6-Chloropyridin-3-yl)methyl]amino}ethanol (vgl. WO 2005055715 A2) in 150 mL Toluol werden mit 2.41 g (24.11 mmol) Tetronsäure und 28 mg (0.16 mmol) 4-Toluolsulfonsäure versetzt und am Wasserabscheider 3 Stunden unter Rückfluss erhitzt. Nach Abkühlen der Reaktionsmischung wird die flüssige Phase abdekantiert und der feste Rückstand mittels Säulenchromatographie über Kieselgel (Kieselgel 60, Merck, Korngröße: 0.04 bis 0.063 mm) mit dem Laufmittelgemisch Dichlormethan : Methanol (95:5) gereinigt. Man erhält 722 mg (16 % der Theorie) 4-[[(6-Chlorpyridin-3-yl)methyl](2-hydroxyethyl)amino]furan-2(5H)-on.

¹H-NMR (CD₃CN): δ [ppm] = 2.88 (t, 1H), 3.28 (t, 2 H), 3.66 (q, 2 H), 4.50 (s, 2 H), 4.58 (s, 1H), 4.82 (s, 2 H), 7.37 (d, 1H), 7.65 (dd, 1 H), 8.28 (d, 1H).

### Beispiel II-2 4-[(3-Chlorpropyl)[(6-chlorpyridin-3-yl)methyl]amino]furan-2(5H)-on (R' = H, -(D¹)-Z' = CH₂CH₂CH₂-Cl; R² = H; E-R³ = CH₂, A = 6-Chlor-pyrid-3-yl)

16.30 g (74.4 mmol) 3-Chlor-*N*-[(6-chlorpyridin-3-yl)methyl]propan-1-amin (IVa-1 vgl. auch B. Latli et al. J. Med. Chem. 1999, 42, 2227-2234) in 300 mL Benzol werden mit 4.26 mL (74.4 mmol) Essigsäure versetzt und 30 Minuten bei Raumtemperatur gerührt. Anschließend gibt man 9.68 g (96.7 mmol) Tetronsäure und 128 mg 4-Toluolsulfonsäure zu und erhitzt am Wasserabscheider 2 Stunden unter Rückfluss. Nach Einengen der Reaktionsmischung im Vakuum nimmt man mit dem Lösungsmittelgemisch Dichlormethan : Methanol (95:5) auf, wäscht nacheinander mit 1N wässriger Salzsäure, 1 N wässriger Natriumhydroxid-Lösung und gesättigter Natriumchlorid-Lösung und trocknet über Natriumsulfat. Nach Einengen der organischen Phase im Vakuum und Reinigung des Rückstandes mittels Säulenchromatographie über Kieselgel (Kieselgel 60, Merck, Korngröße: 0.04 bis 0.063 mm) mit dem Laufmittelgemisch Essigsäureethylester erhält man 9.15g (33 % der Theorie) 4-[(3-Chlorpropyl)[(6-chlorpyridin-3-yl)methyl]amino]furan-2(5H)-on.
¹H-NMR (CDCl₃): δ [ppm] = 2.07 (m, 2 H), 3.38 (t, 3 H), 3.58 (t, 2 H), 4.40 (s, 2 H), 4.81 (s, 1 H), 4.82 (s, 2 H), 7.38 (d, 1H), 7.52 (dd, 1H), 8.28 (d, 1 H).

### Beispiel II-3 4-[[(6-Chlorpyridin-3-yl)methyl](3-iodpropyl)amino]furan-2(5H)-on (R¹ = H, -(D¹)-Z' = CH₂CH₂CH₂-I; R² = H; E-R³ = CH₂, A = 6-Chlor-pyrid-3-yl)

27.50 g (91.3 mmol) 4-[(3-Chlorpropyl)[(6-chlorpyridin-3-yl)methyl]amino]furan-2(5H)-on (1-2) und 51.60 g (344.2 mmol) Natriumiodid werden in 1.2 L Acetonitril 4 Stunden unter Rückflusstemperatur erhitzt. Nach Einengen der organischen Phase im Vakuum und Reinigung des Rückstandes mittels Säulenchromatographie über Kieselgel (Kieselgel 60, Merck, Korngröße: 0.04 bis 0.063 mm) mit dem Laufmittel Essigsäureethylester erhält man 31.5 g 4-[[(6-Chlorpyridin-3-yl)methyl](3-iodpropyl)amino]furan-2(5H)-on.
¹H-NMR (CDCl₃): δ [ppm] = 2.10 (m, 2 H), 3.18 (t, 3 H), 3.31 (t, 2 H), 4.41 (s, 2 H), 4.81 (s, 1 H), 4.83 (s, 2 H), 7.38 (d, 1H), 7.53 (dd, 1H), 8.28 (d, 1H).

### Verbindungen der allgemeine Formel HN(R')-CH₂-A (IVa)

### Beispiel IVa-1 3-Chlor-N-[(6-chlorpyridin-3-yl)methyl]propan-1-amin (R' = CH₂CH₂CH₂-Cl, A = 6-Chlor-pyrid-3-yl) (vgl. B. Latli et al. J. Med. Chem. 1999, 42, 2227-2234)

16.20 g (100 mmol) 2-Chlor-5-(chlormethyl)pyridin, 16.90 g (130 mmol) 3-Chloropropan-1-amin Hydrochlorid und 36.24 mL (260 mmol) Triethylamin werden in 200 mL Acetonitril 20 Stunden bei 60°C gerührt. Nach Zugabe von 30.67 g (230 mmol) 30-prozentiger wässriger Natriumhydroxid-Lösung engt man die Reaktionsmischung im Vakuum ein und extrahiert den Rückstand mit Chloroform. Einengen des Extraktes im Vakuum liefert 19.03 g (87 % der Theorie) 3-Chlor-N-[(6-chlorpyridin-3-yl)methyl]propan-1-amin, das ohne weitere Reinigung in der Folgereaktion (vgl. Bsp. II-2) eingesetzt werden kann.
¹H-NMR (CD₃CN): δ [ppm] = 1.85-2.05 (m, 2 H), 2.69 (t, 2 H), 3.65 (t, 2 H), 3.75 (s, 2 H), 7.32 (d, 1 H), 7.71 (dd, 1 H), 8.30 (d, 1H).

### Beispiel IVa-2 3-{[(6-Chlorpyridin-3-yl)methyl]amino}propan-1-ol (R' = CH₂CH₂CH₂-OH, A = 6-Chlor-pyrid-3-yl) (vgl. EP 192060 A1)

14.16 g (100 mmol) 6-Chlornicotinaldehyd und 8.41 mL (110 mmol) 3-Aminopropanol werden in 100 mL Ethanol 30 Minuten bei Raumtemperatur gerührt. Man versetzt portionsweise mit 3.78 g (100 mmol) Natriumborhydrid und rührt ca. 16 Stunden bei Raumtemperatur. Nach Einengen im Vakuum gibt man Wasser und Kaliumcarbonat hinzu und extrahiert mit Methyl-*tert*.-butylether. Die vereinigte organische Phase wird über Natriumsulfat getrocknet und im Vakuum eingeengt. Säulenchromatographie des Rückstandes über Kieselgel (Kieselgel 60, Merck, Korngröße: 0.04 bis 0.063 mm) mit dem Laufmittelgemisch Dichlormethan : Methanol (95:5) liefert 3.86 g (19 % der Theorie) 3-{[(6-Chlorpyridin-3-yl)methyl]amino}propan-1-ol.
¹H-NMR (CD₃CN): δ [ppm] = 1.65 (m, 2 H), 2.68 (t, 2 H), 3.59 (t, 2 H), 3.74 (s, 2 H), 7.32 (d, 1 H), 7.70 (dd, 1 H), 8.30 (d, 1 H).

### Verbindungen der allgemeinen Formel (V)

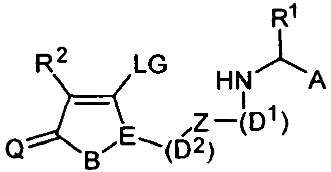

### Beispiel V-1 5-[2-({[(6-Chlorpyridin-3-yl)methyl]amino}methyl)prop-2-en-1-yl]-4-pyrrolidin-1-ylfuran-2(5H)-on (R¹, R² = H; B, Q = O; E-(D²)-Z-(D¹)= CHCH₂C(=CH₂)CH₂-; LG = N-pyrrolidino; A = 6-Chlor-pyrid-3-yl)

600 mg (2.48 mmol) 5-[2-(Chlormethyl)prop-2-en-1-yl]-4-pyrrolidin-1-ylfuran-2(5H)-on (V-1a), 354 mg (2.48 mmol) 1-(6-Chlorpyridin-3-yl)methanamin und 0.43 mL (2.48 mmol) *N-*Ethyl-*N* isopropylpropan-2-amin werden in 10 mL Acetonitril 21 Stunden bei 60°C gerührt. Einengen im Vakuum und Reinigung des Rückstandes durch Säulenchromatographie über Kieselgel (Kieselgel 60, Merck, Korngröße: 0.04 bis 0.063 mm) mit dem Laufmittelgemisch Dichlormethan : Methanol (98:2 bis 90:10) liefern 650mg (73 % der Theorie) 5-[2-({[(6-Chlorpyridin-3-yl)methyl]amino} methyl)prop-2-en-1-yl]-4-pyrrolidin-1-ylfuran-2(5H)-on.
¹H-NMR (CD₃CN): δ [ppm] = 1.85 (m, 2 H), 1.96 (m, 2 H), 2.26 (dd, 1H), 2.80 (dd, 1H), 3.15 (m, 2 H), 3.18 (d, 1H), 3.24 (d, 1 H), 3.39 (m, 2 H), 3.70 (s, 2 H), 4.37 (s, 1 H), 4.97 (s, 1 H), 5.05 (dd, 1 H), 5.09 (s, 1 H), 7.34 (d, 1 H), 7.72 (dd, 1 H), 8.30 (d, 1 H).

### V-1a 5-[2-(Chlormethyl)prop-2-en-1-yl]-4-pyrrolidin-1-ylfuran-2(5H)-on

800 mg (5.22 mmol) 4-Pyrrolidin-1-ylfuran-2(5H)-on (Shandala, M. Y. et al. J. Heterocycl. Chem. 1984, 21, 1753-1754) werden in 80 mL Tetrahydrofuran gelöst, auf -78°C abgekühlt und mit 3.07 mL (5.22 mmol) einer 1.7 M Lösung von *tert*.-Butyllithium in Pentan versetzt. Nach 30 min Rühren bei -78°C gibt man 1.21 mL (10.45 mmol) 3-Chlor-2-(chlormethyl)prop-1-en hinzu, rührt weitere 30 Minuten bei -78°C, wärmt innerhalb von ca. 16 Stunden auf Raumtemperatur auf und rührt weitere 3 Stunden bei Raumtemperatur. Nach Zugabe von Methanol und Einengen im Vakuum wird der Rückstand durch Säulenchromatographie über Kieselgel (Kieselgel 60, Merck, Korngröße: 0.04 bis 0.063 mm) mit dem Laufmittel Essigsäureethylester gereinigt. Man erhält 735 mg (57 % der Theorie) 5-[2-(Chlormethyl)prop-2-en-1-yl]-4-pyrrolidin-1-ylfuran-2(5H)-on.
¹H-NMR (CD₃CN): δ [ppm] = 1.85-2.05 (m, 4 H), 2.42 (dd, 1H), 2.91 (dd, 1H), 3.32 (m, 4 H), 4.15 (d, 1 H), 4.20 (d, 1 H), 4.40 (s, 1 H), 5.05 (dd, 1 H), 5.14 (s, 1 H), 5.31 (s, 1 H).

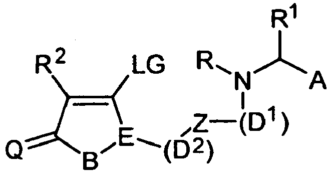

### Beispiel V-2 tert-Butyl-[(6-chlorpyridin-3-yl)methyl][3-hydroxy-3-(5-oxo-3-pyrrolidin-1-yl-2,5-dihydrofuran-2-yl)propyl]carbamat (R¹, R² = H; B, Q = O; E-(D²)-Z-(D¹)-NR = CHCH(OH)CH₂CH₂-N-(tert-butoxycarbonyl)-(BOC); LG = N-pyrrolidino; A = 6-Chlor-pyrid-3-yl)

1.23 g (8.06 mmol) 4-Pyrrolidin-1-ylfuran-2(5H)-on (Shandala, M. Y. et al. J. Heterocycl. Chem. (1984), 21, 1753-1754) werden in 30 mL Tetrahydrofuran gelöst, auf -78°C abgekühlt und mit 6.17 mL (10.48 mmol) einer 1.7 M Lösung von *tert*.-Butyllithium in Pentan versetzt. Nach 30 min Rühren bei -78°C gibt man eine Lösung von 2.65 g (8.87 mmol) *tert*-Butyl-[(6-chlorpyridin-3-yl)methyl](3-oxopropyl)carbamat in 10 mL Tetrahydrofuran hinzu und rührt 1 Stunde bei -78°C. Nach Zugabe von Methanol und Einengen im Vakuum wird der Rückstand durch Säulenchromatographie über Kieselgel (Kieselgel 60, Merck, Korngröße: 0.04 bis 0.063 mm) mit dem Laufmittelgemisch Dichlormethan : Methanol (95:5) gereinigt. Man erhält 2.67 g (57 % der Theorie) *tert*-Butyl-[(6-chlorpyridin-3-yl)methyl][3-hydroxy-3-(5-oxo-3-pyrrolidin-1-yl-2,5-dihydrofuran-2-yl)propyl]carbamat als Diastereomerengemisch, das ohne weitere Reinigung in der Folgereaktion (vgl. Bsp. I-19 und 1-20) eingesetzt werden kann.
LC-MS: *m*/*z* = 451.9 [M+H]⁺ (100 %).

### V-2a tert-Butyl-[(6-chlorpyridin-3-yl)methyl](3-hydroxypropyl)carbamat:

2.00 g (9.97 mmol) 3-{[(6-Chlorpyridin-3-yl)methyl]amino}propan-1-ol (vgl. EP 192060 A1 und Bsp. IVa-2) werden in 24 mL Tetrahydrofuran gelöst und nacheinander mit 10.72 mL (10.72 mmol) 1 N wässrige Natriumhydroxid-Lösung und 2.18 g (9.97 mmol) Di-*tert*.-butyldicarbonat versetzt. Man rührt 1 Stunde bei Raumtemperatur und entfernt das Tetrahydrofuran im Vakuum weitgehend. Die zurückbleibende wässrige Phase wird mit Natriumhydrogensulfat angesäuert (> pH 2) und mit Essigsäureethylester extrahiert. Die vereinigte organische Phase wird über Natriumsulfat getrocknet und im Vakuum eingeengt. Man erhält 3.00 g *tert*-Butyl-[(6-chlorpyridin-3-yl)methyl](3-hydroxypropyl)carbamat (100 % der Theorie), das ohne weitere Reinigung in der Folgereaktion eingesetzt werden kann.

### V-2b tert-Butyl-[(6-chlorpyridin-3-yl)methyl](3-oxopropyl)carbamat:

3.00 g (9.97 mmol) *tert*-Butyl-[(6-chlorpyridin-3-yl)methyl](3-hydroxypropyl)carbamat werden in 50 mL Dichlormethan gelöst und mit 56.38 g (19.94 mmol) einer 15-prozentigen Lösung von Dess-Martin-Periodinan in Dichlormethan versetzt und 1 Stunde bei Raumtemperatur gerührt. Man wäscht die Reaktionsmischung mit 1 N wässriger Natriumhydroxid-Lösung und extrahiert die wässrige Phase mit Dichlormethan. Die vereinigten organischen Phasen werden über Natriumsulfat getrocknet und im Vakuum eingeengt. Nach Reinigung des Rückstandes durch Säulenchromatographie über Kieselgel (Kieselgel 60, Merck, Korngröße: 0.04 bis 0.063 mm) mit dem Laufmittelgemisch Essigsäureethylester : Cyclohexan (1:1) erhält man 2.75 g (92 % der Theorie *tert*-Butyl-[(6-chlorpyridin-3-yl)methyl](3-oxopropyl)carbamat.
¹H-NMR (CD₃CN): δ [ppm] = 1.41 (s, 9 H), 2.62 (t, 2 H), 3.50 (t, 2 H), 4.40 (s, 2 H), 7.34 (d, 1 H), 7.63 (dd, 1 H), 8.26 (d, 1 H), 9.67 (s, 1 H).

### Verbindungen der allgemeinen Formel (VI)

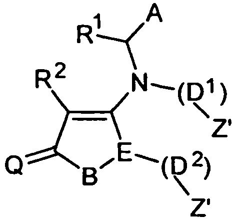

### Beispiel VI-1 5-Allyl-4-{allyl[(6-chlorpyridin-3-yl)methyl]amino}furan-2(5H)-on (R¹ = H, -(D¹)-Z' = CH₂CH=CH₂; R² = H; E-(D²)Z' = CH-CH₂CH=CH₂, A = 6-Chlor-pyrid-3-yl)

500 mg (1.89 mmol) 4-{Allyl[(6-chlorpyridin-3-yl)methyl]amino}furan-2(5H)-on (vgl. WO 9200964 A1) werden in 30 mL Tetrahydrofuran gelöst, auf -78°C abgekühlt und mit 1.11 mL (1.89 mmol) einer 1.7 M Lösung von *tert*.-Butyllithium in Pentan versetzt. Nach 30 Minuten Rühren bei -78°C gibt man 163 µL (1.89 mmol) Allylbromid hinzu, rührt weitere 30 min bei -78°C, wärmt auf Raumtemperatur auf und rührt weitere 2 Stunden bei Raumtemperatur. Nach Zugabe von Methanol engt man im Vakuum ein. Der Rückstand wird mittels Säulenchromatographie über Kieselgel (Kieselgel 60, Merck, Korngröße: 0.04 bis 0.063 mm) mit dem Laufmittelgemisch Essigsäureethylester : Cyclohexan (5:1) gereinigt. Man erhält 336 mg (64 % der Theorie) 5-Allyl-4-{allyl[(6-ch]orpyridin-3-yl)methyl]amino}furan-2(5H)-on.
¹H-NMR (CDCl₃): δ [ppm] = 2.42 (m, 1H), 2.78 (m, 1 H), 3.80 (m, 2 H), 4.37 (d, 1 H); 4.43 (d, 1 H), 4.77 (s, 1 H), 5.05 (dd, 1 H), 5.17-5.28 (m, 3 H), 5.34 (d, 1 H), 5.78 (m, 2 H), 7.35 (d, 1 H), 7.54 (dd, 1 H), 8.28 (d, 1 H).

### Verbindungen der allgemeinen Formel (VII)

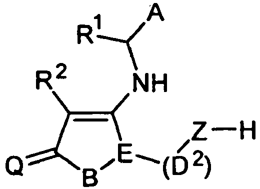

### Beispiel VII-1 3-Chlor-5-hydroxymethyl-4-{[(6-chlorpyridin-3-yl)methyl}amino}furan-2(5H)-on (R¹ = H, R² = Cl; B, Q = O; E-(D²)-Z-H = CHCH₂OH A = 6-Chlor-pyrid-3-yl)

126.0 mg (0,49 mmol) 5-Hydroxymethyl-4-{[(6-chlorpyridin-3-yl)methyl]amino}furan-2(5H)-on (VII-1b) werden in 7,5 ml Acetonitril verrührt und bei Raumtemperatur nacheinander mit 0,10 ml Triethylamin und 118,9 mg (0,89 mmol) *N-*Chlor-succinimid versetzt. Anschließend wird das Reaktionsgemisch 1 Stunde bei Raumtemperatur gerührt. Danach wird der gesamte Reaktionsansatz auf ein Volumen von ca. 2 ml im Vakuum eingeengt und mittels präparativer HPLC (neutral) gereinigt. Man erhält 76,4 mg (38,4 % der Theorie) 3-Chlor-5-hydroxymethyl-4-{[(6-chlorpyridin-3-yl)methyl]amino}furan-2(5H)-on.
LC-MS (m/z): 289 (M⁺) C₁₁H₁₀Cl₂N₂O₃ (289,1)

### VII-1a 4-Hydroxy-5-hydroxymethyl-furan-2(5H)-on:

386,0 mg (1,75 mmol) 5-Benzyloxymethyl-4-hydroxy-furan-2(5H)-on (Aragon, D. T. et al., J. Org. Chem. 68, 3363-3365, 2003) werden in 19,3 ml Ethanol vorgelegt und in Gegenwart von 38,6 mg (0,27 mmol) Pd(OH)₂/C (20 %) versetzt und bis zur Beendigung der Wasserstoffaufnahme bei Raumtemperatur hydriert. Nach Abfiltrieren des Katalysators und Einengen des Lösungsmittels erhält man 216 mg (94,7 % d. Theorie) 4-Hydroxy-5-hydroxymethyl-furan-2(5H)-on, das ohne weitere Reinigung für eine Folgereaktion verwendet werden kann.
LC-MS (m/z): 131 (M⁺+H) CH₆O₄ (130,1)

### VII-16 5-Hydroxymethyl-4-{[(6-chlorpyridin-3-yl)methyl]amino}furan-2(5H)-on:

905,0 mg (6,95 mmol) 4-Hydroxy-5-hydroxymethyl-furan-2(5H)-on (VII-1a) und 991,8 mg (6,95 mmol) 3-Aminomethyl-6-chlor-pyridin werden in einem Gemisch aus 100,5 mL Toluol und 10 ml *N*,*N*-Dimethyl-formamid (DMF) verrührt und mit 10 mg *para*-Toluolsulfonsäure sowie 0,5 ml Essigsäure versetzt. Anschließend wird der gesamte Reaktionsansatz ca. 18 Stunden unter Rühren am Wasserabscheider bei Rückflusstemperatur erhitzt. Nach Einengen im Vakuum und Reinigung des Rückstandes durch Säulen-chromatographie über Kieselgel (Kieselgel 60, Merck, Korngröße: 0.04 bis 0.063 mm; Eluent : Cyclohexan/Aceton = 1:1) erhält man 394,2 mg (22,2 % d. Th) 5-Hydroxymethyl-4-{([(6-chlorpyridin-3-yl)methyl]amino}furan-2(5H)-on.
LC-MS (m/z): 255 (M⁺) C₁₁H₁₁ClN₂O₃ (254,6)

### Verbindungen der allgemeinen Formel G-CH₂-A (X)

### X-1 (5,6-Dichlorpyridin-3-yl)methanol (G = OH, A = 5,6-Dichlor-pyrid-3-yl) (R. Graf et al. J. Prakt. Chem. 1932, 134, 177-87)

Zu 110 g (573 mmol) 5,6-Dichlor-nicotinsäure in 250 mL Tetrahydrofuran tropft man bei 0°C 859 mL (859 mmol) einer 1 M Lösung von Boran-Tetrahydrofuran-Komplex in Tetrahydrofuran hinzu. Man erwärmt auf Raumtemperatur und rührt bei dieser Temperatur 3 Stunden. Nach Abkühlen auf 0°C stellt man die Reaktionsmischung mit gesättigter wässriger Kaliumcarbonat-Lösung alkalisch, rotiert das Tetrahydrofuran weitgehend ab und extrahiert den Rückstand mehrmals mit Essigsäureethylester. Die vereinigten organischen Phasen werden mit Wasser und gesättigter wässriger Natriumchlorid-Lösung gewaschen und über Natriumsulfat getrocknet. Nach Einengen der organischen Phase im Vakuum und Reinigung des Rückstandes durch Säulenchromatographie über Kieselgel (Kieselgel 60 - Merck, Korngröße: 0.04 bis 0.063 mm) mit dem Laufmittelgemisch Essigsäureethylester : Cyclohexan (1:2) erhält man 62 g (61 % der Theorie) (5,6-Dichlorpyridin-3-yl)methanol.
¹H-NMR (CD₃CN): δ [ppm] = 3.31 (t, 1H), 4.60 (d, 2 H), 7.85 (s, 1 H), 8.26 (s, 1 H)

Analog der Vorschrift für die Verbindung (X-1) wurde auch die Verbindung (X-5) aus Tabelle 3 hergestellt.

### X-2 3-Brommethyl-5,6-dichlorpyridin (G = Br, A = 5,6-Dichlor-pyrid-3-yl) (vgl. WO 2000046196 A1)

Eine Lösung von 10.60 g (59.55 mmol) (5,6-Dichlorpyridin-3-yl)methanol (X-1) in 100 mL Dichlormethan versetzt man bei 0°C mit 16.40 g (62.52 mmol) Triphenylphosphin und 11.66 g (65.50 mmol) *N-*Bromsuccinimid. Nach 2 h wird die Reaktionsmischung weitgehend eingeengt und der Rückstand durch Säulenchromatographie über Kieselgel (Kieselgel 60 - Merck, Korngröße: 0.04 bis 0.063 mm) mit dem Laufmittelgemisch Essigsäureethylester : Cyclohexan (1:5) gereinigt. Man erhält 12.4 g (86 % der Theorie) 3-Brommethyl-5,6-dichlorpyridin.
¹H-NMR (CD₃CN): δ [ppm] = 4.53 (s, 2 H), 7.97 (s, 1 H), 8.35 (s, 1H)

Analog der Vorschrift für die Verbindung (X-2) wurden auch die Verbindungen (X-6) bis (X-8) aus (Tabelle 3) hergestellt.

### X-3 3-Brommethyl-6-chlor-5-iod-pyridin (G = Br, A = 6-Chlor-5-iod-pyrid-3-yl)

4.60 g (18.15 mmol) 6-Chlor-5-iod-3-methylpyridin (Setliff et al., J. Chem. Engineering Data (1976), 21(2), 246-7), 3.39 g (19.06 mmol) *N*-Bromsuccinimid und 0.30 g (1.82 mmol) 2,2'-Azobis(2-methylpropannitril) in 500 mL Chlorbenzol werden ca. 16 Stunden unter Rückfluss gekocht. Nach Waschen der Reaktionsmischung mit gesättigter wässriger Natriumsulfit-Lösung und Natriumhydrogencarbonat-Lösung trocknet man über Natriumsulfat und engt im Vakuum ein. Säulenchromatographie des Rückstandes über Kieselgel (Kieselgel 60 - Merck, Korngröße: 0.04 bis 0.063 mm) mit dem Laufmittelgemisch Essigsäureethylester : Cyclohexan (1:10) liefert 3.86 g (38 % der Theorie) 3-Brommethyl-6-chlor-5-iodpyridin.
¹H-NMR (CD₃CN): δ [ppm] = 4.48 (s, 2 H), 8.30 (s, 1 H), 8.40 (s, 1 H)

Analog der Vorschrift für die Verbindung (X-3) wurde auch die Verbindung (X-9) aus (Tabelle 3) hergestellt.

### X-4 6-Chlor-3-chlormethyl-5-fluorpyridin (G = Cl, A = 6-Chlor-5-fluor-pyrid-3-yl)

1.00 g (6.87 mmol) 6-Chlor-5-fluor-3-methylpyridin (F. L. Setliff, Organic Preparations and Procedures International 1971, 3, 217-222), 1.01 g (7.56 mmol) *N*-Chlorsuccinimid und 0.11 g (0.69 mmol) 2,2'-Azobis(2-methylpropannitril) in 100 mL Chlorbenzol werden 2 Tage unter Rückfluss gekocht. Dabei werden nach ca. 16 und 32 Stunden jeweils weitere 1.01 g (7.56 mmol) *N*-Chlorsuccinimid und 0.11 g (0.69 mmol) 2,2'-Azobis(2-methylpropannitril) hinzugefügt. Nach Waschen der Reaktionsmischung mit gesättigter wässriger Natriumsulfit-Lösung und Natriumhydrogencarbonat-Lösung trocknet man über Natriumsulfat und engt im Vakuum ein. Säulenchromatographie des Rückstandes über Kieselgel (Kieselgel 60 - Merck, Korngröße: 0.04 bis 0.063 mm) mit dem Laufmittelgemisch Essigsäureethylester : Cyclohexan (1:20) liefert 0.65 g (53 % der Theorie) 6-Chlor-3-chlormethyl-5-fluorpyridin.
¹H-NMR (CD₃CN): δ [ppm] = 4.68 (s, 2 H), 7.69 (d, 1H), 8.27 (s, 1H)

In der nachstehenden Tabelle 3 sind weitere Verbindungen (X-5) bis (X-10) der Formel (X) aufgeführt.

**Tabelle 3**

| **G-CH₂-A (X)** | | | |
|---|---|---|---|
| Bsp.-Nr. | G | A | Physikalische Daten^{a}) |
| X-5 | OH | 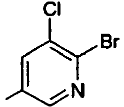 | 3.30 (t, 1H), 4.59 (d, 2H), 7.83 (s, 1H), 8.26 (s, 1H) |
| X-6 | Br | 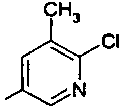 | 2.37 (s, 3H), 4.52 (s, 2H), 7.70 (s, 1H), 8.24 (s, 1H) |
| X-7 | Br | 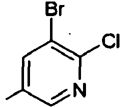 | 4.52 (s, 2H), 8.10 (s, 1H), 8.38 (s, 1H) |
| X-8 | Br | 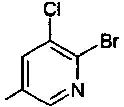 | 4.52 (d, 2H), 7.92 (s, 1H), 8.35 (s, 1H) |
| X-9 | Br | 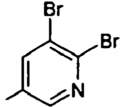 | 4.50 (s, 2H), 8.07 (s, 1H), 8.37 (s, 1H) |
| X-10 | Br | 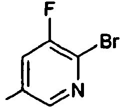 | 4.55 (s, 2H), 7.65 (d, 1H), 8.27 (s, 1H) |

| | | | |
|---|---|---|---|
| ^{a) 1}H-NMR (CD₃CN), δ [ppm] | | | |

### Verbindungen der allgemeinen Formel (XI)

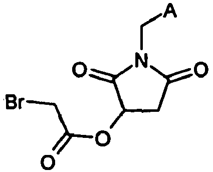

### Beispiel XI-1 1-[(6-Chlorpyridin-3-yl)methyl]-2,5-dioxopyrrolidin-3-yl-bromacetat (A = 6-Chlor-pyrid-3-yl)

1.34 g (5.56 mmol) 1-[(6-Chlorpyridin-3-yl)methyl]-3-hydroxypyrrolidin-2,5-dion (XI-1a) und 674 µL (8.34 mmol) Pyridin werden in 15 mL Dichlormethan gelöst und bei 0°C mit 1.12 g (5.56 mmol) Bromacetylbromid versetzt. Man rührt 30 Minuten bei Raumtemperatur und gibt anschließend Eiswasser hinzu. Die organische Phase wird nacheinander mit gesättigter Natriumhydrogencarbonat-Lösung und gesättigter Natriumchlorid-Lösung gewaschen, über Magnesiumsulfat getrocknet und im Vakuum eingeengt. Nach Reinigung des Rückstandes durch Säulenchromatographie über Kieselgel (Kieselgel 60, Merck, Korngröße: 0.04 bis 0.063 mm) mit dem Laufmittelgemisch Dichlormethan : Methanol (97:3) erhält man 1.44 g (55 % der Theorie) 1-[(6-Chlorpyridin-3-yl)methyl]-2,5-dioxopyrrolidin-3-yl-bromacetat.
¹H-NMR (cd13): δ [ppm] = 2.75 (dd, 1 H), 3.22 (dd, 1H), 3.90 (s, 2 H), 4.69 (s, 2 H), 5.50 (dd, 1 H), 7.31 (d, 1H), 7.71 (dd, 1H), 8.44 (d, 1H).

### XI-1a 1-[(6-Chlorpyridin-3-yl)methyl]-3-hydroxypyrrolidin-2,5-dion:

4.53 (33.79 mmol) D,L-Äpfelsäure und 5.30 g (37.17 mmol) 1-(6-Chlorpyridin-3-yl)methanamin werden in 40 mL Xylol 2 Stunden unter Rückfluss erhitzt. Nach Abkühlen auf Raumtemperatur wird abdekantiert und der Rückstand im Vakuum eingeengt. Man erhält als Rohprodukt 8.30 g (100 % der Theorie) 1-[(6-Chlorpyridin-3-yl)methyl]-3-hydroxypyrrolidin-2,5-dion, das ohne weitere Reinigung in der Folgereaktion eingesetzt werden kann.
¹H-NMR (CDCl₃): δ [ppm] = 2.71 (dd, 1H), 2.92 (br. s., 1H), 3.10 (dd, 1H), 4.65 (m, 1H), 4.66 (s, 2 H), 7.30 (d, 1 H), 7.71 (dd, 1 H), 8.44 (d, 1 H).

### Biologische Beispiele

### Beispiel Nr. 1

### Myzus-Test (MYZUPE Spritzbehandlung)

| | |
|---|---|
| Lösungsmittel: | 78 Gewichtsteile Aceton |
| | 1,5 Gewichtsteile Dimethylformamid |

Emulgator: 0,5 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben (*Brassica pekinensis*)*,* die von allen Stadien der Grünen Pfirsichblattlaus (*Myzus persicae*) befallen sind, werden mit einer Wirkstoffzubereitung der gewünschten Konzentration gespritzt.

Nach der gewünschten Zeit wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele gute Wirksamkeit: siehe Tabelle

| Beispiel | Wirkstoffkonzentration in g/ha | Abtötungsgrad in % nach 5 Tagen |
|---|---|---|
| Beispiel I-1 | 500 | 100 |
| Beispiel I-2 | 500 | 100 |
| Beispiel I-3 | 500 | 100 |
| Beispiel I-4 | 500 | 100 |
| Beispiel I-6 | 500 | 100 |
| Beispiel I-7 | 500 | 100 |
| Beispiel I-8 | 500 | 100 |
| Beispiel I-9 | 500 | 100 |
| Beispiel I-10 | 500 | 100 |
| Beispiel I-11 | 500 | 100 |
| Beispiel I-12a | 500 | 100 |
| Beispiel I-12b | 500 | 100 |
| Beispiel I-14 | 500 | 100 |
| Beispiel I-15 | 500 | 100 |
| Beispiel I-17a | 500 | 100 |
| Beispiel I-17b | 500 | 90 |
| Beispiel I-18 | 500 | 100 |
| Beispiel I-19 | 500 | 100 |
| Beispiel I-20 | 500 | 100 |
| Beispiel I-22 | 500 | 100 |
| Beispiel I-23 | 500 | 100 |
| Beispiel I-24 | 500 | 100 |
| Beispiel I-27a | 500 | 100 |
| Beispiel I-27b | 500 | 100 |

### Beispiel Nr.2

### Myzus-Test; oral; (MYZUPE O)

| | |
|---|---|
| Lösungsmittel: | 80 Gewichtsteile Aceton |

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser auf die gewünschte Konzentration.

Gefäße werden mit allen Stadien der Grünen Pfirsichblattlaus (*Myzus persicae*) besetzt, durch Saugen an der Wirkstoffzubereitung der gewünschten Konzentration wird behandelt.

Nach der gewünschten Zeit wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele gute Wirksamkeit: siehe Tabelle

| Beispiel | Wirkstoffkonzentration in ppm | Abtötungsgrad in % nach 5 Tagen |
|---|---|---|
| Beispiel I-1 | 100 | 100 |
| Beispiel I-12a | 100 | 100 |
| Beispiel I-12b | 100 | 100 |
| Beispiel I-14 | 100 | 100 |

### Beispiel Nr. 3

### Spodoptera frugiperda-Test (SPODFR Spritzbebandlung)

| | |
|---|---|
| Lösungsmittel: | 78 Gewichtsteile Aceton |
| | 1,5 Gewichtsteile Dimethylformamid |
| Emulgator: | 0,5 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Maisblattscheiben (*Zea mays*) werden mit einer Wirkstoffzubereitung der gewünschten Konzentration gespritzt und nach dem Abtrocknen mit Raupen des Heerwurms (*Spodoptera frugiperda*) besetzt.

Nach der gewünschten Zeit wird die Wirkung in % bestimmt. Dabei bedeutet 100 %, dass alle Raupen abgetötet wurden; 0 % bedeutet, dass keine Raupe abgetötet wurde.

Bei diesem Test zeigen z. B. die folgenden Verbindungen der Herstellungsbeispiele gute Wirksamkeit: siehe Tabelle

| Beispiel | Wirkstoffkonzentration in g/ha | Abtötungsgrad in % nach 7 Tagen |
|---|---|---|
| Beispiel I-2 | 500 | 100 |
| Beispiel I-3 | 500 | 100 |

### Beispiel Nr. 4

### Phaedon cochleariae-Test (PHAECO Spritzbehandlung)

| | |
|---|---|
| Lösungsmittel: | 78 Gewichtsteile Aceton |
| | 1,5 Gewichtsteile Dimethylformamid |
| Emulgator: | 0,5 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben (*Brassica pekinensis*) werden mit einer Wirkstoffzubereitung der gewünschten Konzentration gespritzt und nach dem Abtrocknen mit Larven des Meerrettichblattkäfers *(Phaedon cochleariae)* besetzt.

Nach der gewünschten Zeit wird die Wirkung in % bestimmt. Dabei bedeutet 100 %, dass alle Käferlarven abgetötet wurden; 0 % bedeutet, dass keine Käferlarven abgetötet wurden.

Bei diesem Test zeigen z. B. die folgenden Verbindungen der Herstellungsbeispiele gute Wirksamkeit: siehe Tabelle

| Beispiel | Wirkstoffkonzentration in g/ha | Abtötungsgrad in % nach 7 Tagen |
|---|---|---|
| Beispiel I-3 | 500 | 100 |
| Beispiel I-4 | 500 | 100 |
| Beispiel I-17a | 500 | 100 |
| Beispiel I-20 | 500 | 100 |
| Beispiel I-23 | 500 | 100 |
| Beispiel 1-24 | 500 | 100 |
| Beispiel I-27a | 500 | 83 |
| Beispiel I-27b | 500 | 100 |

### Beispiel Nr. 5

### Tetranychus-Test, OP-resistent (TETRUR Spritzbehandlung)

| | |
|---|---|
| Lösungsmittel: | 78 Gewichtsteile Aceton |
| | 1,5 Gewichtsteile Dimethylformamid |
| Emulgator: | 0,5 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Bohnenblattscheiben *(Phaseolus vulgaris),* die von allen Stadien der Gemeinen Spinnmilbe *(Tetranychus urticae)* befallen sind, werden mit einer Wirkstoffzubereitung der gewünschten Konzentration gespritzt.

Nach der gewünschten Zeit wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigen z. B. die folgenden Verbindungen der Herstellungsbeispiele gute Wirksamkeit: siehe Tabelle

| Beispiel | Wirkstoffkonzentration in g/ha | Abtötungsgrad in % nach 6 Tagen |
|---|---|---|
| Beispiel I-12b | 500 | 90 |

### Beispiel Nr. 6

### Myzus persicae-Test, hydroponische Behandlung (MYZUPE sys.)

| | |
|---|---|
| Lösungsmittel: | 7 Gewichtsteile Dimethylformamid |
| Emulgator: | 2 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Die Wirkstoffzubereitung wird mit Wasser gemischt. Die angegebene Konzentration bezieht sich auf die Wirkstoffmenge pro Volumeneinheit Wasser (mg/l = ppm). Man füllt das behandelte Wasser in Gefäße mit einer Erbsenpflanze *(Pisum sativum),* anschließend wird mit der Grünen Pfirsichblattlaus *(Myzus persicae)* infiziert.

Nach der gewünschten Zeit wird die Abtötung in % bestimmt. Dabei bedeutet 100 %, dass alle Blattläuse abgetötet wurden; 0 % bedeutet, dass keine Blattläuse abgetötet wurden.

Bei diesem Test zeigt z. B. die folgende Verbindungen der Herstellungsbeispiele gute Wirksamkeit: siehe Tabelle

| Beispiel | Wirkstoffkonzentration in ppm | Abtötungsgrad in % nach 6 Tagen |
|---|---|---|
| Beispiel I-2 | 20 | 100 |
| Beispiel I-7 | 20 | 100 |
| Beispiel I-9 | 20 | 100 |

### Beispiel Nr. 7

### Aphis gossypii -Test (APHIGO)

| | |
|---|---|
| Lösungsmittel: | 7 Gewichtsteile Dimethylformamid |
| Emulgator: | 2 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Baumwollblätter (*Gossypium hirsutum*), die stark von der Baumwollblattlaus (*Aphis gossypii*) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, dass alle Blattläuse abgetötet wurden; 0 % bedeutet, dass keine Blattläuse abgetötet wurden.

Bei diesem Test zeigt z. B. die folgende Verbindungen der Herstellungsbeispiele gute Wirksamkeit: siehe Tabelle

| Beispiel | Wirkstoffkonzentration in ppm | Abtötungsgrad in % nach 6 Tagen |
|---|---|---|
| Beispiel I-2 | 20 | 95 |
| Beispiel I-7 | 100 | 95 |
| Beispiel I-9 | 100 | 90 |

### Beispiel Nr. 8

### Lucilia cuprina-Test (LUCICU)

| | |
|---|---|
| Lösungsmittel: | Dimethylsulfoxid |

Zur Herstellung einer zweckmäßigen Wirkstoffzubereitung vermischt man 1 Gewichtsteil Wirkstoff mit der angegebenen Menge Wasser und verdünnt das Konzentrat mit Wasser auf die gewünschte Konzentration.

Gefäße, die Pferdefleisch enthalten, das mit der Wirkstoffzubereitung der gewünschten Konzentration behandelt wurde, werden mit *Lucilia cuprina* Larven besetzt.

Nach der gewünschten Zeit wird die Abtötung in % bestimmt. Dabei bedeutet 100 %, dass alle Larven abgetötet wurden; 0 % bedeutet, dass keine Larven abgetötet wurden.

Bei diesem Test zeigen z. B. die folgenden Verbindungen der Herstellungsbeispiele überlegene Wirksamkeit gegenüber dem Stand der Technik: siehe Tabelle

| Beispiel | Wirkstoffkonzentration in ppm | Abtötungsgrad in % nach 2 Tagen |
|---|---|---|
| Beispiel I-2 | 100 | 100 |
| Beispiel I-6 | 100 | 100 |
| Beispiel I-9 | 100 | 80 |

### Beispiel Nr. 9

### Aphis gossypii -Test (APHIGO)

| | |
|---|---|
| Lösungsmittel: | 7 Gewichtsteile Dimethylformamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Baumwollblätter (*Gossypium hirsutum*), die stark von der Baumwollblattlaus (*Aphis gossypii*) befallen sind, werden durch Sprühen mit der Wirkstoffzubereitung in der gewünschten Konzentration behandelt.

Nach der gewünschten Zeit wird die Abtötung in % bestimmt. Dabei bedeutet 100 %, dass alle Tiere abgetötet wurden; 0 % bedeutet, dass keine Tiere abgetötet wurden.

Bei diesem Test zeigen z. B. die folgenden Verbindungen der Herstellungsbeispiele gute Wirksamkeit: siehe Tabelle

| | | |
|---|---|---|
| Beispiel | Wirkstoffkonzentration in g/ha | Abtötungsgrad in % nach 7 Tagen |
| Beispiel I-2 | 12 | 85 |

## Patentansprüche

1. Bicyclische Enaminocarbonyl-verbindungen der Formel (I) 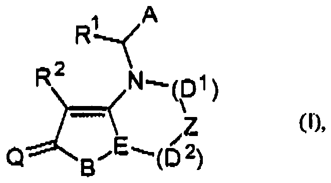 in welcher
A für einen Rest steht, ausgewählt aus der Gruppe bestehend aus 6-Chlor-pyrid-3-yl, 6-Brom-pyrid-3-yl, 6-Methyl-pyrid-3-yl- 6-Trifluormethyl-pyrid-3-yl, 2-Methyl-pyrimidin-5-yl, 2-Chlor-pyrimid-5-yl, 1*H*-Pyrazol-4-yl, welches gegebenenfalls in 1-Position substituiert ist durch Methyl, Ethyl, und in 3-Position durch Chlor, 1*H*-Pyrazol-5-yl, 3-Methyl-pyrazol-5-yl, 2-Brom-1,3-thiazol-5-yl, 2-Chlor-1,3-thiazol-5-yl, Isoxazol-5-yl, welches gegebenenfalls in 3-Position substituiert ist durch Methyl, Ethyl, Chlor oder Brom, 3-Methyl-1,2,4-oxadiazol-5-yl, 1-Methyl-1,2,4-triazol-3-yl, 1,2,5-Thiadiazol-3-yl, 5-Fluor-6-chlor-pyrid-3-yl, 5,6-Dichlor-pyrid-3-yl, 5-Brom-6-chlor-pyrid-3-yl, 5-Fluor-6-brom-pyrid-3-yl, 5-Chlor-6-brom-pyrid-3-yl, 5,6-Dibrom-pyrid-3-yl, 5-Methyl-6-chlor-pyrid-3-yl, 5-Methyl-6-iod-pyrid-3-yl und 5-Difluormethyl-6-chlor-pyrid-3-yl,
B für Sauerstoff, oder Methylen steht,
E für CH oder C-alkyl steht,
D¹-Z-D² für eine der folgenden chemischen Gruppierungen steht: -CH₂-CH₂-, -HC=CH-, -CH₂-CH₂-CH₂-, -CH₂-C(=CH₂)-CH₂-, CH₂-CO-CH₂-, -CH₂-CH(OH)-CH₂-, -CH₂-CF₂-CH₂-, -CH₂-CHF-CH₂-, -CH₂-CHCl-CH₂-, -CH=H-CH₂-, -CH₂-CH₂-CH=, -CH₂-CH₂-CH(OH)-, -CH₂-CH(CH₃)-CH₂-, -CH₂-CH₂-CH₂-CH₂-, -CH₂-CH=CH-CH₂, -CH₂-O-CH₂-, -CH₂-CH₂-O-, -CH₂-N(CH₃)-CH₂-, -CH₂-CH₂-N(CH₃)- oder -CH₂-S-CH₂-,
R¹ für Wasserstoff steht,
R² für Wasserstoff, Fluor oder Chlor steht, und
Q für Sauerstoff steht.

2. Bicyclischo Enaminocarbonyl-verbindungen der Formel (I) gemüß Anspruch I, worin
A für einen Rest steht, ausgewählt aus der Gruppe bestehend aus 6-Chlor-pyrid-3-yl, 6-Brom-pyrid-3-yl, 2-Chlor-pyrimid-5-yl, 5-Fluor-6-chlor-pyrid-3-yl, 5,6-Dichlor-pyrid-3-yl, 5-Fluor-6-brom-pyrid-3-yl, 2-Chlor-1,3,-thiazol-5-yl;
B für Sauerstoff steht,
E für CH oder C-alkyl steht,
D¹-Z-D² für eine der folgenden chemischen Gruppierungen steht: -CH₂-CH₂-CH₂-, -CH₂-C(=CH₂)-CH₂-, -CH₂-C(CH₃)-CH₂-, -CH₂-CH₂-CH₂-CH₂-, -CH₂-CH=CH-CH₂-, -CH₂-O-CH₂, -CH₂-CH₂-O-, CH₂-CHF-CH₂-, -CH₂-CHCl-CH₂-, -CH=CH-CH₂-, -CH₂-CH₂-CH= und -CH₂-CH₂-CH(OH)-,
R¹ für Wasserstoff steht,
R² für Wasserstoff steht,
Q für Sauerstoff steht.

3. Bicyclische Enaminocarbonyl-verbindungen der Formel (1) gemäß Anspruch 1, worin
R¹ und R² für Wasserstoff steht,
D¹-Z-D² für -(CH₂)₃- steht,
E für Kohlenstoff steht,
B und Q für Sauerstoff stehen,
A für 6-Chlor-pyrid-3-yl, 6-Brom-pyrid-3-yl, 2-Chlor-pyrimidin-5-yl, 5-Fluor-6-chlor-pyrid-3-yl, 2-Chlor-1,3-thiazol-5-yl, 5,6-Dichlor-pyrid-3-yl, 5-Brom-6-Chlor-pyrid-3-yl, 5-Methyl-6-chlor-pyrid-3-yl, 5-Fluor-6-brom-pyrid-3-yl, 5-Chlor-6-brom-pyrid-3-yl, oder 5-Chlor-6-iod-pyrid-3-yl steht,

4. Mittel enthaltend mindestens eine der bicyclischen Enaminocarbonyl-verbindungen wie in einem der Ansprüche 1 bis 3 definiert und zusätlich übliche Streckmittel und/oder oberflüchenaktiven Substanzen.

5. Verfahren zum Bekämpfen von Arthropoden, **dadurch gekennzeichnet, dass** man eine bicyclische Enaminocarbonyl-verbindung der Formel (1) wie in einem der Ansprüche 1 bis 3 definiert, oder ein Mittel wie Anspruch 4 definiert, auf die Arthropoden und/oder ihren Lebensraum einwirken lässt,

6. Verwendung von bicyclischen Enaminocarbonyl-verbindungen der Formel (I) wie in einem der Ansprüche 1 bis 3 definiert, oder von Mitteln wie in Anspruch 4 definiert, zum Bekämpfen von Arthropoden.

7. Verwendung von bicyclischen Enaminocarbonyl-verbindungen der Formel (1) wie in einem der Ansprüche 1 bis 3 definiert, oder von Mitteln wie in Anspruch 4 definiert, zum Schutz von Pflanzen und Pflanzenorganen und zur Bekämpfung von Insekten, Spinnentieren, Helminthen, Nematoden und Mollusken, die in der Landwirtschaft, im Gartenbau, bei der Tierzucht, in Forsten, in Glirten und Freizeiteinrichtungen, im Vorrats- und Materialschutz sowie auf dem Hygienesektor vorkommen.

## Claims

1. Bicyclic enaminocarbonyl compounds of the formula (I) 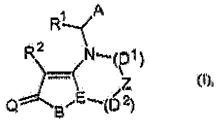 in which
A represents a radical selected from the group consisting of 6-chloropyrid-3-yl, 6-bromopyrid-3-yl, 6-methylpyrid-3-yl, 6-trifluoromethyl-pyrid-3-yl, 2-methylpyrimidin-5-yl, 2-chloro-pyrimid-5-yl, 1*H*-pyrazol-4-yl which is optionally substituted in the 1-position by methyl, ethyl and in the 3-position by chlorine, 1*H*-pyrazol-5-yl, 3-methylpyrazol-5-yl, 2-bromo-1,3-thiazol-5-yl, 2-chloro-1,3-thiazol-5-yl, isoxazol-5-yl which is optionally substituted in the 3-position by methyl, ethyl, chlorine or bromine, 3-methyl-1,2,4-oxadiazol-5-yl, 1-methyl-1,2,4-triazol-3-yl, 1,2,5-thiadiazol-3-yl, 5-fluoro-6-chloropyrid-3-yl, 5,6-dichloropyrid-3-yl, 5-bromo-6-chloropyrid-3-yl, 5-fluoro-6-bromopyrid-3-yl, 5-chloro-6-bromopyrid-3-yl, 5,6-dibromopyrid-3-yl, 5-methyl-6-chloropyrid-3-yl-, 5-methyl-6-iodopyrid-3-yl and 5-difluoromethyl-6-chloropyrid-3-yl,
B represents oxygen or methylere,
E represents CH or C-alkyl,
D¹-Z-D² represents one of the chemical groupings below: -CH₂-CH₂-, -HC=CH-, -CH₂-CH₂-CH₂-, -CH₂-C(=CH₂)-CH₂-, -CH₂-CO-CH₂-, -CH₂-CH(OH)-CH₂-, -CH₂-CF₂-CH₂-, -CH₂-CHF-CH₂-, -CH₂-CHCl-CH₂-, -CH=CH-CH₂-, -CH₂-CH₂-CH=, -CH₂-CH₂-CH (OH)-, -CH₂-CH(CH₃) -CH₂-, -CH₂-CH₂-CH₂-CH₂-, -CH₂-CH=CH-CH₂-, -CH₂-O-CH₂-, -CH₂-CH₂-O-, -CH₂-N(CH₂)-CH₂-, -CH₂-CH₂-N(CH₃)- or -CH₂-S-CH₂-,
R¹ represents hydrogen,
R² represents hydrogen, fluorine or chlorine and
Q represents oxygen.

2. Bicyclic enaminocarbonyl compounds of the formula (I) according to Claim 1 in which
A represents a radical selected from the group consisting of 6-chloropyrid-3-yl, 6-bromopyrid-3-yl, 2-chloropyrimid-5-yl, 5-fluoro-6-chloro-pyrid-3-yl, 5,6-dichloropyrid-3-yl, 5-fluoro-6-bromopyrid-3-yl, 2-chloro-1,3-thiazol-5-yl,
B represents oxygen,
E represents CH or C-alkyl,
D¹-Z-D² represents one of the chemical groupings below: -CH₂-CH₂-CH₂-, CH₂-C(=CH₂)-CH₂-, -CH₂-C (CH₃) -CH₂-, -CH₂-CH₂-CH₂-CH₂-, -CH₂-CH=CH-CH₂-, -CH₂-O-CH₂-, -CH₂-CH₂-O-, -CH₂-CHF-CH₂-, -CH₂-CHCl-CH₂-, -CH=CH-CH₂-, -CH₂-CH₂-CH- and -CH₂-CH₂-CH(OH)-,
R¹ represents hydrogen,
R² represents hydrogen,
Q represents oxygen.

3. Bicyclic enaminocarbonyl compounds of the formula (I) according to Claim 1 in which
R¹ and R² represent hydrogen,
D¹-Z-D² represents -(CH₂)₃-,
E represents carbon,
B and Q represent oxygen,
A represents 6-chloropyrid-3-yl, 6-bromopyrid-3-yl, 2-chloropyrimidin-5-yl, 5-fluoro-6-chloro-pyrid-3-yl, 2-chloro-1,3-thiazol-5-yl, 5,6-dichloropyrid-3-yl, 5-bromo-6-chloropyrid-3-yl, 5-methyl-6-chloropyrid-3-yl, 5-fluoro-6-bromopyrid-3-yl, 5-chloro-6-bromopyrid-3-yl or 5-chloro-6-iodopyrid-3-yl.

4. Composition, comprising at least one of the bicyclic enaminocarbonyl compounds as defined in any of Claims 1 to 3 and additionally customary extenders and/or surfactants.

5. Method for controlling arthropods, **characterized in that** a bicyclic enaminocarbonyl compound of the formula (I) as defined in any of Claims 1 to 3 or a composition as defined in Claim 4 is allowed to act on the arthropods and/or their habitat.

6. Use of bicyclic enaminocarbonyl compounds of the formula (I) as defined in any of Claims 1 to 3 or of compositions as defined in Claim 4 for controlling arthropods.

7. Use of bicyclic enaminocarbonyl compounds of the formula (I) as defined in any of Claims 1 to 3 or of compositions as defined in Claim 4 for protecting plants and plant organs and for controlling insects, arachnids, helminths, nematodes and molluscs encountered in agriculture, in horticulture, in animal breeding, in forests, in gardens and in leisure facilities, in the protection of stored goods and materials and in the hygiene sector.

## Revendications

1. Composés énaminocarbonyle bicycliques de formule (I) 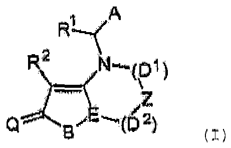 dans laquelle
A représente un radical choisi dans le groupe constitué par 6-chloro-pyrid-3-yle, 6-bromo-pyrid-3-yle, 6-méthyl-pyrid-3-yle, 6-trifluorométhyl-pyrid-3-yle, 2-méthyl-pyrimidin-5-yle, 2-chloro-pyrimid-5-yle, 1*H*-pyrazol-4-yle, qui est éventuellement substitué en position 1 par méthyle, éthyle, et en position 3 par chloro, 1H-pyrazol-5-yle, 3-méthyl-pyrazol-5-yle, 2-bromo-1,3-thiazol-5-yle, 2-chloro-1,3-thiazol-5-yle, isoxazol-5-yle, qui et éventuellement substitué en position 3 par méthyle, éthyle, chloro ou bromo, 3-méthyl-1,2,4-oxadiazol-5-yle, 1-méthyl-1,2,4-triazol-3-yle, 1,2,5-thiadiazol-3-yle, 5-fluoro-6-chloro-pyrid-3-yle, 5,6-dichloro-pyrid-3-yle, 5-bromo-6-chloro-pyrid-3-yle, 5-fluoro-6-bromo-pyrid-3-yle, 5-chloro-6-bromo-pyrid-3-yle, 5,6-dibromo-pyrid-3-yle, 5-méthyl-6-chloro-pyrid-3-yle, 5-méthyl-6-iodo-pyrid-3-yle et 5-difluoro-méthyl-6-chloro-pyrid-3-yle,
B représente un atome d'oxygène ou le groupe méthylène,
E représente CH ou un groupe C-alkyle,
D¹-Z-D² représente l'un des groupements chimiques suivants : -CH₂-CH₂-, -HC=CH-, -CH₂-CH₂-CH₂-, -CH₂-C(=CH₂)-CH₂, -CH₂-CO-CH₂-, -CH₂-CH(OH)-CH₂-, -CF₂-CF₂-CH₂-, -CH₂-CHF-CH₂-, -CH₂-CHCl-CH₂-, -CH=CH-CH₂-, -CH₂-CH₂-CH=, -CH₂-CH₂-CH(OH)-, -CH₂-CH(CH₃) -CH₂-, -CH₂-CH₂-CH₂-CH₂-, -CH₂-CH=CH-CH₂-, -CH₂-O-CH₂-, -CH₂-CH₂-O-, -CH₂-N(CH₃)-CH₂-, -CH₂-CH₂-N(CH₃)- ou -CH₂-S-CH₂-,
R¹ représente un atome d'hydrogène,
R² représente un atome d'hydrogène, de fluor ou de chlore, et
Q représente un atome d'oxygène.

2. Composés énaminocarbonyle bicycliques de formule (I) selon la revendication 1, dans lesquels
A représente un radical choisi dans le groupe constitué par 6-chloro-pyrid-3-yle, 6-bromo-pyrid-3-yle, 2-chloro-pyrimid-5-yle, 5-fluoro-6-chloro-pyrid-3-yle, 5,6-dichloro-pyrid-3-yle, 5-fluoro-6-bromo-pyrid-3-yle, 2-chloro-1,3-thiazol-5-yle ;
B représente un atome d'oxygène,
E représente CH ou un groupe C-alkyle,
D¹-Z-D² représente l'un des groupements chimiques suivants : -CH₂-CH₂-CH₂-, -CH₂-C(=CH₂)-CH₂, -CH₂-C(CH₃)-CH₂-, -CH₂-CH₂-CH₂-CH₂-, -CH₂-CH=CH-CH₂--CH₂-O-CH₂-, -CH₂-CH₂-O-, -CH₂-CHF-CH₂-, -CH₂-CHCl-CH₂-, -CH=CH-CH₂-, -CH₂-CH₂-CH= et -CH₂-CH₂-CH(OH) -,
R¹ représente un atome d'hydrogène,
R² représente un atome d'hydrogène,
Q représente un atome d'oxygène.

3. Composés énaminocarbonyle bicycliques de formule (I) selon la revendication 1, dans lesquels
R¹ et R² représentent des atomes d'hydrogène,
D¹-Z-D² représente -(CH₂)₃-,
E représente un atome de carbone,
B et Q représentent des atomes d'oxygène,
A représente le groupe 6-chloro-pyrid-3-yle, 6-bromo-pyrid-3-yle, 2-chloro-pyrimidin-5-yle, 5-fluoro-6-chloro-pyrid-3-yle, 2-chloro-1,3-thiazol-5-yle, 5,6-dichloro-pyrid-3-yle, 5-bromo-6-chloro-pyrid-3-yle, 5-méthyl-6-chloro-pyrid-3-yle, 5-fluoro-6-bromo-pyrid-3-yle, 5-chloro-6-bromo-pyrid-3-yle, ou 5-chloro-6-iodo-pyrid-3-yle.

4. Composition contenant au moins l'un des composés énaminocarbonyle bicycliques tels que définis dans l'une quelconque des revendications 1 à 3 et additionnellement des excipients usuels et/ou des substances tensioactives usuelles.

5. Procédé pour la lutte contre des arthropodes, **caractérisé en ce qu'**on fait agir sur les arthropodes et/ou leur habitat un composé énaminocarbonyle bicycliques de formule (=) tel que défini dans l'une quelconque des revendications 1 à 3, ou une composition telle que définie dans la revendication 4.

6. Utilisation de composés énaminocarbonyle bicycliques de formule (I) tels que définis dans l'une quelconque des revendications 1 à 3, ou de compositions telles que définies dans la revendication 4, pour la lutte contre des arthropodes.

7. Utilisation de composés énaminocarbonyle bicycliques de formule (I) tels que définis dans l'une quelconque des revendications 1 à 3, ou de compositions telles que définies dans la revendication 4, pour la protection de plantes et d'organes de plantes et pour la lutte contre des insectes, arachnides, helminthes, nématodes et mollusques, qui sont présents en agriculture, en horticulture, dans l'élevage, dans des forêts, dans des jardins et des installations de loisir, dans la protection de stocks et de matériaux ainsi que dans le secteur de l'hygiène.
